# EUROPEAN PATENT SPECIFICATION

(11) **EP 1 698 635 B1**
(45) Date of publication and mention of the grant of the patent: **14.12.2011**
(21) Application number: 04807647.5
(22) Date of filing: 16.12.2004
(51) Int. Cl.: C07H 19/052, A61K 31/7056, A61P 13/12, A61P 19/06, A61P 43/00

(54) **BENZIMIDAZOLE DERIVATIVES AND MEDICAL USES THEREOF**
BENZIMIDAZOLDERIVATE UND DEREN MEDIZINISCHE VERWENDUNGEN
DERIVES BENZIMIDZOLE ET LEURS UTILISATIONS MEDICALES

(30) Priority: 26.12.2003 JP 2003432581
(43) Date of publication of application: 06.09.2006
(73) Proprietor: Kissei Pharmaceutical Co., Ltd., Matsumoto-shi, Nagano 399-8710 (JP)
(72) Inventor: KIKUCHI, Norihiko, Kissei Pharmaceutical Co., Ltd, Minamiazumi-gun, Nagano 399-8304 (JP); NONAKA, Yoshinori, Kissei Pharm. Co., Ltd., Minamiazumi-gun, Nagano 399-8304 (JP); TATANI, Kazuya, Kissei Pharmaceuticals Co., Ltd., Minamiazumi-gun, Nagano 399-8304 (JP); HIRATOCHI, Masahiro, Kissei Pharm. Co., Ltd., Minamiazumi-gun, Nagano 399-8304 (JP); KURAMOCHI, Yu, Kissei Pharmaceuticals Co., Ltd., Minamiazumi-gun, Nagano 399-8304 (JP); ISAJI, Masayuki, Kissei Pharmaceuticals Co., Ltd., Minamiazumi-gun, Nagano 399-8304 (JP); SHIMIZU, Kazuo, Kissei Pharmaceuticals Co., Ltd., Minamiazumi-gun, Nagano 399-8304 (JP); MIYAGI, Takashi, Kissei Pharmaceuticals Co., Ltd., minamiazumi-gun, Nagano 399-8304 (JP)
(74) Representative: Hayes, Adrian Chetwynd
(86) International application number: PCT/JP2004/019290
(87) International publication number: WO 2005/063788

(56) References cited:
- JP-A- 2000 515 119
- SHIN H-C ET AL: "Functional expression and characterization of a sodium-dependent nucleoside transporter hCNT2 cloned from human duodenum" BIOCHEMICAL AND BIOPHYSICAL RESEARCH COMMUNICATIONS, ACADEMIC PRESS INC. ORLANDO, FL, US, vol. 307, no. 3, 9 July 2003 (2003-07-09), pages 696-703, XP004441485 ISSN: 0006-291X
- ZOLLNER N. ET AL: 'Purine and pyrimidine metabolism' PROC.NUTR.SOC. vol. 41, 1982, pages 329 - 342, XP002989883
- RITZEL M.W.L. ET AL: 'Molecular Identification and Characterization of Novel Human and Mouse Concentrative Na+-Nucleoside Cotransporter Proteins (hCNT3 and mCNT3) Broadly Selective for Purine and Pyrimidine Nucleosides (System cib)' J.BIOL.CHEM. vol. 276, 2001, pages 2914 - 2927, XP002974895

## Description

### Technical Field

The present invention relates to benzimidazole derivatives which are useful as medicaments.

More particularly, the present invention relates to benzimidazole derivatives or pharmaceutically acceptable salts thereof, or prodrugs thereof which exhibit an inhibitory activity in sodium-dependent nucleoside transporter 2 (hereinafter referred to as CNT2) and are useful as agents for the prevention or treatment of a disease associated with an abnormality of plasma uric acid level.

### Background

Uric acid is the end product of purine bodies in human. The upper limit of normal uric acid concentration solved in plasma is 7.0 mg/dL independently from sex and age, and the condition with higher concentration is clinically defined as hyperuricemia. Hyperuricemia affects mostly in adult men and is considered to result from combination of a genetic factors involved in metabolism of purine bodies and secondary factors such as, for example, consumption of a high-energy food or nucleic acid rich food. Conditions of persistent hyperuricemic increase a risk of developing arthritis following to urate crystal deposition in intra- or peri-joints. The condition with such developed arthritis is called gout, and the arthritis is called gouty attack. Hyperuricemia is classified broadly into types consisting of a uric acid overproduction-type wherein the uric acid production increases, a uric acid underexcretion-type wherein the uric acid excretion in urine decreases, and a mixed type of them (for example, see Guideline for the management of hyperuricemia and gout, Version 1, 2002 (hereinafter referred to as the Management guideline), pp.12-22; and Diagnosis and Treatment, Vol.90, No.2, pp.186-191, 2002).

In the prevention or treatment of hyperuricemia or gout, the basis is to control the plasma uric acid level under a certain level to prevent the incidence of gouty arthritis, and the incidence of the gouty arthritis is considered the lowest in the case to control plasma uric acid level within the range from 4.4 to 6.6 mg/dL. So far, for the treatment of hyperuricemia or gout, allopurinol of a uric acid synthesis inhibitor or probenecid, bucolome, benzbromarone of uricosuric drugs or the like have been used for the improvement of the plasma uric acid level. In addition, in the treatment of gouty attacks, an agent for the pain attack such as colchicine, a nonsteroidal anti-inflammatory agent such as indometacin, naproxen, fenbufen, pranoprofen, oxaprozin, and an adrenocortical steroid are used (for example, see the above Management guideline, pp.23-45).

Allopurinol of a uric acid synthesis inhibitor has side effects such as, for example, poisoning syndrome (hypersensitivity angiitis), Stevens-Johnson syndrome, exfoliative dermatitis, aplastic anemia or hepatic insufficiency. In addition, a uricosuric drug has a restriction not to be used for a patient with renal failure, and probenecid, bucolome and benzbromarone have side effects such as gastrointestinal disorder, urinary lithiasis, especially, benzbromarone sometimes causes fulminant hepatic failure in a patient with idiosyncrasy (for example,' see the above Management guideline, pp.32-33).

It has been desired to develop a new preventative or therapeutic drug having few side effects which can solve such problems of these existing drugs, especially with a different mechanism compared with existing drugs from the viewpoint of broadening the choices of treatment methods.

Since hyperuricemia is brought on by life style such as for, example, overeating, food preference for high purine, high fat or high protein, habitual drinking or insufficient exercise and highly correlated with, for example, obesity, hypertension or abnormality in the metabolism of sugar or lipid, life style guidance plays an important role as non-drug therapy in order to correct the life style. In particular, dietary therapy to avoid excessive intake of purine has a major rule. However, it is difficult to continue such diet therapy and improvement of the life style, and they often fail.

An agent to regulate the digestion and absorption of purine which is different from existing agents such as a uric acid synthesis inhibitor or a uricosuric drug has been suggested for use as a part of or instead of dietary therapy (for example, see the Japanese patent publication no.2001-163788). The invention described in the patent publication relates to a drug to regulate the digestion and absorption of purine including chitosan for human, and the dosage is within the range of 2 to 2000 mg/kg/day which is rather high. In addition, it is used in the form of drink or food, and thus mainly used as an supplement in the dietary therapy. Moreover, an agent and food for the improvement of hyperuricemia including chitosan or dietary fiber as an active ingredient other than the invention described in the above patent publication have been developed (for example, see the Japanese patent no.2632577). Although the effects of chitosan or dietary fiber described in these gazettes are not clear, it is suspected that purine binds to or is trapped by a polymer, chitosan or dietary fiber, and so the production of uric acid decreases.

On the digestion and absorption pathway of nucleic acid in human, nucleic acids are released in the intestine from a nucleic acid and nucleoproteins ingested, and these nucleic acids are broken down into mononucleotides by ribonucleases, deoxyribonucleases and polynucleotidases. Furthermore, it is considered that the pathway wherein mononucleotide is degraded into nucleoside by nucleotidases and phosphatase and then the nucleosides are absorbed is the main pathway. In the pathway, it is considered that the absorbed purine nucleoside is changed to uric acid (for example, see Harper's Biochemistry, translation of the original edition 25, p. 417, 2001). As other pathways, it can be suspected that purine nucleoside is broken down to form purine base and then absorbed, or purine base contained in food is directly absorbed. However, these pathways have not been yet unexplained in detail.

Membrane proteins called nucleoside transporter relate to the nucleoside uptake in the intestine. As such transporters, there are Equilibrative transporters which have transport process of nucleoside into the cell by the concentration gradient of nucleoside (hereinafter referred to as ENT) and sodium-dependent nucleoside transporters which are driven by the concentration gradient of ion between in and out of the cell (hereinafter referred to as CNT) in mammalian cells (for example, see Membrane Transporters as Drug Targets, pp.318-321, 1999). As human nucleoside transporters, two types of ENT, Type 1 (hereinafter referred to as ENT1) and Type 2 (hereinafter referred to as ENT2), have been identified and cloned so far (for example, see NATURE MEDICINE, Vol.3, No.1, pp.89-93, 1997; and The Journal of Biological Chemistry, Vol.273, No.9, pp.5288-5293, 1998). In addition, three types of CNT, Type 1 (hereinafter referred to as CNT1), Type 2 (hereinafter referred to as CNT2) and Type 3 (hereinafter referred to as CNT3) have been identified and cloned (for example, see American Journal of Physiology Cell Physiology, Vol.272, pp.C707-C714, 1997; American Journal of Physiology Renal Physiology, Vol.273, pp.F1058-F1065, 1997; The Journal of Biological Chemistry, Vol.276, No.4, pp.2914-2927, 2001).

The distribution and characteristics of these transporters have been confirmed to some extent. Regarding ENTs, both ENT1 and ENT2 exist broadly in human normal tissues and transport both purine and pyrimidine nucleosides. In terms of function, their sensitivities to the inhibition by nitrobenzylthioinosine (hereinafter referred to as NBMPR) are different, that is, ENT1 is markedly inhibited by a low concentration of NBMPR (IC₅₀ < 5 nM), while ENT2 is hardly inhibited by NBMPR, but is inhibited only by a high concentration of NBMPR (IC₅₀ > 1 µM) (for example, see Membrane Transporters as Drug Targets, pp.316-318, 1999).

On the other hand, regarding CNTs, CNT1 transports pyrimidine nucleoside and adenosine, and the messenger RNA (hereinafter referred to as mRNA) has been confirmed to exist in the jejunum and kidney in rats. CNT2 transports purine nucleoside and uridine, and various kinds of mRNA have been confirmed to exist in organs including, for example, the heart, liver, skeletal muscles, kidney or intestines in human. CNT3 has been recently cloned and transports both purine and pyrimidine nucleosides, and the mRNA has been confirmed to exist in the bone marrow, pancreas, intestines and mammary gland in human. In addition, in terms of function, it has been confirmed that all of these CNTs are not influenced by NBMPR (for example, see The Journal of Biological Chemistry, Vol.276, No.4, pp.2914-2927, 2001; and Membrane Transporters as Drug Targets, pp.327-332, 1999).

In addition, in the previous studies on transport mechanism in the intestines, it is shown that nucleoside is taken up through CNT from mucosal side and transported through ENT from serosal side (for example, see Gastrointestinal transport, molecular physiology, pp.334-337, 2001). However, the contribution of nucleoside transporters in the human intestines, especially in the human small intestine has been not clarified in detail.

On the other hand, in the gazettes of Japanese patent publication no. 2001-163788 and Japanese patent no. 2632577, it has been reported that plasma uric acid level is lowered by inhibiting purine absorption. Additionally, it was confirmed that plasma uric acid level is lowered by restriction on eating dietary sources of purine in human, and that uric acid synthesized from purine nucleosides absorbed in the intestine reflects plasma uric acid concentration (for example, see Proceedings of the Nutrition Society, Vol.41, pp. 329-342, 1982). Therefore, plasma uric acid level can be controlled by effective inhibition of the purine nucleoside absorption through the intestines.

Some compounds including dipyridamole have been reported so far as an inhibitor of a nucleoside transporter (for example, see Japanese patent publication no.H6-247942, Japanese patent publication no. Tokuhyo 2002-504134, and Japanese patent publication no. Tokuhyo 2001-517226). All of these inhibitors are ENT inhibitors, and mainly used as a drug for, for example, the cardioprotection, treatment of pain or enhancement of antitumor drugs. On the other hand, there has not been any report on a CNT inhibitor so far. Moreover, it has not ever been reported or suggested that a compound having an inhibitory activity on CNT2 can inhibit the purine nucleoside absorption through the intestines effectively, and is useful as a drug for a disease associated with an abnormality of plasma uric acid level.

In addition, it was reported that as a glycosylated benzimidazole derivative, a benzimidazole derivative glycosylated with L-ribose is useful for the prevention or treatment of virus infection such as herpes virus or coronary restenosis. However, any benzimidazole derivative glycosylated with D-ribose has not been reported. Furthermore, it has not ever been reported or suggested that a glycosylated benzimidazole derivative is useful for the prevention or treatment of a disease associated with an abnormality of plasma uric acid level such as, for example, gout or hyperuricemia (see International publication no. WO97/25337 pamphlet, U.S. patent no.6,204,249 gazette, U.S. patent no. 6, 617, 315 gazette or the like).

### Disclosure of the Invention

The present inventors have studied earnestly on the nucleoside absorption in the human intestines. As a result, it was found that CNT2 is the most distributed in the human intestines, especially upper small intestines and that a phenylalkylamiobenzimidazole derivative which is glycosylated with, for example, D-ribose at its 1-position and also may have a substituent at its 2-position has an inhibitory activity on CNT2, and the purine nucleoside absorption in the body is inhibited by inhibiting CNT2. Thus, CNT2 is deeply involved in the purine nucleoside absorption, and since plasma uric acid level can be lowered by inhibiting CNT2, the above benzimidazole derivative having an inhibitory activity on CNT2 can be a novel drug for the prevention or treatment for a disease associated with an abnormality of plasma uric acid level by a mechanism completely different from that of the currently existing drugs, thereby forming the basis of the present invention.

The present inventors practiced cDNA cloning of human CNTs, firstly analyzed the distribution pattern of CNTs in human tissues and confirmed that CNT2 is expressed abundantly in the human small intestines. In addition, they analyzed the distribution pattern at each portion of digestive tract, and confirmed that CNT1 is expressed mostly in jejunum and ileum of the lower small intestines, and CNT2 is expressed mostly in the duodenum of the upper small intestines and next in the jejunum.

The present inventors further studied to find a compound having an inhibitory activity on CNT2, and finally confirmed that a benzimidazole derivative represented by the following general formula (I) has a strongly inhibitory activity on the uptake of adenosine in an experiment by using COS 7 cells transfected with a human CNT2 gene. In addition, in a purine tolerance test in rats, such a compound inhibits the increase of plasma uric acid level. Therefore, it was found that since a benzimidazole derivative represented by the following general formula (I) or a pharmaceutically acceptable salt thereof, or a prodrug thereof exerts an excellent inhibitory activity on CNT2 and inhibits the increase of plasma uric acid level markedly, the same is useful as a drug for the prevention or treatment of a disease associated with an abnormality of plasma uric acid level.

That is, the present invention relates to:
[1] a benzimidazole derivative represented by the general formula: 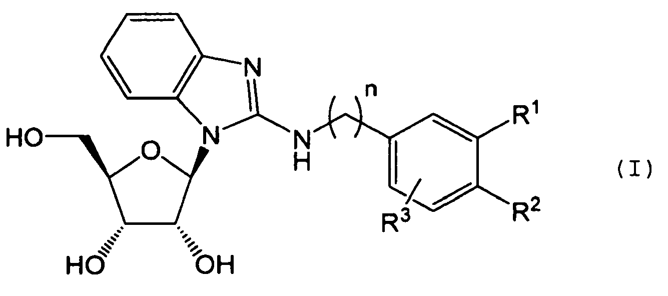 wherein
   n represents 1 or 2;
   R¹ and R² independently represents a hydrogen atom, a halogen atom, a cyano group, any of the following substituents (A) to (C) which may have the same or different 1 to 3 groups selected from a substituent group α, any of the following substituents (D) to (G) which may have the same or different 1 to 3 groups selected from substituent groups α or β, or any of the following substituents (H) to (M);
   R³ represents a hydrogen atom, a halogen atom, any of the following substituents (A) to (C) which may have the same or different 1 to 3 groups selected from a substituent group α, or any of the following substituents (H) to (M);
      (A) a C₁₋₆ alkyl group;
      (B) a C₂₋₆ alkenyl group;
      (C) a C₂₋₆ alkynyl group;
      (D) a C₃₋₈ cycloalkyl group;
      (E) a 3 to 10-membered cyclic heterocycloalkyl group;
      (F) a C₆₋₁₀ aryl group;
      (G) a 5 to 10-membered cyclic heteroaryl group;
      (H) OR⁷;
      (I) SR⁸;
      (J) NR⁹R¹⁰;
      (K) COOR¹¹;
      (L) CONR¹²R¹³;
      (M) NHCOR¹⁴
      (in the groups R⁷ to R¹⁴ independently represent a hydrogen atom, or any of the following substituents (N) to (P) which may have the same or different 1 to 3 groups selected from a substituent group α, or any of the following substituents (Q) to (V) which may have the same or different 1 to 3 groups selected from substituent groups α and β
      (N) a C₁₋₆ alkyl group;
      (O) a C₂₋₆ alkenyl group;
      (P) a C₂₋₆ alkynyl group;
      (Q) a C₃₋₈ cycloalkyl group;
      (R) a 3 to 10-membered cyclic heterocycloalkyl group;
      (S) a quaternary salt of a 3 to 10-membered cyclic nitrogen-containing heterocycloalkyl group;
      (T) a C₆₋₁₀ aryl group;
      (U) a 5 to 10-membered cyclic heteroaryl group;
      (V) a quaternary salt of a 5 to 10-membered cyclic nitrogen-containing heteroaryl group)
   and with the proviso that at least one of R¹, R² and R³ does not represent a group selected from a hydrogen atom, a halogen atom, a hydroxy group, a C₁₋₆ alkoxy group, NH₂ and COOH
   [Substituent group α]
      (a) a halogen atom;
      (b) a cyano group;
         any of the following substituents (c) to (h) which may have the same or different 1 to 3 groups selected from a substituent group γ, or any of the following substituents (i) to (v):
      (c) a C₃₋₈ cycloalkyl group;
      (d) a 3 to 10-membered cyclic heterocycloalkyl group;
      (e) a quaternary salt of a 3 to 10-membered cyclic nitrogen-containing heterocycloalkyl group;
      (f) a C₆₋₁₀ aryl group;
      (g) a 5 to 10-membered cyclic heteroaryl group;
      (h) a quaternary salt of a 5 to 10-membered cyclic nitrogen-containing heteroaryl group;
      (i) OR¹⁵;
      (j) SR¹⁶;
      (k) NR¹⁷R¹⁸;
      (l) N⁺R^{D}R^{E}R^{F};
      (m) COOR¹⁹;
      (o) NHCOR²⁰;
      (p) NHC(=NH)-NH₂;
      (q) C(=NH)-NH₂ (which is bound to a nitrogen atom of a nitrogen-containing heterocycloalkyl group);
      (r) NR²¹CONR²²R²³;
      (s) NR^{G}SO₂R^{H};
      (t) SO₂R^{I} (R^{I} represents a C₁₋₆ alkyl group, a C₂₋₆ alkenylene group or a hydroxy (C₁₋₆ alkyl) group);
      (u) CONR²⁴R²⁵;
      (v) SO₂NR²⁶R²⁷
      (in the groups R^{D-F} independently represent any of the following substituents (y1) to (y11) which may have the same or different 1 to 3 groups selected from a substituent group γ; R¹⁵, R¹⁶, R¹⁹⁻²¹ and R^{G-H} independently represent a hydrogen atom, or any of the following substituents (y1) to (y11) which may have the same or different 1 to 3 groups selected from a substituent group γ; R¹⁷, R¹⁸ and R²² to R²⁷ independently represent a hydrogen atom, or any of the following substituents (y1) to (y11) which may have the same or different 1 to 3 groups selected from a substituent group γ; or R¹⁷ and R¹⁸, R²² and R²³, R²⁴ and R²⁵, and R²⁶ and R²⁷ independently may bind together with the neighboring nitrogen atom to form a 3 to 8-membered aliphatic cyclic amino group
      (y1) a C₁₋₆ alkyl group;
      (y2) a C₂₋₆ alkenyl group;
      (y3) a C₂₋₆ alkynyl group;
      (y4) a C₃₋₈ cycloalkyl group;
      (y5) a 3 to 10-membered cyclic heterocycloalkyl group;
      (y6) a C₆₋₁₀ aryl group;
      (y7) a 5 to 10-membered cyclic heteroaryl group;
      (y8) a C₃₋₈ cycloalkyl-C₁₋₆ alkyl group;
      (y9) a 3 to 10-membered cyclic heterocycloalkyl-C₁₋₆ alkyl group;
      (y10) a C₆₋₁₀ aryl-C₁₋₆ alkyl group;
      (y11) a 5 to 10-membered cyclic heteroaryl-C₁₋₆ alkyl group)
   [Substituent group β]
      any of the following substituents (z1) to (z3) which may have the same or different 1 to 3 groups selected from a substituent group γ:
      (z1) a C₁₋₆ alkyl group;
      (z2) a C₂₋₆ alkenyl group;
      (z3) a C₂₋₆ alkynyl group
   [Substituent group γ]
      (1) a halogen atom;
      (2) a nitro group;
      (3) a cyano group;
      (4) OR²⁸;
      (5) SR²⁹;
      (6) NR³⁰R^{J} (R³⁰ and R^{J} independently represent a hydrogen atom, a C₁₋₆ alkyl group, a C₂₋₆ alkenyl group, a hydroxyl (C₁₋₆ alkyl) group, a C₆₋₁₀ aryl-C₁₋₆ alkyl group or a C₆₋₁₀ aryl group) ;
      (7) N⁺R^{K}R^{L}R^{M} (R^{K-M} independently represent a C₁₋₆ alkyl group, a C₂₋₆alkenyl group, a hydroxyl (C₁₋₆ alkyl) group, a C₆₋₁₂ aryl-C₁₋₆ alkyl group or a C₆₋₁₀ aryl group);
      (8) COR³¹;
      (9) COOR³²;
      (10) OCOR³³;
      (11) NHCOR³⁴;
      (12) NHC(=NH)-NH₂;
      (13) C(=NH)-NH₂ (which is bound to a nitrogen atom of a heterocycloalkyl group)
      (14) NR³⁵CONR³⁶R³⁷;
      (15) NR^{N}COOR^{O};
      (16) CONR³⁸R³⁹;
      (17) SO₂NR⁴⁰R⁴¹;
      (18) a hydroxyl(C₂₋₆ alkyl) group;
      (19) a 5 to 10-membered cyclic nitrogen-containing heteroaryl group
      (in the groups R²⁸, R²⁹, R³¹⁻³⁵, R^{N} and R^{O} independently represent a hydrogen atom, a C₁₋₆ alkyl group or a C₆₋₁₀ aryl-C₁₋₆ alkyl group; R³⁶ to R⁴¹ independently represent a hydrogen atom, or a C₁₋₆ alkyl group, or R³⁶ and R³⁷, R³⁸ and R³⁹, and R⁴⁰ and R⁴¹ independently may bind together with the neighboring nitrogen atom to form a 3 to 8-membered aliphatic cyclic amino group) ;
   or a pharmaceutically acceptable salt thereof; or a prodrug thereof wherein a group selected from a hydroxy group and an amino group of the benzimidazole derivative is substituted by a group selected from the group consisting of C₁₋₆ alkyl-CO-, C₁₋₆ alkyl-O-C₁₋₆ alkyl-CO-, C₁₋₆ alkyl-OCO-C₁₋₆ alkyl-CO-, C₁₋₆ alkyl-OCO- and C₁₋₆ alkyl-O-C₁₋₆ alkyl-OCO-;
[2] a benzimidazole derivative as defined in the above [1] wherein n represents 1, or a pharmaceutically acceptable salt thereof, or a prodrug thereof;
[3] a benzimidazole derivative as defined in the above [1] to [2] wherein R¹ and R³ independently represent a hydrogen atom, a halogen atom, any of the substituents (A) to (C) which may have the same or different 1 to 3 groups selected from the substituent group α, or any of the substituents (H) to (M), R² independently represents a hydrogen atom, a halogen atom, a cyano group, any of the substituents (A) to (C) which may have the same or different 1 to 3 groups selected from the substituent group α, any of the substituents (D) to (G) which may have the same or different 1 to 3 groups selected from the substituent groups a and β, or any of the substituents (H) to (M), or a pharmaceutically acceptable salt thereof, or a prodrug thereof;
[4] a benzimidazole derivative as defined in the above [3] wherein R¹ represents OR⁷ (with the proviso that R⁷ represents a C₁₋₆ alkyl group which has a hydroxy group, NR¹⁷R¹⁸ or N⁺R^{D}R^{E}R^{F} (R¹⁷, R¹⁸ and R^{D-F} have the same meanings as defined in the above [1])) or a hydroxy group; R² represents OR⁷ (with the proviso that R⁷ represents a C₁₋₆ alkyl group which has a hydroxy group, NR¹⁷R¹⁸ or N⁺R^{D}R^{E}R^{F} (R¹⁷, R¹⁸ and R^{D-F} have the same meanings as defined in the above [1])), a hydroxy group, or a C₆₋₁₀ aryl group which may have a hydroxy group or OR¹⁵ (R¹⁵ has the same meaning as defined in the above [1]); R³ represents a hydrogen atom, or a pharmaceutically acceptable salt thereof, or a prodrug thereof;
[5] a pharmaceutical composition comprising as an active ingredient a benzimidazole derivative as defined in any one of the above [1] to [4], or a pharmaceutically acceptable salt thereof, or a prodrug thereof;
[6] a pharmaceutical composition as defined in the above [5] for the prevention or treatment of a disease associated with an abnormality of plasma uric acid level;
[7] a pharmaceutical composition as defined in the above [6] wherein the disease associated with an abnormality of plasma uric acid level is a disease selected from gout, hyperuricemia, urinary lithiasis, hyperuricemic nephropathy and acute uric acid nephropathy;
[8] a pharmaceutical composition as defined in the above [6] wherein the disease associated with an abnormality of plasma uric acid level is gout;
[9] a pharmaceutical composition as defined in the above [6] wherein the disease associated with an abnormality of plasma uric acid level is hyperuricemia;
[10] a pharmaceutical composition as defined in any one of the above [5] to [9] comprising in combination as an active ingredient at least one agent selected from a group consisting of colchicine, a nonsteroidal anti-inflammatory agent, an adrenocortical steroid, a uric acid synthesis inhibitor, a uricosuric drug, a urinary alkalinizer and a uric acid oxidase;
[11] a pharmaceutical composition as defined in the above [10] wherein the nonsteroidal anti-inflammatory agent is indometacin, naproxen, fenbufen, pranoprofen, oxaprozin, ketoprofen, etoricoxib or tenoxicam; the uric acid synthesis inhibitor is allopurinol, oxypurinol, febuxostat or Y-700; the uricosuric drug is probenecid, bucolome or benzbromarone; the urinary alkalinizer is sodium hydrogen carbonate, potassium citrate or sodium citrate; the uric acid oxidase is rasburicase, uricase PEG-20, a recombinant uric acid oxidase (uricase).

In the compounds represented by the above general formula (I) of the present invention, the term "C₁₋₆ alkyl group" means a straight-chained or branched alkyl group having 1 to 6 carbon atoms such as, for example, a methyl group, an ethyl group, a propyl group, an isopropyl group, a butyl group, an isobutyl group, a *sec*-butyl group, a *tert-*butyl group, a pentyl group, an isopentyl group; a neopentyl group, a *tert-*pentyl group or a hexyl group; the term "C₂₋₆ alkenyl group" means a straight-chained or branched alkenyl group having 2 to 6 carbon atoms such as, for example, a vinyl group, an allyl group, a 1-propenyl group, an isopropenyl group; a 1-butenyl group, a 2-butenyl group or a 2-methylallyl group; the term "C₂₋₆ alkynyl group" means a straight-chained or branched alkenyl group having 2 to 6 carbon atoms such as, for example, an ethynyl group or a 2-propynyl group; the term "halogen atom" means a fluorine atom, a chlorine atom, a bromine atom or an iodine atom; and the term "hydroxy (C₁₋₆ alkyl) group" means the above C₁₋₆ alkyl group substituted by a hydroxy group.

The term "C₁₋₆ alkoxy group" means a straight-chained or branched alkoxy group having 1 to 6 carbon atoms such as, for example, a methoxy group, an ethoxy group, a propoxy group, an isopropoxy group, a butoxy group, an isobutoxy group, a *sec-*butoxy group, a *tert-*butoxy group, a pentyloxy group, an isopentyloxy group, a neopentyloxy group, a *tert-*pentyloxy group or a hexyloxy group, preferably a straight-chained alkoxy group such as, for example, a propoxy group or a butoxy group.

The term "C₃₋₈ cycloalkyl group" or "C₃₋₈ cycloalkyl" means a cyclopropyl group, a cyclobutyl group, a cyclopentyl group, a cyclohexyl group, a cycloheptyl group or a cyclooctyl group, for example, preferably a cyclopentyl group or a cyclohexyl group. The term "C₆₋₁₀ aryl group" or "C₆₋₁₀ aryl" means an aromacyclic hydrocarbon group having 6 or 10 carbon atoms such as, for example, a phenyl group or a naphthyl group, for example, preferably a phenyl group (for example, examples of a C₆₋₁₀ aryl-C₁₋₆ alkyl group include a benzyl group, a phenylethyl group, a naphthylmethyl group or a naphthylethyl group, preferably a benzyl group.

The term "3 to 10-membered cyclic heterocycloalkyl group" or "3 to 10-membered cyclic heterocycloalkyl" means a 3 to 10-membered monocyclic, polycyclic or bridged (for example, a 1-azabicyclo[2, 2, 2]octyl group or a 1,4-diazabicyclo[2,2,2]-octo-1-yl group) heterocycloalkyl group having 1 or 2 hetero atoms selected from an oxygen atom, a sulfur atom or a nitrogen atom in the ring, which may have 1 or 2 oxo group such as, for example, an aziridinyl group, an azetidinyl group, a morpholino group, a 2-morpholinyl group, a thiomorpholinyl group, a pyrrolidino group, a piperidino group, a 4-piperidinyl group, a 1-piperazinyl group or a 2-oxopyrrolidin-1-yl group or the above heterocycloalkyl group fused with a benzene ring (for example, a 1,3-dioxoisoindolin-2-yl group), for example, preferably a morpholino group, a 4-piperidinyl group, a 1-piperidinyl group, a 1-piperadinyl group, a 1-pyrrolidinyl group or a 1,3-dioxoisoindolin-2-yl group.

The term "3 to 10-membered cyclic nitrogen-containing heterocycloalkyl group" means the above 3 to 10-membered cyclic heterocycloalkyl group containing at least one nitrogen atom in the ring.

The term "3 to 8-membered aliphatic cyclic amino group" means a 3 to 8-membered cyclic amino group which may contain any hetero atom other than the nitrogen atom at the binding position selected from an oxygen atom, a sulfur atom and nitrogen atom in the ring, such as, for example, an aziridinyl group, an azetidinyl group, a morpholino group, a thiomorpholinyl group, a pyrrolidinyl group, a piperadinyl group or a 2-oxopyrrolidin-1-yl group, for example, preferably a 4-piperidinyl group, a 1-piperidinyl group, a 1-piperadinyl group or a 1-pyrrolidinyl group.

The term "5 to 10-membered cyclic heteroaryl group" or "5 to 10-membered cyclic heteroaryl" means a 5 or 6-membered cyclic aromatic heterocyclic group containing any 1 to 4 hetero atoms selected from an oxygen atom, a sulfur atom and a nitrogen atom in the ring, which is derived from, for example, thiazole, oxazole, isothiazole, isooxazole, pyridine, pyrimidine, pyrazine, pyridazine, pyrrole, furan, thiophene, imidazole, pyrazole, oxadiazole, thiadiazole, triazole, tetrazole or furazan or a 5 or 6-membered cyclic aromatic heterocyclic group containing any 1 to 4 hetero atoms selected from an oxygen atom, a sulfur atom and a nitrogen atom in the ring, fused with a 6-membered ring, which is derived from, for example, indole, isoindole, benzofuran, isobenzofuran, benzothiophene, benzooxazole, benzothiazole, benzoisooxazole, benzoisothiazole, indazole, benzoimidazole, quinoline, isoquinoline, phthalazine, quinoxaline, quinazoline, sinoline, indolizine, naphthyridine or pteridine.

The term "5 to 10-membered cyclic nitrogen-containing heteroaryl group" means the above 5 to 10-membered cyclic heteroaryl group containing at least one nitrogen atom in the ring, for example, preferably a group derived from pyridine or imidazole.

As quaternary salts, for example, a quaternary ammonium salt, a pyridinium salt and a piperadinium salt can be illustrated. In addition, as anion ligands of the same, for example, a fluoride, a chloride, a bromide, an iodide, a hydroxide, an acetate, a methanesulfonate, a trifluoromethanesulfonate, a p-toluenesulfonate, a sulfate, a tetrafluoroborate and a chlorochromate can be illustrated, and, for example, an iodide, a hydroxide, an acetate, a methanesulfonate and a sulfate are preferable.

In compounds represented by the above general formula (I) of the present invention, the above R¹, R² and R³ preferably represent OR⁷ and R⁷ represents a substituent (N) having (i) or (k) selected from the above substituent group α, the substituent (N) represents more preferably an alkyl group having 3 or 4 carbon atoms. R³ preferably represents a hydrogen atom.

The representative manufacture methods of the compounds represented by the above general formula (I) of the present invention are illustrated by way of the following examples. However, they are not limited thereto.

Of compounds represented by the above general formula (I) of the present invention, a compound wherein n is 1 (Ia) can be prepared, for example, according to the following methods 1 to 3, other methods described in literatures or similar methods to the same or the like (for example, International publication no. WO 97/25337 pamphlet, U.S. patent no. 6,204,249 gazette and U.S. patent no. 6,617,315 gazette). When a protective group is needed, any suitable introduction and elimination procedures can be optionally combined in the usual way.

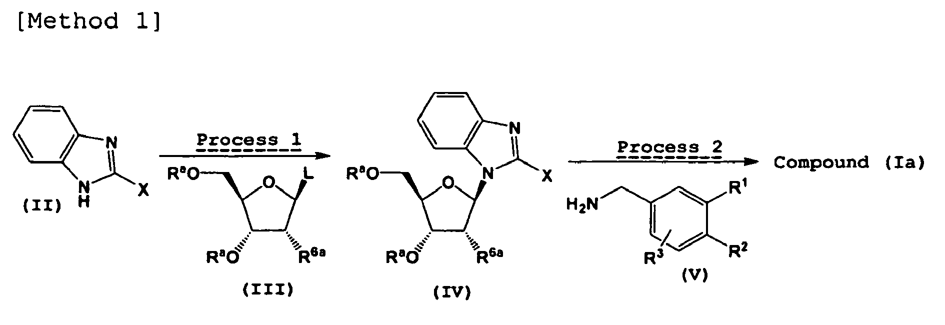

In the formula, R^{a} independently represents a hydroxy-protective group, R^{6a} represents a hydroxy group having a protective group, L represents a leaving group such as, for example, a halogen atom or an acetoxy group, X represents a leaving group such as, for example, a halogen atom or a toluenesulfonyloxy group, R¹ to R³ have the same meanings as defined above.

### Process 1

1) In a case that the substituent L of a sugar donor represented by the above general formula (III) is a halogen atom such as a bromine atom, a compound represented by the above general formula (IV) can be prepared by subjecting a benzimidazole derivative represented by the above general formula (II) to glycosidation in the presence of a base such as, for example, sodium hydride or potassium carbonate in an inert solvent, or 2) in a case that the substituent L of a sugar donor represented by the above general formula (III) is a leaving group such as an acetoxy group, a compound represented by the above general formula (IV) can be prepared by subjecting a benzimidazole derivative represented by the above general formula (II) to glycosidation in the presence of a Lewis acid such as, for example, trimethylsilyl trifluoromethanesulfonate, tin (IV) chloride or trifluoroborate in an inert solvent after pretreatment using a silylating agent such as, for example, *N,O*-bis(trimethylsilyl)acetamide, trimethylsilyl chloride or hexamethyldisilazane. As an inert solvent used in the glycosidation reaction, for example, *N,N*-dimethylacetamide, *N,N*-dimethylformamide, *N-*methylpyrrolidinone, dimethylsulfoxide, acetonitrile, 1,2-dichloroethane, tetrahydrofuran, or a mixed solvent thereof can be illustrated. The reaction temperature is usually from 0°C to reflux temperature, and the reaction time is usually from 30 minutes to 1 day, varying based on a used starting material, solvent and reaction temperature.

### Process 2

A compound represented by the above general formula (Ia) of the present invention can be prepared by subjecting a compound represented by the above general formula (IV) to condensation with a compound represented by the above general formula (V) in the presence or absence of a base such as, for example, sodium hydride, potassium carbonate, triethylamine or diisopropylethylamine in an inert solvent, and optionally by removing a protective group of the sugar moiety or the like according to a method used in general organic synthesis such as, for example, alkaline hydrogenation. As an inert solvent used in the condensation reaction, for example, toluene, tetrahydrofuran, dichloromethane, *N,N*-dimethylformamide, ethanol, isobutanol, water, or mixed solvent thereof can be illustrated. The reaction temperature is usually from room temperature to reflux temperature, and the reaction time is usually from 1 hour to 3 days, varying based on a used start material, solvent and reaction temperature.

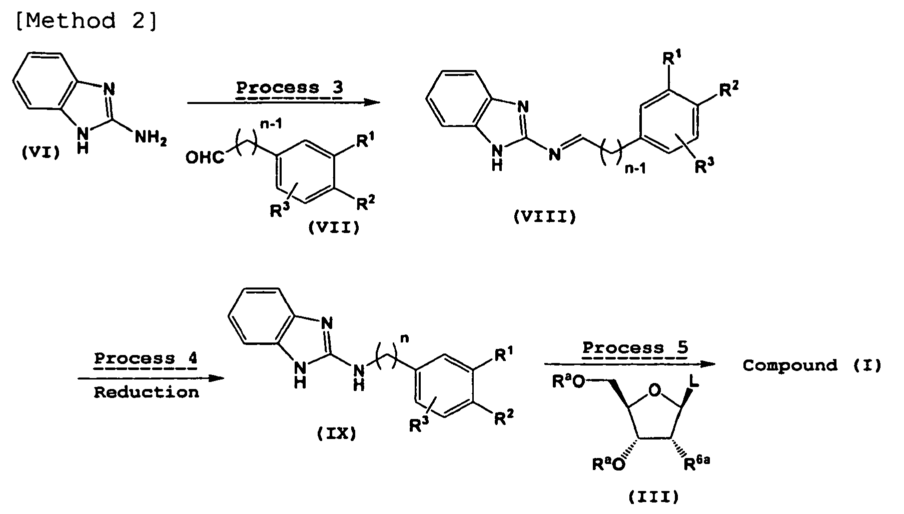

In the formula, L, R^{a}, R^{6a}, R¹ to R³ and n have the same meanings as defined above.

### Process 3

A compound represented by the above general formula (VIII) can be prepared by subjecting a 2-aminobenzimidazole derivative represented by the above formula (VI) to condensation with an aldehyde compound represented by the above general formula (VII) in the presence or absence of a base such as, for example, sodium acetate, sodium carbonate or sodium ethoxide or an acid such as, for example, acetic acid or methanesulfonic acid in an inert solvent. As an inert solvent used in the condensation reaction, for example, toluene, tetrahydrofuran, dichloromethane, *N,N*-dimethylformamide, ethanol, water, or a mixed solvent thereof can be illustrated. The reaction temperature is usually from room temperature to reflux temperature, and the reaction time is usually from 10 minutes to 1 day, varying based on a used starting material, solvent and reaction temperature.

### Process 4

A benzimidazole derivative represented by the above general formula (IX) can be prepared by reducing a compound represented by the above general formula (VIII) using a reducing agent such as, for example, lithium aluminum hydride or sodium borohydride in an inert solvent. As an inert solvent used in the reduction reaction, for example, toluene, tetrahydrofuran, dichloromethane, or a mixed solvent thereof can be illustrated. The reaction temperature is usually from -78°C to reflux temperature, and the reaction time is usually from 30 minutes to 1 day, varying based on a used starting material, solvent and reaction temperature.

### Process 5

1) In a case that the substituent L of a sugar donor represented by the above general formula (III) is a halogen atom such as a bromine atom, a compound represented by the above general formula (I) of the present invention can be prepared by subjecting a benzimidazole derivative represented by the above general formula (IX) to glycosidation in the presence of a base such as, for example, sodium hydride or potassium carbonate in an inert solvent, or 2) in a case that the substituent L of a sugar donor represented by the above general formula (III) is a leaving group such as an acetoxy group, a compound represented by the above general formula (I) of the present invention can be prepared by subjecting a benzimidazole derivative represented by the above general formula (IX) to glycosidation in the presence of a Lewis acid such as, for example, as trimethylsilyl trifluoromethanesulfonate, tin (IV) chloride or trifluoroborate in an inert solvent after pretreatment using a silylating agent such as, for example, *N,O*-bis(trimethylsilyl)acetamide, trimethylsilyl chloride or hexamethyldisilazane, and optionally by removing a protective group of the sugar moiety or the like according to a method used in general organic synthesis such as, for example, alkaline hydrogenation. As an inert solvent used in the glycosidation reaction, for example, *N,N*-dimethylacetamide, *N,N*-dimethylformamide, *N*-methylpyrrolidinone, dimethylsulfoxide, acetonitrile, 1,2-dichloroethane, tetrahydrofuran, or a mixed solvent thereof can be illustrated. The reaction temperature is usually from 0°C to reflux temperature, and the reaction time is usually from 30 minutes to 1 day, varying based on a used starting material, solvent and reaction temperature.

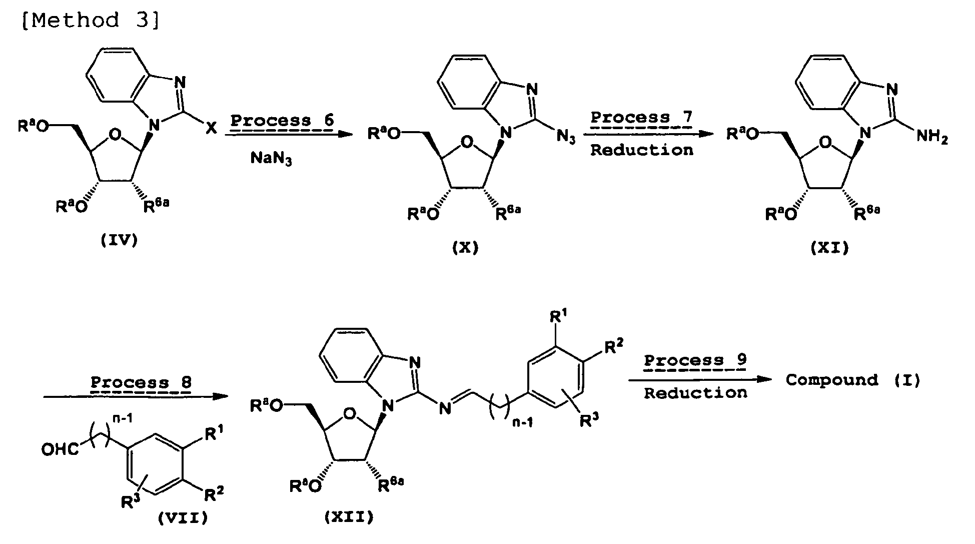

In the formula X, R^{a}, R^{6a}, R¹ to R³ and n have the same meanings as defined above.

### Process 6

A compound represented by the above general formula (X) can be prepared by subjecting a compound represented by the above general formula (IV) to azidation using an aziding reagent such as, for example, sodium azide or lithium azide in an inert solvent. As an inert solvent used in the azidation reaction for example, toluene, tetrahydrofuran, dichloromethane, *N,N*-dimethylformamide, ethanol, isobutanol, water, or a mixed solvent thereof can be illustrated. The reaction temperature is usually from room temperature to reflux temperature, and the reaction time is usually from 1 hour to 3 days, varying based on a used starting material, solvent and reaction temperature.

### Process 7

A compound represented by the above general formula (XI) can be prepared by subjecting a compound represented by the above general formula (X) to catalytic reduction using a metal catalyst such as, for example, palladium-carbon powder or platinum oxide in the presence or absence of an acid such as hydrochloric acid in an inert solvent. As an inert solvent used in the catalytic reduction reaction, for example, methanol, ethanol, tetrahydrofuran, ethyl acetate, acetic acid, or a mixed solvent thereof can be illustrated. The reaction temperature is usually from room temperature to reflux temperature, and the reaction time is usually from 30 minutes to 1 day, varying based on a used starting material, solvent and reaction temperature.

### Process 8

A compound represented by the above general formula (XII) can be prepared by subjecting a 2-aminobenzimidazole derivative represented by the above general formula (XI) to condensation with an aldehyde compound represented by the above general formula (VII) in the presence or absence of a base such as, for example, sodium acetate, sodium carbonate or sodium ethoxide or an acid such as, for example, acetic acid or methanesulfonic acid in an inert solvent. As an inert solvent used in the condensation reaction, for example, toluene, tetrahydrofuran, dichloromethane, *N,N*-dimethylformamide, ethanol, water, or a mixed solvent thereof can be illustrated. The reaction temperature is usually from room temperature to reflux temperature, and the reaction time is usually from 30 minutes to 1 day, varying based on a used starting material, solvent and reaction temperature.

### Process 9

A compound represented by the above general formula (I) of the present invention can be prepared by subjecting compound represented by the above general formula (XII) to reduction using a reducing agent such as, for example, lithium aluminum hydride or sodium borohydride, and optionally by removing the protective group at the sugar moiety or the like in accordance with a method used in general organic synthesis such as, for example, alkaline hydrogenation in an inert solvent. As an inert solvent used in the reduction reaction, for example, toluene, tetrahydrofuran, dichloromethane, acetic acid, or a mixed solvent thereof can be illustrated. The reaction temperature is usually from -78°C to reflux temperature, and the reaction time is usually from 30 minutes to 1 day, varying based on a used starting material, solvent and reaction temperature.

In addition, a compound represented by the above general formula (I) of the present invention can be also prepared, for example, in accordance with the following or similar method or in combination with the same. In a case that a protective group is necessary, introduction and removal procedures can be optionally combined in the usual way.

Among the compounds represented by the above general formula (I) of the present invention, a compound wherein at least one of R¹ to R³ is OR⁷, SR⁸ or NR⁹R¹⁰ (with the proviso that at least one of R⁷, R⁸ and R⁹/R¹⁰ is not a hydrogen atom), or the above substituent (A) to (G) having OR¹⁵, SR¹⁶, NR¹⁷R¹⁸ or NR⁺R^{D}R^{E}R^{F} (with the proviso that at least one of R¹⁵, R¹⁶ and R¹⁷/R¹⁸ is not a hydrogen atom) can be prepared by subjecting a compound wherein the corresponding group is a hydroxy group, a thiol group or an amino group, or any of the above substituents (A) to (G) having a hydroxy group, a thiol group or an amino group to alkylation using an alkylating agent such as, for example, a corresponding halogenated alkyl compound in the presence of a base such as, for example, sodium hydroxide, potassium carbonate, triethylamine or diisopropylethylamine in an inert solvent optionally in the presence of a catalytic amount of sodium iodide. As an inert solvent used in the alkylation reaction, for example, methanol, ethanol, tetrahydrofuran, *N,N*-dimethylformamide, water, or a mixed solvent thereof can be illustrated. The reaction temperature is usually from room temperature to reflux temperature, and the reaction time is usually from 10 minutes to 1 day, varying based on a used starting material, solvent and reaction temperature.

Among the compounds represented by the above general formula (I) of the present invention, a compound wherein at least one of R¹ to R³ is OR⁷ or COOR¹¹ (with the proviso that neither R⁷ nor COOR¹¹ is a hydrogen atom), or any of the above subst ituents (A) to (G) having OR¹⁵ or COOR¹⁹ (with the proviso that neither R¹⁵ nor R¹⁹ is not a hydrogen atom) can be prepared by subjecting a compound wherein the corresponding group is a hydroxy group or a carboxy group, or any of the above substituents (A) to (G) having a hydroxy group or a carboxy group to condensation using a corresponding alcohol compound in the presence of Mitsunobu reagent such as, for example, diethyl azodicarboxylate or diisopropyl azodicarboxylate and an organic phosphorus reagent such as, for example, triphenylphosphine in an inert solvent. As an inert solvent used in the condensation reaction, for example, toluene, tetrahydrofuran, *N,N*-dimethylformamide, or a mixed solvent thereof can be illustrated. The reaction temperature is usually from room temperature to reflux temperature, and the reaction time is usually from 10 minutes to 1 day, varying based on a used starting material, solvent and reaction temperature.

Among the compounds represented by the above general formula (I) of the present invention, a compound wherein at least one of R¹ to R³ is CONR¹²R¹³, or any of the above substituents (A) to (G) having CON²⁴R²⁵ can be prepared by subjecting an amine compound wherein the corresponding group is a carboxy group, or any of the above substituents (A) to (G) having a carboxy group to amidation, using a corresponding amine compound and a condensing agent such as, for example, diphenylphospholylazide or dicyclohexylcarbodiimide in an inert solvent, optionally in the presence of an activated esterifing reagent such as, for example, 1-hydroxybenzotriazole. As an inert solvent used in the amidation reaction, for example, toluene, tetrahydrofuran, *N,N*-dimethylformamide, or a mixed solvent thereof can be illustrated. The reaction temperature is usually from room temperature to reflux temperature, and the reaction time is usually from 10 minutes to 1 day, varying based on a used starting material, solvent and reaction temperature.

Among the compounds represented by the above general formula (I) of the present invention, a compound wherein at least one of R¹ to R³ is a C₂₋₆ alkenyl group or a C₂₋₆ alkynyl group can be prepared by subjecting a compound wherein the corresponding group is a halogen atom to condensation using a corresponding alkene or alkyne compound in the presence of a palladium catalyst such as, for example, palladium acetate, an organic phosphorus ligand such as, for example, triphenylphosphine and a base such as, for example, cesium carbonate or sodium *tert*-butoxide in an inert solvent. As an inert solvent used in the condensation reaction, for example, toluene, tetrahydrofuran, *N,N*-dimethylformamide, or a mixed solvent thereof can be illustrated. The reaction temperature is usually from room temperature to reflux temperature, and the reaction time is usually from 10 minutes to 1 day, varying based on a used starting material, solvent and reaction temperature.

Among the compounds represented by the above general formula (I) of the present invention, a compound wherein at least one of R¹ to R³ is a C₂₋₆ alkenyl group, a C₂₋₆ alkynyl group, a C₃₋₈ cycloalkyl group, a C₆₋₁₀ aryl group or a 5 to 10-membered cyclic heteroaryl group can be prepared by subjecting a compound wherein the corresponding group is a halogen atom to condensation with a corresponding boric acid compound in the presence of a base such as, for example, cesium carbonate or sodium *tert*-butoxide and in the presence of a catalyst such as, for example, tetrakis(triphenylphosphine)palladium and a ligand such as, for example, 2,2'-bis(diphenylphosphino)-1,1'-binaphthyl in an inert solvent. As an inert solvent used in the condensation reaction, for example, toluene, tetrahydrofuran, *N,N*-dimethylformamide, or a mixed solvent thereof can be illustrated. The reaction temperature is usually from room temperature to reflux temperature, and the reaction time is usually from 10 minutes to 1 day, varying based on a used starting material, solvent and reaction temperature.

Among the compounds represented by the above general formula (I) of the present invention, a compound wherein at least one of R¹ to R³ is a group having an acylamino group, an alkoxycarbonylamino group, a sulfonylamino group or an ureido group can be prepared by subjecting a compound having an amino group to reaction using an acylating agent such as, for example, a corresponding acylhalide derivative, a carbamating agent such as, for example, chloroformate compound, a sulfonylating agent such as, for example, a sulfonylhalide compound, an agent to introduce into an ureide such as, for example, an isocyanate compound in the presence or absence of a base such as, for example, sodium hydroxide, pyridine, triethylamine or diiisopropylethylamine in an inert solvent. As an inert solvent used in each reaction, forexample, tetrahydrofuran, *N,N*-dimethylformamide, water, or a mixed solvent thereof can be illustrated. The reaction temperature is usually from room temperature to reflux temperature, and the reaction time is usually from 10 minutes to 1 day, varying based on a used starting material, solvent and reaction temperature.

Compounds represented by the above general formula (II) and (VI) used as a starting material in the above-mentioned production processes are commercially available or can be prepared in a known or similar method or the like, and the following method, for example, can be illustrated. In a case that a protective group is necessary, introduction or removal procedures can be optionally combined in the usual way.

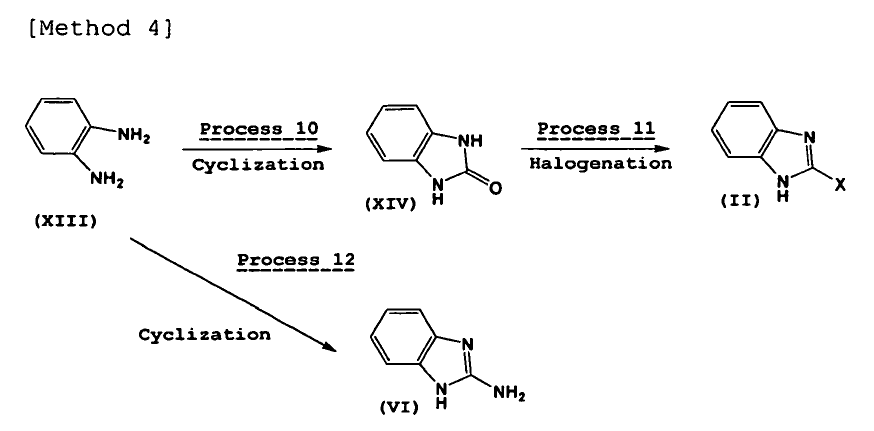

In the formula, X has the same meaning as defined above.

### Process 10

A compound represented by the above formula (XIV) can be prepared by subjecting a compound represented by the above formula (XIII) to cyclixation using a reagent such as, for example, phosgene or carbodiimidazole in the presence or absence of a base such as, for example, sodium carbonate, triethylamine or pyridine in an inert solvent. As an inert solvent used in the cyclization reaction, for example, tetrahydrofuran, dichloromethane, acetic acid, toluene, *N,N*-dimethylformamide, or a mixed solvent thereof can be illustrated. The reaction temperature is usually from 0°C to reflux temperature, and the reaction time is usually from 10 minutes to 1 day, varying based on a used starting material, solvent and reaction temperature.

### Process 11

A compound represented by the above general formula (II) can be prepared by subjecting a compound represented by the above formula (XIV) to halogenation using an acid halogenating reagent such as, for example, thionylchloride, phosphorus trichloride, phosphorus pentachloride, phosphorus oxychloride, phosphorus tribromide or fluorosulfuric acid without or in an inert solvent. As an inert solvent used in the halogenation reaction, for example, toluene, dichloromethane, or a mixed solvent thereof can be illustrated. The reaction temperature is usually from -78°C to reflux temperature, and the reaction time is usually from 30 minutes to 1 day, varying based on a used starting material, solvent and reaction temperature.

### Process 12

A compound represented by the above formula (VI) can be prepared by subjecting a compound represented by the above formula (XIII) to cyclization using a reagent such as, for example, cyanogen bromide in an inert solvent. As an inert solvent used in the cyclization reaction, for example, tetrahydrofuran, dichloromethane, acetonitrile, toluene, or a mixed solvent thereof can be illustrated. The reaction temperature is usually from 0°C to reflux temperature, and the reaction time is usually from 10 minutes to 1 day, varying based on a used starting material, solvent and reaction temperature.

Among the compounds represented by the above general formula (IX) used in the above-mentioned Method 2, a compound wherein n is 1 can be prepared in a known or similar method or the like, and the following method, for example, can be illustrated. In a case that a protective group is necessary, introduction or removal procedures can be optionally combined in the usual way.

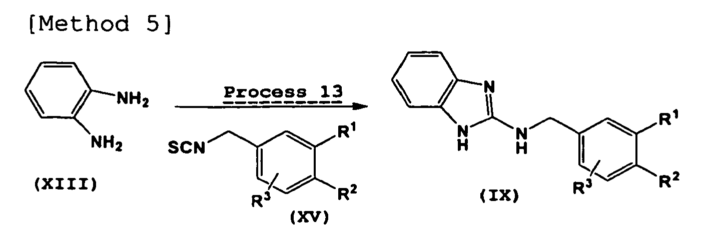

In the formula, R¹ to R³ have the same meanings as defined above.

### Process 13

A compound represented by the above general formula (IX) can be prepared by allowing a compound represented by the above formula (XIII) to react with a thioisocyanate derivative represented by the above general formula (XV) in the presence or absence of a base such as, for example, triethylamine, sodium carbonate or pyridine without or in an inert solvent. As an inert solvent used in the reaction, for example, toluene, tetrahydrofuran, dichloromethane, *N,N*-dimethylformamide, ethanol, water, or a mixed solvent thereof can be illustrated. The reaction temperature is usually from room temperature to reflux temperature, and the reaction time is usually from 10 minutes to 1 day, varying based on a used starting material, solvent and reaction temperature.

A compound represented by the above general formula (V) used as a starting material in the above-mentioned Method 1 is commercially available or can be prepared in a known or similar method or the like, and the following method, for example, can be illustrated In a case that a protective group is necessary, introduction or removal procedures can be optionally combined in the usual way.

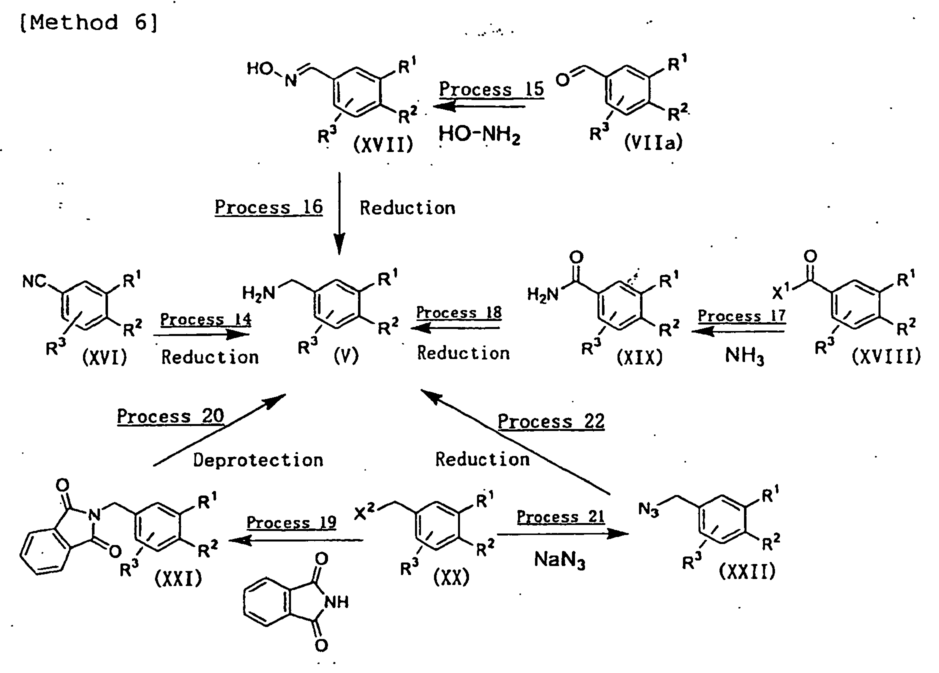

In the formula, X¹ represents a halogen atom, X² represents a halogen atom and R¹ to R³ have the same meanings as defined above.

### Process 14

A compound represented by the above general formula (V) can be prepared by subjecting a compound represented by the above general formula (XVI) according to a general reduction methodofanitrile, forexample, 1) to reduction using a reducing agent such as, for example lithium aluminum hydride or diisobutylalminum hydride in an inert solvent, or 2) to catalytic reduction using a metal catalyst such as, for example, palladium-carbon powder or platinum oxide in the presence or absence of an acid such as, for example, hydrochloric acid in an inert solvent. As a solvent used in the reduction reaction 1), for example, toluene, tetrahydrofuran, dichloromethane, or a mixed solvent thereof can be illustrated. The reaction temperature is usually from -78°C to reflux temperature, and the reaction time is usually from 30 minutes to 1 day, varying based on a used starting material, solvent and reaction temperature. As a solvent used in the reduction reaction 2), for example, methanol, ethanol, tetrahydrofuran, ethyl acetate, acetic acid, or a mixed solvent thereof can be illustrated. The reaction temperature is usually from room temperature to reflux temperature, and the reaction time is usually from 30 minutes to 1 day, varying based on a used starting material, solvent and reaction temperature.

### Process 15

A corresponding oxime represented by the above general formula (XVII) can be prepared by allowing a compound represented by the above.general formula (VIIa) to react with hydroxylamine in an inert solvent . As an inert solvent used in the reaction, for example, toluene, tetrahydrofuran, dichloromethane, or a mixed solvent thereof can be illustrated. The reaction temperature is usually from -78°C to reflux temperature, and the reaction time is usually from 30 minutes to 1 day, varying based on a used starting material, solvent and reaction temperature.

### Process 16

A compound represented by the above general formula (V) can be prepared by subjecting a compound represented by the above general formula (XVII) according to a general reduction method of an oxime, for example, 1) to reduction using a reducing agent such as, for example, lithium aluminum hydride or diisobutylalminum hydride in an inert solvent, or 2) to catalytic reduction using a metal catalyst such as, for example, palladium-carbon powder or platinum oxide in the presence or absence of an acid such as, for example, hydrochloric acid in an inert solvent. As a solvent used in the reduction reaction 1), for example, toluene, tetrahydrofuran, dichloromethane, or a mixed solvent thereof can be illustrated. The reaction temperature is usually from -78 °C to reflux temperature, and the reaction time' is usually from 30 minutes to 1 day, varying based on a used starting material, solvent and reaction temperature. As a solvent used in the reduction reaction 2), for example, methanol, ethanol, tetrahydrofuran, ethyl acetate, acetic acid, or a mixed solvent thereof can be illustrated. The reaction temperature is usually from room temperature to reflux temperature, and the reaction time is usually from 30 minutes to 1 day, varying based on a used starting material, solvent and reaction temperature.

### Process 17

A compound represented by the above general formula (XIX) can be prepared by allowing a compound represented by the above general formula (XVIII) to react with ammonia without or in an inert solvent. As an inert solvent used in the reaction, for example, toluene, dichloromethane, or a mixed solvent thereof can be illustrated. The reaction temperature is usually from-78°C to reflux temperature, and the reaction time is usually from 30 minutes to 1 day, varying based on a used starting material, solvent and reaction temperature.

### Process 18

A compound represented by the above general formula (V) can be prepared by subjecting a compound represented by the above general formula (XIX) according to a general reduction method of a carbamoyl group, for example, to reduction using a reducing agent such as, for example, borane-dimethylsulfide complex, borane-tetrahydrofuran complex, lithium aluminum hydride or diisobutylalminum hydride in an inert solvent. As an inert solvent used in the reduction reaction, for example, toluene, tetrahydrofuran, dichloromethane, or mixed solvent thereof can be illustrated. The reaction temperature is usually from -78°C to reflux temperature, and the reaction time is usually from 30 minutes to 1 day, varying based on a used starting material, solvent and reaction temperature.

### Process 19

A compound represented by the above general formula (XXI) can be prepared by allowing a compound represented by the above general formula (XX) to react with a phthalimide or a salt thereof in the presence or absence of a base such as, for example, sodium hydride, sodium carbonate or potassium hydroxide in an inert solvent. As an inert solvent used in the reaction, for example, toluene, tetrahydrofuran, dichloromethane, *N,N*-dimethylformamide, ethanol, or a mixed solvent thereof can be illustrated. The reaction temperature is usually from room temperature to reflux temperature, and the reaction time is usually from 10 minutes to 1 day, varying based on a used starting material, solvent and reaction temperature.

### Process 20

A compound represented by the above general formula (V) can be prepared by subjecting a compound represented by the above general formula (XXI) according to a general deprotection method of a phthalimide, for example, to deprotection using, for example, methylamine or hydrazine in an inert solvent. As an inert solvent used in the deprotection reaction, for example, toluene, tetrahydrofuran, or a mixed solvent thereof can be illustrated. The reaction temperature is usually from -78°C to reflux temperature, and the reaction time is usually from 30 minutes to 1 day, varying based on a used starting material, solvent and reaction temperature.

### Process 21

A compound represented by the above general formula (XXII) can be prepared by allowing a compound represented by the above general formula (XX) to react with an azidating reagent such as, for example, sodium azide or lithium azide in an inert solvent. As an inert solvent used in the azidation reaction, for example, toluene, tetrahydrofuran, *N,N*-dimethylformamide, ethanol, or a mixed solvent thereof can be used. The reaction temperature is usually from room temperature to reflux temperature, and the reaction time is usually from 10 minutes to 1 day, varying based on a used starting material, solvent and reaction temperature.

### Process 22

A compound represented by the above general formula (V) can be prepared by subjecting a compound represented by the above general formula (XXII) according to a general reduction method of an azide, for example, 1) to reduction using a reducing agent such as, for example, lithium aluminum hydride or diisobutylalminum hydride in an inert solvent, or 2) to catalytic reduction using a metal catalyst such as, for example, palladium-carbon powder or platinum oxide in the presence or absence of an acid such as, for example, hydrochloric acid in an inert solvent. As an inert solvent used in the reduction reaction 1), for example, toluene, tetrahydrofuran, dichloromethane, or mixed solvent thereof can be illustrated. The reaction temperature is usually from -78°C to reflux temperature, and the reaction time is usually from 30 minutes to 1 day, varying based on a used starting material, solvent and reaction temperature. As a solvent used in the reduction reaction 2), for example, methanol, ethanol, tetrahydrofuran, ethyl acetate, acetic acid, or a mixed solvent thereof can be illustrated. The reaction temperature is usually from room temperature to reflux temperature, and the reaction time is usually from 30 minutes to 1 day, varying based on a used starting material, solvent and reaction temperature.

Among the compounds of the above general formula (VII) used in the above-mentioned Method 2 or 3, a compound represented by a general formula (VIIa) wherein n is 1 and a compound represented by the above general formula (XVI), (XVIII) or (XX) used in Method 6 is commercially available or can be prepared in a known or similar method or the like (J. Med. Chem. , Vol.46, pp.1845-1857, 2003; Synthesis, Vol.17, pp.2503-2512, 2002 or the like), and as the preparation methods, the following Methods 7 to 10, for example, can be illustrated. In a case that a protective group is necessary, introduction or removal procedures can be optionally combined in the usual way.

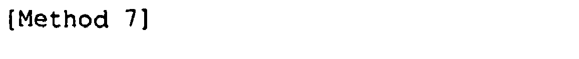

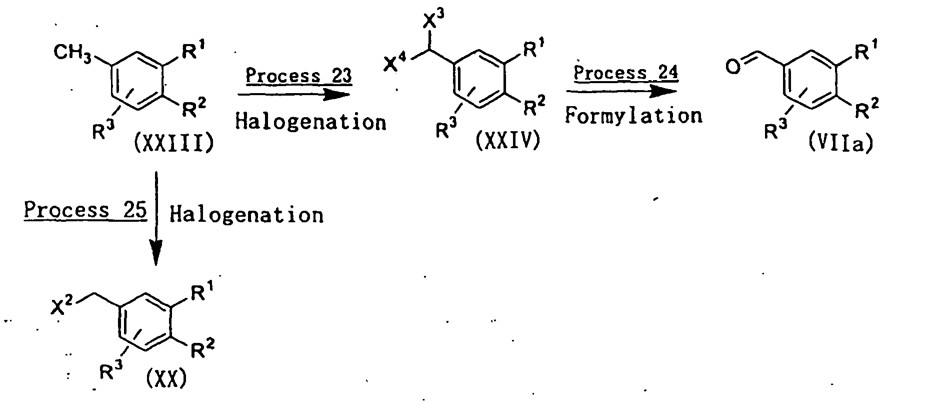

In the formula, X³ and X⁴ represent a halogen atom, and R¹ to R³ and X³ have the same meanings as defined above.

### Process 23

A compound represented by the above general formula (XXIV) can be prepared by subjecting a compound represented by the above general formula (XXIII) to halogenation using a halogenating reagent such as, for example, *N*-chlorosuccinimide or *N*-bromosuccinimide, and optionally using an initiating agent such as, for example, benzoyl peroxide or α,α'-azobisisobutylonitrile in an inert solvent. As an inert solvent used in the halogenation reaction, for example, tetrahydrofuran, dichloromethane, acetic acid, toluene, *N,N*-dimethylformamide, or mixed solvent thereof can be illustrated. The reaction temperature is usually from 0°C to reflux temperature, and the reaction time is usually from 10 minutes to 1 day, varying based on a used starting material, solvent and reaction temperature.

### Process 24

A formyl compound represented by the above general formula (VIIa) can be prepared by allowing a compound represented by the above general formula (XXIV) to react with a reagent such as, for example, silver nitrate in methanol and then by treating the same with an aqueous solution of hydrochloric acid or nitric acid. The reaction temperature is usually from room temperature to reflux temperature, and the reaction time is usually from 1 hour to 1 day, varying based on a used starting material, solvent and reaction temperature.

### Process 25

A compound represented by the above general formula (XX) can:be prepared by subjecting a compound represented by the above general formula (XXIII) to halogenation using a halogenating reagent such as, for example, *N*-chlorosuccinimide or *N*-bromosuccinimide, and optionally using an initiating agent such as, for example, benzoyl peroxide or α,α'-azobisisobutylonitrile in an inert solvent. As an inert solvent used in the halogenation reaction, for example, tetrahydrofuran, dichloromethane, acetic acid, toluene, *N,N*-dimethylformamide, or a mixed solvent thereof can be illustrated. The reaction temperature is usually from 0°C to reflux temperature, and the reaction time is usually from 10 minutes to 1 day, varying based on a used starting material, solvent and reaction temperature.

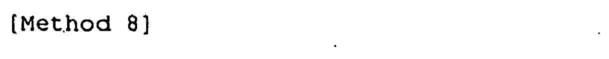

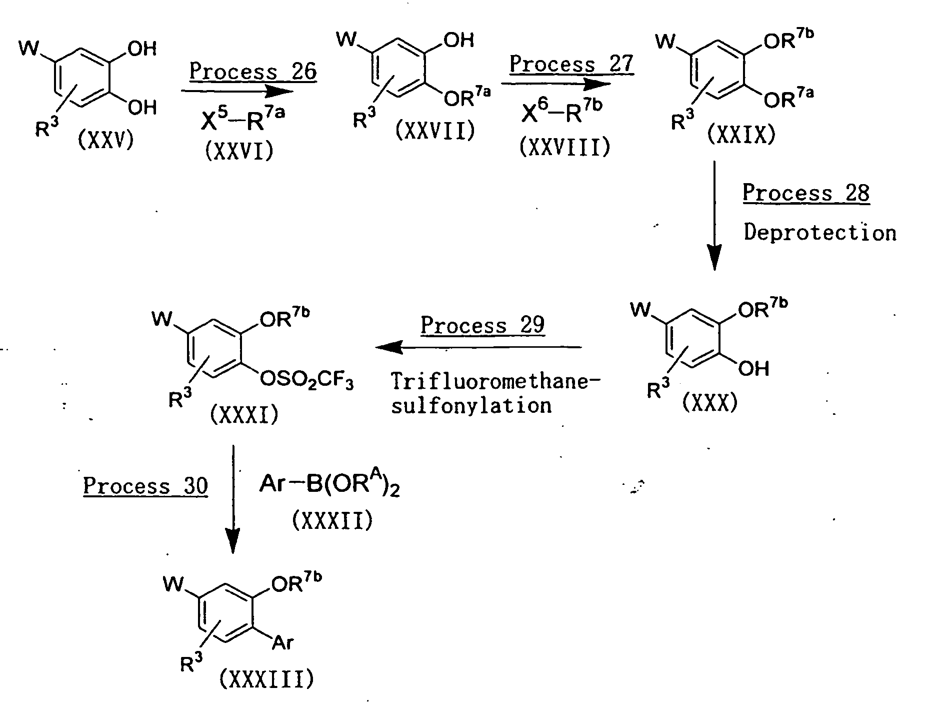

In the formula, R^{7a} represents a hydroxy-protective group or has the same meaning to R⁷; R^{7b} has the same meaning to R⁷; X⁵ and X⁶ independently represent a halogen atom; Ar represents a C₂₋₆ alkenyl group, a C₂₋₆ alkynyl group, a C₃₋₈ cycloalkyl group, a C₆₋₁₀ aryl group or a 5 to 10-membered cyclic heteroaryl group; R^{A} represents a hydrogen atom or a C₁₋₆ alkyl group; W represents a formyl group, a cyano group or a carbamoyl group; and R³ has the same meaning as defined above.

### Process 26

A compound represented by the above general formula
(XXVII) can be prepared by subjecting a compound represented by the above general formula (XXV) to *O*-alkylation using an alkylating agent or an agent to introduce a hydroxy-protective group represented by the above general formula (XXVI) in the presence of a base such as, for example, sodiumhydroxide, potassiumcarbonate, triethylamine or diisopropylethylamine, optionally in the presence of a catalytic amount of sodium iodide in an inert solvent. As an agent to introduce a hydroxy-protective group, for example, benzyl bromide or chloromethylmethyl ether can be illustrated. As an inert solvent used in the *O-*alkylation reaction, for example, methanol, ethanol, tetrahydrofuran, *N,N*-dimethylformamide, water, or a mixed solvent thereof can be illustrated. The reaction temperature is usually from room temperature to reflux temperature, and the reaction time is usually from 10 minutes to 1 day, varying based on a used starting material, solvent and reaction temperature.

### Process 27

A compound represented by the above general formula (XXIX) can be prepared by subjecting a compound represented by the above general formula (XXVII) to *O-*alkylation using an alkylating agent represented by the above general formula (XXVIII) in the presence of a base such as, for example, sodium hydroxide, potassium carbonate, triethylamine or diisopropylethylamine, optionally in the presence of a catalytic amount of sodium iodide in an inert solvent. As an inert solvent used in the *O-*alkylation reaction, for example, methanol, ethanol, tetrahydrofuran, *N,N*-dimethylformamide, water, or a mixed solvent thereof can be illustrated. The reaction temperature is usually from room temperature to reflux temperature, and the reaction time is usually from 10 minutes to 1 day, varying based on a used starting material, solvent and reaction temperature.

### Process 28

A compound represented by the above general formula (XXX) can be prepared by removing a hydroxy-protective group of a compound represented by the above general formula (XXIX) wherein R^{7a} represents a hydroxy-protective group in the usual way. For example, in a case that the protective group is a benzyl group, a compound represented by the above general formula (XXX) can be prepared by catalytic reduction using a metal catalyst such as, for example, palladium-carbon powder in the presence or absence of an acid such as, for example, hydrochloric acid in an inert solvent. As an inert solvent used in the catalytic reduction reaction, for example, methanol, ethanol, tetrahydrofuran, ethyl acetate, acetic acid, or a mixed solvent thereof can be illustrated. The reaction temperature is usually from room temperature to reflux temperature, and the reaction time is usually from 30 minutes to 1 day, varying based on a used starting material, solvent and reaction temperature.

### Process 29

A compound represented by the above general formula (XXXI) can be prepared by allowing a compound represented by the above general formula (XXX) to react with a reagent to drive into a trifluoromethanesulfonyl compound such as, for example, trifluoromethanesulfonic anhydride in the presence of a base such as, for example pyridine, triethylamine or diisopropylethylamine in an inert solvent. As an inert solvent used in the reaction, for example, toluene, tetrahydrofuran, *N,N*-dimethylformamide, dichloromethane, or a mixed solvent thereof can be illustrated. The reaction temperature is usually from 0°C to reflux temperature, and the reaction time is usually from 10 minutes to 1 day, varying based on a used starting material, solvent and reaction temperature.

### Process 30

A compound represented by the above general formula (XXXIII) can be prepared by subjecting a compound represented by the above general formula (XXXI) to condensation with a boric acid compound represented by the above general formula (XXXII) using a catalyst such as, for example, tris(dibenzylidenacetone)dipalladium and a ligand such as, for example, 2,2'-bis(diphenylphosphino)-1,1'-binaphthyl in the presence of a base such as, for example, cesium carbonate or sodium *tert*-butoxide in an inert solvent. As an inert solvent used in the condensation reaction, forexample, *N,N*-dimethylacetamide, toluene, tetrahydrofuran, or a mixed solvent thereof can be illustrated. The reaction temperature is usually from room temperature to reflux temperature, and the reaction time is usually from 1 hour to 1 day, varying based on a used starting material, solvent and reaction temperature.

In the Method 8, an example is described regarding a compound wherein R¹ represents OR⁷. A raw material of a compound represented by the above general formula (I) wherein at least once of R¹ to R³ has SR⁸ or NR⁹R¹⁰ can be also prepared in a similar or known method described in literatures or the like (for example, a method by Melvin et.al.,: Journal of Organic Chemistry, 31, pp.3980-3984, 1996) (similarly in the following Methods 9 and 10). In addition, in a case that a protective group is necessary in a compound wherein R¹ represents NR⁹NR¹⁰, introduction and removal procedures can be optionally combined in the usual way.

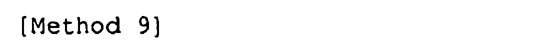

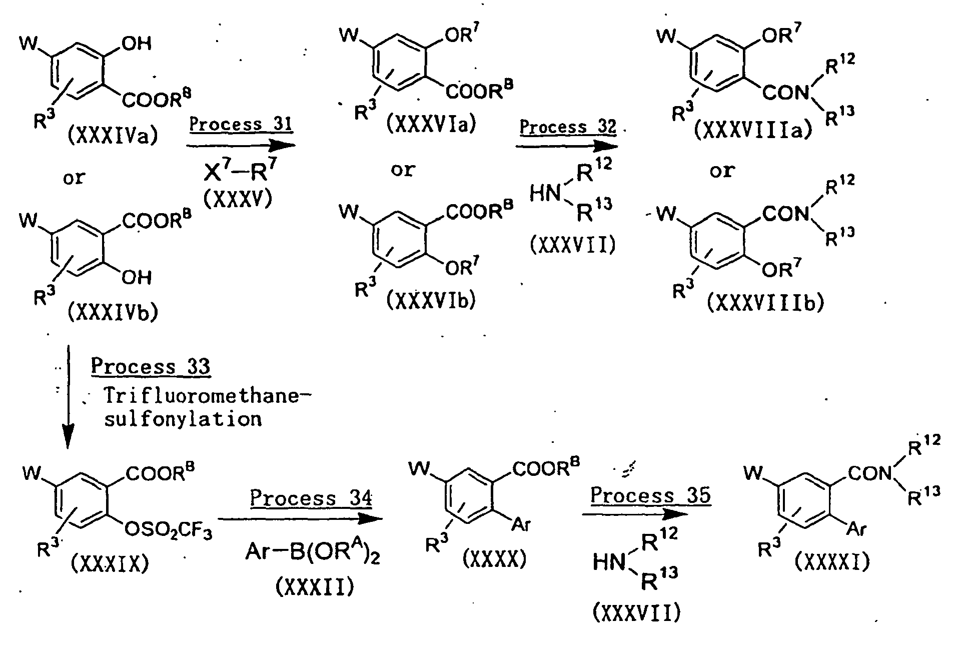

In the formula, R represents a C₁₋₆ alkyl group; X⁷ represents a halogen atom; and Ar, R^{A}, R³, R⁷, R¹², R¹³ and W have the same meaning as defined above.

### Process 31

A compound represented by the above general formula (XXXVIa) or (XXXVIb) can be prepared by subjecting a compound represented by the above general formula (XXXIVa) or (XXXIVb) to O-alkylation using an alkylating agent represented by the above general formula (XXXV) in the presence of a base such as, for example, sodium hydroxide, potassium carbonate, triethylamine or diisopropylethylamine, optionally in the presence of a catalytic amount of sodium iodide in an inert solvent. As an inert solvent used in the *O*-alkylation reaction, for example, methanol, ethanol, tetrahydrofuran, *N,N*-dimethylformamide, water, or a mixed solvent thereof can be illustrated. The reaction temperature is usually from room temperature to reflux temperature, and the reaction time is usually from 10 minutes to 1 day, varying based on a used starting material, solvent and reaction temperature.

### Process 32

A compound represented by the above general formula (XXXVIIIa) or (XXXVIIIb) can be prepared: method 1) by deriving a compound represented by the above general formula (XXXVIa) or (XXXVIb) into a corresponding carbonic acid by a general alkaline hydrogenation, allowing to react with an activated esterifing reagent such as, for example, diphenylphosphoryladize in the presence of a base such as, for example, triethylamine, and then condensing with a corresponding amine represented by the above general formula (XXXVII) in an inert solvent, or method 2) by allowing a corresponding amine represented by the above general formula (XXXVII) to react with an activating agent such as, for example, trimethylalminum and then to react with a compound represented by the above general formula (XXXVIa) or (XXXVIb) in an inert solvent. As an inert solvent used in the condensation reaction in method 1), for example, tetrahydrofuran, *N,N*-dimethylformamide, or a mixed solvent thereof can be illustrated. As an inert solvent used in the condensation reaction in method 2), for example, toluene or tetrahydrofuran can be illustrated. The reaction temperature for both reactions is usually from room temperature to reflux temperature, and the reaction time is usually from 10 minutes to 1 day, varying based on a used starting material, solvent and reaction temperature.

### Process 33

A compound represented by the above general formula (XXXIX) can be prepared by allowing a compound represented by the above general formula (XXXIVb) to react with a reagent to drive into a trifluoromethanesulfonyl compound such as for example, trifluoromethanesulfonic anhydride in the presence of a base such as, for example, pyridine, triethylamine or diisopropylethylamine in an inert solvent. As an inert solvent used in the reaction, for example, toluene, tetrahydrofuran, *N,N*-dimethylformamide, dichloromethane, or a mixed solvent thereof can be illustrated. The reaction temperature is usually from 0°C to reflux temperature, and the reaction time is usually from 10 minutes to 1 day, varying based on a used starting material, solvent and reaction temperature.

### Process 34

A compound represented by the above general formula (XXXX) can be prepared by subjecting a compound represented by the above general formula (XXXIX) to condensation with a boric acid compound represented by the above general formula (XXXII) using a catalyst such as, for example, tris(dibenzylidenacetone) dipalladium and a ligand such as, for example, 2,2'-bis(diphenylphosphino)-1,1'-binaphthyl in the presence of a base such as, for example, cesium carbonate or sodium *tert*-butoxide in an inert solvent. As an inert solvent used in the condensation reaction, for example, *N,N*-dimethylacetamide, toluene, tetrahydrofuran, or a mixed solvent thereof can be illustrated. The reaction temperature is usually from room temperature to reflux temperature, and the reaction time is usually from 1 hour to 1 day, varying based on a used starting material, solvent and reaction temperature.

### Process 35

A compound represented by the above general formula (XXXXI) can be prepared: method 1) by deriving a compound represented by the above general formula (XXXX) into a corresponding carbonic acid by a general alkaline hydrogenation, allowing to react with an activated esterifing reagent such as, for example, diphenylphosphoryladize in the presence of a base such as, for example, triethylamine, and then condensing with a corresponding amine represented by the above general formula (XXXVII) in an inert solvent, or method 2) by allowing a corresponding amine represented by the above general formula (XXXVII) to react with an activating agent such as, for example, trimethylalminum and then to react with a compound represented by the above general formula (XXXX) in an inert solvent. As an inert solvent used in the condensation reaction in method 1), for example, tetrahydrofuran, *N,N*-dimethylformamide, or a mixed solvent thereof can be illustrated. As an inert solvent used in the condensation reaction in method 2), for example, toluene or tetrahydrofuran can be illustrated. The reaction temperature for both reactions is usually from room temperature to reflux temperature, and the reaction time is usually from 10 minutes to 1 day, varying based on a used starting material, solvent and reaction temperature.

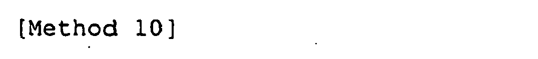

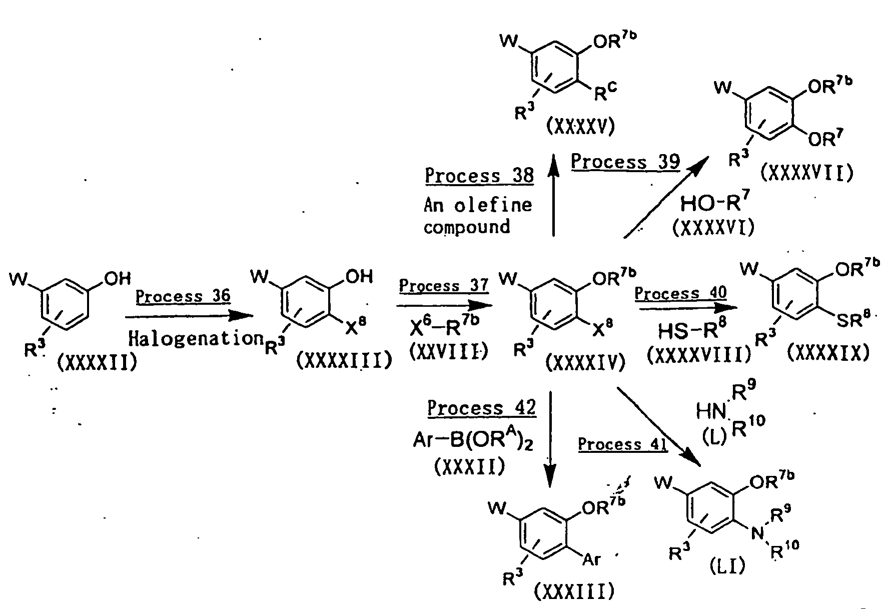

In the formula, R^{c} represents a 1-alkenyyl group; X⁸ represents a halogen atom; and Ar, R^{A}, R³, R⁷, R^{7b}, R⁸, R⁹, R¹⁰ and W have the same meaning as defined above.

### Process 36

A compound represented by the above general formula (XXXXIII) can be prepared by allowing a compound represented by the above general formula (XXXXII) to react with a halogenating reagent such as, for example, iodine monochloride in the presence or absence of an acid such as, for example, acetic acid, in an inert solvent. As an inert solvent used in the halogenation reaction, for example, toluene, acetic acid, N,N-dimethylformamide, dichloromethane, or a mixed solvent thereof can be used. The reaction temperature is usually from room temperature to reflux temperature, and the reaction time is usually from 10 minutes to 1 day, varying based on a used starting material, solvent and reaction temperature. Process 37

A compound represented by the above general formula (XXXXIV) can be prepared by subjecting a compound represented by the above general formula (XXXXIII) to *O*-alkylation using an alkylating agent represented by the above general formula (XXVIII) in the presence of a base such as, for example, sodium hydroxide, potassium carbonate, triethylamine or diisopropylethylamine, optionally in the presence of a catalytic amount of sodium iodide in an inert solvent. As an inert solvent used in the *O*-alkylation reaction, for example, methanol, ethanol, tetrahydrofuran, *N,N*-dimethylformamide, water, or a mixed solvent thereof can be illustrated. The reaction temperature is usually from room temperature to reflux temperature, and the reaction time is usually from 10 minutes to 1 day, varying based on a used starting material, solvent and reaction temperature.

### Process 38

A compound represented by the above general formula (XXXXV) can be prepared by allowing a compound represented by the above general formula (XXXXIV) to react with an olefine compound using a catalyst such as, for example, tris(dibenzylidenacetone) dipalladium and a ligand such as, for example, 2,2'-bis(diphenylphosphino)-1,1'-binaphthyl in the presence of a base such as , for example, cesium carbonate or sodium *tert*-butoxide in an inert solvent. As an inert solvent used in the alkenylating reaction, for example, *N,N*-dimethylacetamide, toluene, tetrahydrofuran, or a mixed solvent thereof can be illustrated. The reaction temperature is usually from room temperature to reflux temperature, and the reaction time is usually from 1 hour to 1 day, varying based on a used starting material, solvent and reaction temperature.

### Process 39

A compound represented by the above general formula (XXXXVII) can be prepared by subjecting a compound represented by the above general formula (XXXXIV) to condensation with an alcohol compound represented by the above general formula (XXXXVI) in the presence or absence of a base such as, for example, sodium hydride or potassium carbonate in an inert solvent. As an inert' solvent used in the condensation reaction, for example, *N,N*-dimethylacetamide, toluene, tetrahydrofuran, or a mixed solvent thereof can be illustrated. The reaction temperature is usually from room temperature to reflux temperature, and the reaction time is usually from 1 hour to 1 day, varying based on a used starting material, solvent and reaction temperature.

### Process 40

A compound represented by the above general formula (XXXXIX) can be prepared by subjecting a compound represented by the above general formula (XXXXIV) to condensation with a thiol compound represented by the above general formula (XXXXVIII) in the presence or absence of a base such as, for example, sodium hydride or potassium carbonate in an inert solvent. As an inert solvent used in the condensation reaction, for example, *N,N*-dimethylacetamide, toluene, tetrahydrofuran, or a mixed solvent thereof can be illustrated. The reaction temperature is usually from room temperature to reflux temperature, and the reaction time is usually from 1 hour to 1 day, varying based on a used starting material, solvent and reaction temperature.

### Process 41

A compound represented by the above general formula (LI) can be prepared by subjecting a compound represented by the above general formula (XXXXIV) : method 1) to condensation with an amine compound represented by the above general formula (L) in the presence or absence of a base such as, for example, sodium hydride or potassium carbonate in an inert solvent, or method 2) to condensation with an amine compound represented by the above general formula (L) using a catalyst such as, for example, tris(dibenzylidenacetone) dipalladium and a ligand such as, for example, 2,2'-bis(diphenylphosphinp)-1,1'-binaphthyl in the presence of a base such as, for example, cesium carbonate or sodium *tert*-butoxide in an inert solvent. As an inert solvent used in the Method 1), for example, *N,N*-dimethylacetamide, toluene, tetrahydrofuran, or a mixed solvent thereof can be illustrated. The reaction temperature is usually from room temperature to reflux temperature, and the reaction time is usually from 1 hour to 1 day, varying based on a used starting material, solvent and reaction temperature. In addition, as an inert solvent used in the Method 2), for example, *N,N*-dimethylacetamide, toluene, tetrahydrofuran, or a mixed solvent thereof can be illustrated. The reaction temperature is usually from room temperature to reflux temperature, and the reaction time is usually from 1 hour to 1 day, varying based on a used starting material, solvent and reaction temperature.

### Process 42

A compound represented by the above general formula (XXXIII) can be prepared by subjecting a compound represented by the above general formula (XXXXIV) to condensation with a compound represented by the above general formula (XXXII) using a catalyst such as, for example, tris(dibenzylidenacetone) dipalladium and a ligand such as, for example, 2,2'-bis(diphenylphosphino)-1,1'-binaphthyl in the presence of a base such as , for example, cesium carbonate or sodium *tert*-butoxide in an inert solvent. As an inert solvent used in the condensation reaction, for example, *N,N*-dimethylacetamide, toluene, tetrahydrofuran, or a mixed solvent thereof can be illustrated. The reaction temperature is usually from room temperature to reflux temperature, and the reaction time is usually from 1 hour to 1 day, varying based on a used starting material, solvent and reaction temperature.

### [Method 11]

Among the compounds represented by the above general formula (VII) used as starting materials in the above-mentioned Methods 2 and 3, a compound (VIIb) wherein n is 2 is commercially available or can be prepared in a known or similar method or the like, and the following method, for example, can be illustrated.

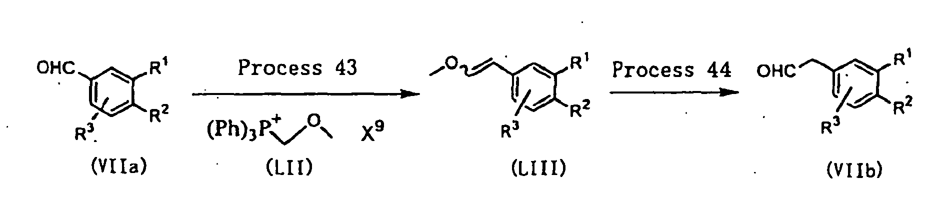

In the formula, Ph represents a phenyl group; X⁹ represents a halogen ion; and R¹, R², R³ have the same meanings as defined above.

### Process 43

A compound represented by the above general formula (LI II) can be prepared by subjecting a compound represented by the above general formula (VIIa) to condensation with a compound represented by the above general formula (LII) in the presence of a base such as, for example, sodium hydride, potassium *tert*-butoxide, *n*-butyllithium, lithium bis(trimethylsilyl)amide, sodium bis(trimethylsilyl)amide or sodium hydroxide in an inert solvent. As an inert solvent used in the reaction, for example, tetrahydrofuran, dimethylsulfoxide, *N,N*-dimethylformamide, *N,N*-dimethylacetamide, *N*-methylpyrrolidinone, 1,3-dimethyl-2-imidazolidinone, acetonitrile, or a mixed solvent thereof can be illustrated. The reaction temperature is usually from -78°C to reflux temperature, and the reaction time is usually from 30 minutes to 1 day, varying based on a used starting material, solvent and reaction temperature.

### Process 44

A compound represented by the above general formula (VIIb) can be prepared by subjecting a compound represented by the above general formula (LIII) to hydrogenation in the presence of an acid such as, for example, hydrochloric acid, sulfuric acid, methanesulfonic acid, p-toluenesulfonic acid or pyridinium p-toruenesulfonate. As a solvent used in the reaction, for example, tetrahydrofuran, acetone, acetonitrile, water, or a mixed solvent thereof can be illustrated. The reaction temperature is usually from 0°C to reflux temperature, and the reaction time is usually from 30 minutes to 1 day, varying based on a used starting material, solvent and reaction temperature.

As protective groups used in the above-mentioned production methods, various protective groups generally used in organic syntheses can be used. For example, as a hydroxy-protective group, a p-methoxybenzyl group, a benzyl group, a methoxymethyl group, an acetyl group, a pivaroyl group, a benzoyl group, a *tert*-butyldimethylsilyl group, a *tert*-butyldiphenylsilyl group and an aryl group, in addition, in a case that there are two neighboring hydroxy groups, an isopropylidene group, a cyclopentylidene group and a cyclohexylidene group can be illustrated. As a thiol-protective group, for example, a p-methoxybenzyl group, a benzyl group, an acetyl group, a pivaroyl group, a benzoyl group and a benzyloxycarbonyl group can be illustrated. As an amino-protective group, for example, a benzyloxycarbonyl group, a *tert*-butoxycarbonyl group, a benzyl group, a p-methoxybenzyl group,a trifluoroacetyl group, an acetyl group and a phthaloyl group can be illustrated. As a carboxyl-protective group, for example a benzyl group, a *tert*-butyldimethylsilyl group and an aryl group can be illustrated.

The compounds represented by the above general formula (I)of the present invention obtained by the above production methods can be isolated and purified by conventional separation means such as, for example, fractional recrystallization, purification using chromatography, solvent extraction and solid phase extraction.

The benzimidazole derivatives represented by the above general formula (I) of the present invention can be converted into their pharmaceutically acceptable salts in the usual way. Examples of such salts include acid addition salts with mineral acids such as, for example, hydrochloric acid, hydrobromic acid, hydroiodic acid, sulfuric acid, nitric acid and phosphoric acid, acid addition salts with organic acids such as, for example, formic acid, acetic acid, methanesulfonic acid, benzenesulfonic acid, p-toluenesulfonic acid, propionic acid, citric acid, succininc acid, tartaric acid, fumaric acid, butyric acid, oxalic acid, malonic acid, maleic acid, lactic acid, malic acid, carbonic acid, benzoic acid, glutamic acid and aspartic acid, salts with inorganic bases such as, for example, sodium salt and potassium salt, addition salts with organic bases such as, for example, *N*-methyl-D-glucamin, *N,N'*-dibenzylethylenediamine, 2-aminoethanol, tris(hydroxymethyl)aminomethane, arginine and lysine.

The benzimidazole derivatives represented by the above general formula (I) of the present invention or pharmaceutically acceptable salts thereof include their solvates with pharmaceutically acceptable solvents such as, for example, ethanol and water.

Among the benzimidazole derivatives represented by the above general formula (I) of the present invention, there can be two geometric isomers, cis(*Z*)-isomer and trans(*E*)-isomer, in each compound having an unsaturated bond. In the present invention, either of cis(*Z*)-isomer and trans(*E*)-isomer can be employed.

Among the benzimidazole derivatives represented by the above general formula (I) of the present invention, there can be two optical isomers, *R*-isomer and *S*-isomer, in each compound having an asymmetric carbon atom excluding the sugar-residue moiety. In the present invention, either of R-isomer and S-isomer can be employed, and a mixture of both isomers can be also employed.

Among the benzimidazole derivatives represented by the above general formula (I) of the present invention, there can be some tautomers. The compounds of the present invention include their tautomers.

In addition, in the present invention, various prodrugs of the compounds represented by the above general formula (I) can be also used. The term "prodrug" means a compound obtained by modifying a parent compound with a pharmaceutically acceptable group generally used in a prodrug, and such compound can be expected, for example, to have additional characteristics such as, for example, improved stability or long action and, for example, exert an efficacy after being converted into the parent compound in the intestine tract. The prodrugs of the compound represented by the above general formula (I) of the present invention are prepared by suitably introducing a group forming a prodrug into one or more group selected from a hydroxy group and an amino group using an agent to form a prodrug such as, for example, a corresponding halide in the usual way and then optionally isolating and purifying in the usual way as an occasion demand (see "Gekkan-yakuji The clinical pharmacokinetics for proper uses of pharmaceutical drugs", Extra edition, March 2000, Vol.42, No.4, pp.669-707; "New drug delivery system", issued by CMC Co. Ltd., January 31, 2000, pp. 67-173). The group forming a prodrug used in a hydroxy group or an amino group is selected from the group consisting of C₁₋₆ alkyl-CO-, C₁₋₆ alkyl-O-C₁₋₆ alkyl-CO-, C₁₋₆ alkyl-OCO-C₁₋₆ alkyl-CO-, C₁₋₆ alkyl-OCO- and C₁₋₆ alkyl-O-C₁₋₆ alkyl-OCO-.

In the present invention, the diseases associated with an abnormality of plasma uric acid level include, for example, gout, hyperuricemia, urinary lithiasis, hyperuricemic nephropathy, and acute uric acid nephropathy, especially gout and hyperuricemia.

When the pharmaceutical compositions of the present invention are employed in the practical prevention or treatment, the dosage of a compound represented by the above general formula (I) or a pharmaceutically acceptable salt thereof, or a prodrug thereof as the active ingredient is appropriately decided depending on the age, sex, body weight and degree of symptoms and treatment of each patient, for example, which is approximately within the range of 1 to 2,000 mg per day per adult human in the case of oral administration, and the daily dose can be divided into one or several doses and administered suitably.

When the pharmaceutical compositions of the present invention are employed in the practical prevention or treatment, various dosage forms are used depending on their usages for oral or parenteral administration. As examples of the dosage forms, orally administration forms such as, for example, powders, fine granules, granules, tablets, capsules or dry syrups are preferable.

These pharmaceutical compositions can be prepared by admixing with an appropriate pharmaceutical additive such as, for example, excipients, disintegrators, binders or lubricants in accordance with pharmaceutically conventional methods and formulating the mixture depending on their dosage forms in the usual way.

For example, powders can be formulated by, if desired, admixing well an active ingredient with, for example, appropriate excipients and lubricants. Tablets can be formulated by, if desired, admixing an active ingredient with, for example, appropriate excipients, disintegrators, binders and lubricants, and compressing the mixture in accordance with conventional methods. The tablets, further if desired, can be suitably coated to provide, for example, film-coated tablets, sugar-coated tablets, and enteric-coated tablets. Capsules can be formulated by, if desired, admixing well an active ingredient with, for example, appropriate excipients and lubricants, or formulating granules or fine-powders in accordance with conventional methods, and then filling the compositions in appropriate capsules. Such orally administration forms can be formulated as immediate release or sustained release preparations depending on the prevention or treatment methods.

The active ingredient of the present invention can be used in combination with a drug for the treatment of hyperuricemia or gout which does not substantially inhibit the absorption of nucleosides. As drugs usable for the treatment of hyperuricemia in the present invention, for example, a uricosuric drug such as, for example, probenecid, bucolome or benzbromarone; a uric acid synthesis inhibitor such as, for example, allopurinol, oxypurinol, febuxostat or Y-700; a urinary alkalinizer such as, for example, sodium hydrogen carbonate, potassium citrate or sodium citrate; and a uric acid oxidase such as, for example, rasburicase, uricase PEG-20 or a recombinant uric acid oxidase (uricase) can be illustrated. In addition, as drugs for the treatment of gout, for example, colchicines; a nonsteroidal anti-inflammatory agent such as, for example, indometacin, naproxen, fenbufen, pranoprofen, oxaprozin, ketoprofen, etoricoxib or tenoxicam; an adrenocortical steroid such as, for example, prednisolone; can be illustrated. In the present invention, the active ingredient of the present invention can be used in combination with at least one of these drugs, and the pharmaceutical composition comprising in combination at least one of these drugs is not limited to a single preparation simultaneously formulated with the active ingredient of the present invention, and includes administration modes such as a combination of a separated preparation formulated separately from a pharmaceutical composition containing the active ingredient of the present invention to be administered at the same or different dosage intervals. In addition, in a case of use in combination with a drug other than the active ingredient of the present invention, the dose of the compound of the present invention can be decreased according to the dosage of the other drug used in combination with, occasionally, beneficial effect more than additive effect in the prevention or treatment of the above diseases can be obtained, and adverse effects of coadministrated drugs can be avoided or declined.

### Brief Description of Drawings

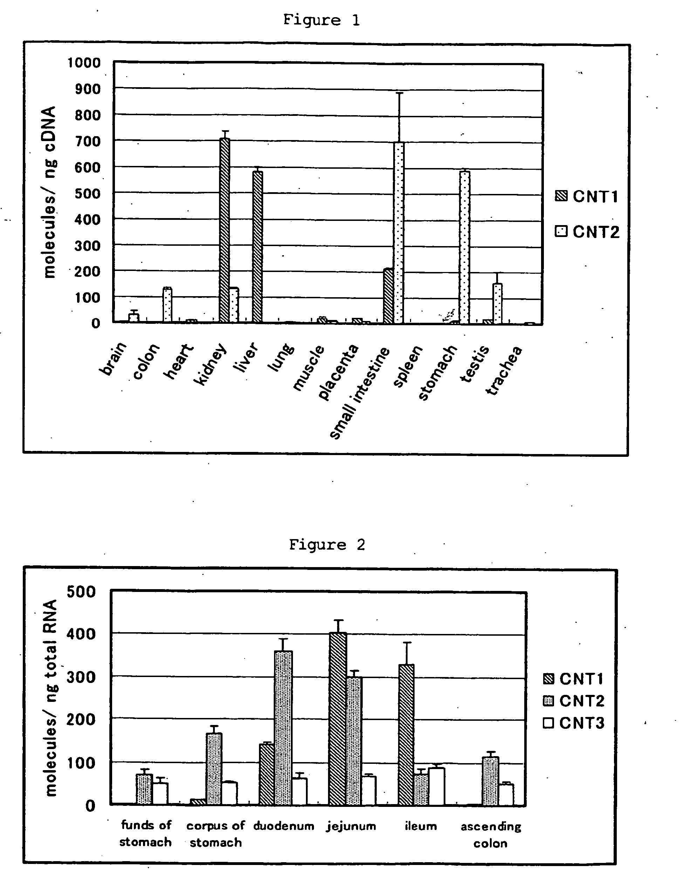

The Figure1 is a graph showing the pattern of CNT1 and CNT2 distribution in human tissues. The vertical axis is the number of molecular per 1 ng cDNA (molecular number/ng cDNA). The horizontal axis is the name of tissues. The left bar graph shows CNT1 and the right bar graph shows CNT2.

The Figure 2 is a graph showing the pattern of CNT1 to CNT3 distribution in human stomach and intestines. The vertical axis is the number of molecular per 1 ng total RNA (molecular number/ng total RNA). The horizontal axis is the name of part. The left bar graph shows CNT1, the central bar graph shows CNT2 and the right bar graph shows CNT3.

### Best Mode for Carrying Out the Invention

The present invention is further illustrated in more detail by way of the following Reference Examples, Examples and Test Examples. However, the present invention is not limited thereto.

### Reference Example 1

### 5,6-Dichloro-1,3-dihydro-2H-benzimidazol-2-one

4,5-Dichloro-1,2-phenylenediamine (10g) was dissolved in tetrahydrofuran (35mL), and to the mixture was added a suspension of carbonyldiimidazole (9.6g) in tetrahydrofuran (15mL) at room temperature. The reaction mixture was stirred at room temperature for 1 hour, and to the reaction mixture was added water under ice-cooling. The insoluble material was collected by filtration and dried to give.the title compound (11.6g).
¹H-NMR (DMSO-d₆) δ ppm:
7.11 (2H, s), 10.93 (2H, s)

### Reference Example 2

### 2,5,6-Trichloro-1H-benzimidazole

5,6-Dichloro-1,3-dihydro-2H-benzimidazol-2-one (11.5g) was suspended in phosphorus oxychloride (40mL), and the mixture was stirred at 120°C for 24 hours. After cooling the reaction mixture, water was added to the reaction mixture, and to the mixture was added 28% aqueous ammonia solution to alkalize. The solid was collected by filtration and dried to give the title compound (5.0g).
¹H-NMR (DMSO-d₆) δ ppm:
7.83 (2H, brs), 13.30-14.00 (1H, br)

### Reference Example 3

### 2-Chloro-1-(2,3,5-tri-O-acetyl-β-D-ribofuranosyl)-1H-benzimidazole

2-Chlorobenzimidazole (7.5g) and *N,O*-bis(trimethylsilyl)acetoamide (18.3mL) were suspended in acetonitrile (150mL), and the mixture was stirred at 80°C for 1 hour. The reaction mixture was allowed to cool to room temperature. To the mixture was added trifluoromethanesulfonic acid trimethylsilyl ester (17.9mL), and the mixture was stirred for 15 minutes. To the reaction mixture was added 1,2,3,5-tetra-*O*-acetyl-D-ribofuranose (17.3g), and the mixture was stirred at room temperature for 6 hour. To the reaction mixture was added a saturated aqueous sodium hydrogen carbonate solution, and the resulting mixture was extracted with ethyl acetate. The organic layer was washed with brine and dried over anhydrous magnesium sulfate, and the solvent, was removed under reduced pressure. The obtained residue was purified by column chromatography on silica gel (eluent:ethyl acetate/hexane= 1/ 10) to give the title compound (12.4g).
¹H-NMR (CDCl₃) δ ppm:
2.05 (3H, s), 2.17 (3H, s), 2.22 (3H, s), 4.30-4.60 (3H, m), 5.51 (1H, dd, J=4.0Hz, 6.6Hz), 5.65 (1H, dd, J=6.6Hz, 7.3Hz), 6.24, (1H, J=7.3Hz), 7.20-7.40 (2H, m), 7.61 (1H, d, J=7.4Hz), 7.71 (1H, d, J=7.4Hz)

### Reference Example 4

The following compound was prepared in a similar manner to that described in Reference Example 3 using the corresponding materials.
1-(2,3,5-Tri-*O*-acetyl-β-D-ribofuranosyl)-2,5,6-trichloro-1H-benzimidazole
¹H-NMR (CDCl₃) δ ppm:
2.05 (3H, s), 2.19 (3H, s), 2.32 (3H, s), 4.30-4.45 (2H, m), 4.55-4.65 (1H, m), 5.46 (1H, dd, J=2.7Hz, 6.8Hz), 5.50 (1H, dd, J=6.8Hz, 7.5Hz), 6.18, (1H, J=7.5Hz), 7.80 (1H, s), 7.81 (1H, s)

### Reference Example 5

### 2-Chloro-1-(β-D-ribofuranosyl)-1H-benzimidazole

2-Chloro-1-(2,3,5-tri-*O*-acetyl-β-D-ribofuranosyl)-1H-benzimidazole (12.3g) was dissolved in methanol (150mL). To the mixture was added 28% sodiummethoxide-methanol solution (1mL), and the mixture was stirred for 1 hour. The reaction mixture was concentrated under reduced pressure to give the title compound (8.5g).
¹H-NMR (DMSO-d₆) δ ppm:
3.60-3.78 (2H, m), 3.90-4.03 (1H, m), 4.13 (1H, dd, J=2.2Hz, 5.8Hz), 4.50 (1H, dd, J=5.8Hz, 7.5Hz), 5.89 (1H, d, J=7.5Hz), 7.15-7.35 (2H, m), 7.62 (1H, d, J=7.5Hz), 7.99 (1H, d, 7.5Hz)

### Reference Example 6

The following compound was prepared in a similar manner to that described in Reference Example 5 using the corresponding materials.
1-(β-D-Ribofuranosyl)-2,5,6-trichloro-*1H*-benzimidazole ¹H-NMR (DMSO-d₆) δ ppm:
3.60-3.80 (2H, m), 3.95-4.25 (2H, m), 4.35-4.50 (1H, m), 5.20-5.60 (3H, m), 5.89 (1H, d, J=7.9Hz), 7.97 (1H, s), 8.56 (1H, s)

### Reference Example 7

### 2-Azido-1-(2,3,5-tri-O-acetyl-β-D-ribofuranosyl-1H-benzimidazole

2-Chloro-1-(2,3,5-tri-*O*-acetyl-β-D-ribofuranosyl)-1H-benzimidazole (1.0g) was dissolved in *N,N*-dimethylformamide (10mL). To the mixture was added sodium azide (1.1g), and the mixture was stirred at 100°C for 24 hours. To the reaction mixture was added water, and the resulting mixture was extracted with ethyl acetate. The organic layer was washed with brine and dried over anhydrous magnesium sulfate, and the solvent was removed under reduced pressure. The obtained residue was purified by column chromatography on silica gel (eluent:ethyl acetate/hexane=1/2) to give the title compound (0.45g).
¹H-NMR (CDCl₃) δ ppm:
2.06 (3H, s), 2.16 (3H, s), 2.19 (3H, s), 4.30-4.50 (3H, m), 5.51 (1H, dd, J=4.1Hz, 6.5Hz), 5.70 (1H, dd, J=6.5Hz, 6.7Hz), 5.59 (1H, d, J=6.7Hz), 7.15-7.35 (2H, m), 7.47 (1H, d, J=8.0Hz), 7.63 (1H, d, J=8.0Hz)

### Reference Example 8

### 2-Amino-1-(2,3,5-tri-O-acetyl-β-D-ribofuranosyl)-1H-benzimidazole

2-Azido-1-(2,3,5-tri-*O*-acetyl-β-D-ribofuranosyl)-*1H-*benzimidazole (100mg) was dissolved in methanol (2mL). To the solution was added a catalytic amount of 10% palladium-carbon powder, and the mixture was stirred at room temperature under a hydrogen atmosphere for 1 hour. The insoluble material was removed by filtration, and the solvent of filtrate was removed under reduced pressure to give the title compound (97mg).
¹H-NMR (CDCl₃) δ ppm:
1.99 (3H, s), 2.16 (3H, s), 2.17 (3H, s), 4.27-4.43 (2H, m), 4.55-4.68 (1H, m), 5.07 (2H, brs), 5.43 (1H, dd, J=3.7Hz, 6.6Hz), 5.57 (1H, dd, J=6.6Hz, 7.5Hz), 6.04 (1H, d, J=7.5Hz), 7.08 (1H, t, J=7.8Hz), 7.15 (1H, t, J=7.8Hz), 7.21 (1H, d, J=7.8Hz), 7.42 (1H, d, J=7.8Hz)

### Reference Example 9

### 4-Benzyloxy-3-hydroxybenzaldehyde

3,4-Dihydroxybenzaldehyde (21.6g) and potassium carbonate (21.56g) were suspended in *N,N*-dimethylformamide (200mL). To the mixture was added dropwise benzylbromide (18.5mL) under ice-cooling, and the mixture was stirred at room temperature for 16 hours. To the reaction mixture was added dropwise 2mol/L hydrochloric acid (400mL), and the resulting mixture was extracted with ethyl acetate. The organic layer was washed with brine and dried over anhydrous magnesium sulfate. The solvent was removed under reduced pressure, and the obtained residue was purified by column chromatography on silica gel (eluent:ethyl acetate/hexane=1/5) to give the title compound (19.0g).
¹H-NMR (CDCl₃) δ ppm:
5.20 (2H, s), 7.04 (1H, d, J=8.2Hz), 7.20-7.60 (7H, m), 9.84 (1H, s)

### Reference Example 10

The following compound was prepared in a similar manner to that described in Reference Example 9 using the corresponding materials.
3-Benzyloxybenzonitrile
¹H-NMR (CDCl₃) δ ppm:
5.08 (2H, s), 7.13-7.50 (9H, m)

### Reference Example 11

### 3-Methoxy-4-phenylbenzonitrile

4-Hydroxy-3-methoxybenzonitrile (14.9g) and pyridine (24mL) were dissolved in dichloromethane (150mL). To the stirred mixture was added dropwise trifluoromethanesulfonic anhydride (19. 4mL) under ice-cooling. The mixture was stirred at room temperature for 30 minutes. To the reaction mixture was added dilute hydrochloric acid, and the resulting mixture was extracted with ethyl acetate. The organic layer was washed with brine and dried over anhydrous magnesium sulfate. The solvent was removed under reduced pressure to give the trifluoromethanesulfonic acid ester. The obtained trifluoromethanesulfonic acid ester, phenylboronic acid (14.7g), tetrabutylammonium bromide (1. 6g), sodium carbonate (21.2g), tetrakis(triphenylphosphine)palladium (5.7g) and water (24mL) were suspended in toluene (150mL), and the mixture was stirred at 80°C for 12 hours. The insoluble material was removed by filtration through celite, and the solvent of filtrate was removed under reduced pressure. To the residue was added dropwise water, and then added brine, and the resulting mixture was extracted with ethyl acetate. The organic layer was washed with brine and dried over anhydrous magnesium sulfate. The solvent was removed under reduced pressure, and the obtained residue was purified by column chromatography on silica gel (eluent:ethyl acetate/hexane=1/5) to give the title compound (18.0g).
¹H-NMR (CDCl₃) δ ppm:
3.84 (3H, s), 7.15-7.60 (8H, m)

### Reference Example 12

The following compound was prepared in a similar manner to that described in Reference Example 11 using the corresponding materials.
2-Methoxy-4-phenylbenzaldehyde
¹H-NMR (CDCl₃) δ ppm:
4.01 (3H, s), 7.17 (1H, d, J=1.9Hz), 7.20-7.70 (6H, m), 7.90 (1H, d, J=8.3Hz), 10.49 (1H, s)

### Reference Example 13

### 4-Benzyloxy-3-hydroxybenzonitrile

4-Benzyloxy-3-hydroxybenzaldehyde (19.0g), hydroxylamine hydrochloride (8.6g), sodiumacetate (13.7g) and water (30mL) were suspended in ethanol (150mL), and the mixture was stirred at 80°C for 8 hours. To the reaction mixture was added water (100mL), and the resulting mixture was extracted with ethyl acetate. The organic layer was washed with brine and dried over anhydrous magnesium sulfate. The solvent was removed under reduced pressure to give the oxime compound. The obtained oxime compound was dissolved in dichloromethane (100mL), and to the reaction mixture was added pyridine (20 mL). To the stirred mixture was added dropwise trifluoroacetic anhydride (35.3mL) under ice-cooling. The mixture was stirred at room temperature for 6 hours. To the stirred mixture was added 2mol/L hydrochloric acid (100mL), and the resulting mixture was extracted with ethyl acetate. The organic layer was washed with a saturated aqueous sodium hydrogen carbonate solution and brine successively and dried over anhydrous magnesium sulfate. The solvent was removed under reduced pressure, and the obtained residue was purified by column chromatography on silica gel (eluent:ethyl acetate/hexane=1/10 ) to give the title compound (11.0g).
¹H-NMR (CDCl₃) δ ppm:
5.17 (2H, s), 5.82 (1H, s), 6.96 (1H, d, J=8.3Hz), 7.13-7.25 (2H, m), 7.35-7.50 (5H, m)

### Reference Example 14

### 3-Hydroxy-4-phenylbenzonitrile

3-Methoxy-4-phenylbenzonitrile (17.8g) was dissolved in dichloromethane (200mL). To the stirred mixture was added dropwise boron tribromide (14mL) under ice-cooling, and the mixture was stirred at continuous temperature for 6 hours. To the stirred reaction mixture was added dropwise water (200mL) under ice-cooling, and the resulting mixture was extracted with ethyl acetate. The organic layer was washed with brine and dried over anhydrous magnesium sulfate. The solvent was removed under reduced pressure, and the obtained residue was purified by column chromatography on silica gel (eluent:ethyl acetate/hexane=1/5) to give the title compound (11.0g).
¹H-NMR (CDCl₃) δ ppm:
5.55 (1H, brs), 7.20-7.60 (8H, m)

### Reference Example 15

The following compound was prepared in a similar manner to that described in Reference Example 14 using the corresponding materials.
2-Hydroxy-4-phenylbenzaldehyde
¹H-NMR (CDCl₃) δ ppm:
7.15-7.32 (2H, m), 7.35-7.53 (3H, m), 7.57-7.70 (3H, m), 9.93 (1H, s), 11.12 (1H, s)

### Reference Example 16

### 2-Hydroxy-4-phenylbenzaldehyde oxime

2-Hydroxy-4-phenylbenzaldehyde (3.7g), hydroxylamine hydrochloride (1.4g), sodium acetate(3.1g) and water (10mL) were suspended in ethanol (50 mL), and the mixture was stirred at 80°C for 3 hours. To the reaction mixture was added water (50mL), and the resulting mixture was extracted with ethyl acetate. The organic layer was washed with brine and dried over anhydrous magnesium sulfate, and the solvent was removed under reduced pressure to give the title compound (3.0g).
¹H-NMR (CDCL₃) δ ppm:
7.17 (1H, dd, J=2.0Hz, 7.9Hz), 7.20-7.30 (2H, m), 7.34-7.50 (3H, m), 7.55-7.65 (2H, m), 8.26 (1H, s), 9.87 (1H, brs)

### Reference Example 17

The following compound was prepared in a similar manner tothat described in Reference Example 16 using the corresponding materials.
2-Methoxy-4-phenylbenzaldehyde oxime
¹H-NMR (CDCL₃) δ ppm:
3.93 (3H, s), 7.11 (1H, d, J=1.6Hz), 7.20 (1H, dd, J=1.6Hz, 7.9Hz), 7.33-7.50 (3H, m), 7.55-7.65 (2H, m), 7.78 (1H, d, J=7.9Hz), 8.53 (1H, s)

### Reference Example 18

### 4-Benzyloxy-3-(4-benzyloxybutoxy)benzonitrile

4-Benzyloxy-3-hydroxybenzonitrile (3.4g) and potassium carbonate (6.3g) were dissolved in *N,N*-dimethylformamide (20mL). To the mixture was added benzyl 4-bromobutylether (3mL), and the mixture was stirred at 50°C for 16 hours. To the reaction mixture was added dropwise 2mol/L hydrochloric acid (4mL), and the resulting mixture was extracted with ethyl acetate. The organic layer was washed with brine and dried over anhydrous magnesium sulfate. The solvent was removed under reduced pressure, and the obtained residue was purified by column chromatography on silica gel (eluent:ethyl acetate/hexane=1/5) to give the title compound (5.9g).
¹H-NMR (CDCl₃) δ ppm:
1.70-2.10 (4H, m), 3.55 (2H, t, J=6.1Hz), 4.04 (2H, t, J=6.5Hz), 4.49 (2H, s), 5.17 (2H, s), 6.90 (1H, d, J=8,2Hz), 7.08 (1H, d, J=1.9Hz), 7.19 (1H, dd, J=1.9Hz, 8.2Hz), 7.24-7.45 (10H, m)

### Reference Example 19

The following compound was prepared in a similar manner to that described in Reference Example 18 using the corresponding materials.
3-(3-Benzyloxypropoxy)benzonitrile
¹H-NMR (CDCl₃) δ ppm:
1.95-2.25 (2H, m), 3.65 (2H, t, J=6.0Hz), 4,10 (2H, t, J=6.0Hz), 9.52 (2H, s), 7.00-7.50 (9H, m)

### Reference Example 20

### 4-Hydroxy-3-(4-hydroxybutoxy)benzonitrile

4-Benzyloxy-3-(4-benzyloxybutoxy)benzonitrile (4.0g) was dissolved in a mixed solvent of trifluoroacetic acid (9mL), dimethylsulfide (0.5 mL) and water (5mL), and the mixture was stirred at room temperature for 18 hours. The solvent was removed under reduced pressure, and the obtained residue was purified by column chromatography on silica gel (eluent:ethyl acetate/hexane=1/5) to give the title compound (1.0g). ¹H-NMR (CDCl₃) δ ppm:
1.90-2.05 (4H, m), 4.05-4.20 (2H, m), 4.40-4.55 (2H, m), 6.06 (1H, s), 6.98 (1H, d, J=8.3Hz), 7.07 (1H, d, J=1.8Hz), 7.24 (1H, dd, J=1.8Hz, 8.3Hz)

### Reference Example 21

### 4-(3-Benzyloxyphenyl)-3-(4-hydroxybutoxy)benzonitrile

4-Hydroxy-3-(4-hydroxybutoxy)benzonitrile (1.0g) and pyridine (1.9mL) were dissolved in dichloromethane (15mL), and to the mixture was added dropwise trifluoromethanesulfonic anhydride (1.7mL) under ice-cooling. The mixture was stirred at room temperature for 30 minutes, and to the reaction mixture was added 1mol/L hydrochloric acid (50mL). The resulting mixture was extracted with ethyl acetate, and the organic layer was washed with a saturated aqueous sodium hydrogen carbonate solution and brine successively and dried over anhydrous magnesium sulfate. The solvent was removed under reduced pressure to give trifluoromethanesulfonic acid ester. The obtained trifluoromethanesulfonic acid ester, 3-benzyloxyphenylboronicacid (1.3g), sodium carbonate (1.0g), tetrakis(triphenylphosphine)palladium (0.3g) and water (2mL) were suspended in *N,N*-dimethylformamide (15mL), and the mixture was stirred at 80°C for 12 hours. To the reaction mixture was added dropwise 1mol/L hydrochloric acid (30mL), and the resulting mixture was extracted with ethyl acetate. The organic layer was washed with brine and dried over anhydrous magnesium sulfate. The solvent was removed under reduced pressure, and the obtained residue was purified by column chromatography on aminopropylated silica gel (eluent:ethyl acetate/hexane=1/5) to give the title compound (0.8g).
¹H-NMR (CDCl₃) δ ppm:
1.60-1.72 (2H, m), 1.78-1.90 (2H, m), 3.62 (2H, t, J=6.5Hz), 4.02 (2H, t, J=6.2Hz), 5.10 (2H, s), 6.90-7.50 (12H, m)

### Reference Example 22

### 4-Benzyloxy-3-(4-benzyloxybutoxy)benzylamine

Lithium aluminium hydride (2.6g) was suspended in tetrahydrofuran (30mL), and to the mixture was added dropwise a solution of 4-benzyloxy-3-(4-benzyloxybutoxy)benzonitrile (5.9g) in tetrahydrofuran (30mL) under ice-cooling. The mixture was stirred at 60°C for 2 hours. After the mixture was cooled in an ice bath, ethanol and water were successively added dropwise to the reaction mixture. To the reaction mixture was added anhydrous sodium sulfate, and the insoluble material was removed by filtration through celite. The filtrate was concentrated under reduced pressure, and the obtained residue was purified by column chromatography on aminopropylated silica gel (eluent:ethyl acetate/hexane=1/5) to give the title compound (2.5g).
¹H-NMR (CDCl₃) δ ppm:
1.70-2.00 (4H, m), 3.55 (2H, t, J=6.4Hz), 3.78 (2H, s), 4.06 (2H, t, J=6.5Hz), 4.49 (2H, s), 5.10 (2H, s), 6.70-7.00 (3H, m), = 7.20-7.50 (10H, m)

### Reference Example 23

The following compounds were prepared in a similar manner to that described in Reference Example 20 using the corresponding nitrile compound or oxime compound.
3-Methoxy-4-phenylbenzylamine
¹H-NMR (CDCl₃) δ ppm:
3.83 (3H, s), 3.92 (2H, s), 6.80-7.70 (8H, m) 2-Methoxy-4-phenylbenzylamine
¹H-NMR (CDCl₃) δ ppm:
3.87 (2H, s), 3.92 (3H, s), 7.00-7.70 (8H, m)
3-(3-Benzyloxypropoxy)benzylamine
¹H-NMR (CDCl₃) δ ppm:
1.95-2.15 (2H, m), 3.65 (2H, t, J=6.1Hz), 3.83 (2H, s), 4.07 (2H, t, J=6.1Hz), 4.51 (2H, s), 6.70-7.50 (9H, m)
3-Hydroxy-4-phenylbenzylamine
¹H-NMR (DMSO-d₆) δ ppm:
3.67 (2H, s), 6.83 (1H, d, J=7.8Hz), 6.91 (1H, s), 7.16 (1H, d, J=7.8Hz), 7.26 (1H, t, J=7.7Hz), 7.37 (2H, t, J=7.7Hz), 7.52 (2H, d, J=7.7Hz)
2-Hydroxy-4-phenylbenzylamine
¹H-NMR (CDCl₃) δ ppm:
4.17 (2H, s), 6.95-7.70 (8H, m)
3-Benzyloxybenzylamine
¹H-NMR (CDCl₃) δ ppm:
3.85 (2H, s), 5.08 (2H, s), 6.86 (1H, d, J=8.2Hz), 6.91 (1H, d, J=7.7Hz), 6.96 (1H, s), 7.20-7.50 (6H, m)
4-(3-Benzyloxyphenyl)-3-(4-hydroxybutoxy)benzylamine
¹H-NMR (CDCl₃) δ ppm:
1.60-1.72 (2H, m), 1.75-1.90 (2H, m), 3.60 (2H, t, J=6.4Hz), 3.89 (2H, s), 4.02 (2H, t, J=6.2Hz), 5.09 (2H, s), 6.85-7.55 (12H, m)

### Reference Example 24

### 3-(4-Acetoxybutoxy)-5-hydroxybenzaldehyde

3,5-Dihydroxybenzaldehyde (0.96g) and potassium carbonate (1.44g) were suspended in *N,N*-dimethylformamide (5 mL). To the mixture was added 4-bromobutyl acetate (1.49g) under ice-cooling, and the mixture was stirred at room temperature for 16 hours. To the reaction mixture was added 1mol/L hydrochloric acid, and the resulting mixture was extracted with diethyl ether. The organic layer was washed with a saturated aqueous sodium hydrogen carbonate solution and brine successively and dried over anhydrous magnesium sulfate. The filtrate was concentrated under reduced pressure, and the obtained residue was purified by column chromatography on silica gel (ethyl acetate/hexane=1/5) to give the title compound (0.57g).
¹H-NMR (CDCl₃) δ ppm:
1.75-1.95 (4H, m), 2.07 (3H, s), 3.95-4.25 (4H, m), 5.65 (1H, s), 6.67 (1H, s), 6.90-7.05 (2H, m), 9.88 (1H, s)

### Reference Example 25

### 3-(4-Benzyloxybutoxy)-4-hydroxybenzaldehyde

3,4-Dihydroxybenzaldehyde (0,1g) andsodiumhydride (60%, 0.064g) were suspended in *N,N*-dimethylformamide (2 mL), and to the reaction mixture was added benzyl 4-bromobutylether (0.185g) under ice-cooling. The mixture was stirred at room temperature for 17 hours. To the reaction mixture was added water, and the resulting mixture was extracted with ethyl acetate . The organic layer was washed with brine and dried over anhydrous magnesium sulfate. The filtrate was concentrated under reduced pressure, and the obtained residue was purified by column chromatography on silicagel (ethylacetate/hexane=1/3) to give the title compound (0.1g).
¹H-NMR (CDCl₃) δ ppm:
1.70-1.85 (2H, m), 1.90-2.05 (2H, m), 3.56 (2H, t, J=6.1Hz), 4.15 (2H, t, J=6.3Hz), 4.53 (2H, s), 6.42 (1H, s), 7.03 (1H, d, J=8.3Hz), 7.20-7.50 (7H, m), 9.81 (1H, s)

### Reference Example 26

### 3-(4-Acetoxybutoxy)-5-phenylbenzaldehyde

3-(4-Acetoxybutoxy)-5-hydroxybenzaldehyde (0.35g) and pyridine (0.50mL) were dissolved in dichloromethane (5mL), and to the mixture was added dropwise trifluoromethanesulfonic anhydride (0.27mL) under ice-cooling. The mixture was stirred at room temperature for 30 minutes. To the reaction mixture was added 1mol/L hydrochloric acid, and the resulting mixture was extracted with ethyl acetate. The organic layer was washed with brine and dried over anhydrous magnesium sulfate, and the solvent was removed under reduced pressure to give the trifluoromethanesulfonic acid ester. The obtained trifluoromethanesulfonic acid ester, phenylboronic acid (0.20g), potassium carbonate (0.29g), z, tetrakis(triphenylphosphine)palladium (0.08g) and water (1mL) were suspended in *N,N*-dimethylformamide (5mL), and the mixture was stirred at 80°C for 12 hours. The insoluble material was removed by filtration through celite, and the filtrate was concentrated under reduced pressure. To the residue was added dropwise water, and then added brine, and the resulting mixture was extracted with ethyl acetate. The organic layer was washed with brine and dried over anhydrous magnesium sulfate. The filtrate was concentrated under reduced pressure, and the obtained residue was purified by column chromatography on silica gel (eluent:ethyl acetate/hexane=1/5) to give the title compound (0.22g).
¹H-NMR (CDCl₃) δ ppm:
1.80-2.00 (4H, m), 2.06 (3H, s), 4.05-4.25 (4H, m), 7.30-7.52 (5H, m), 7.56-7.75 (3H, m), 10.04 (1H, s)

### Reference Example 27

The following compound was prepared in a similar manner to that described in Reference Example 26 using the corresponding materials.
3-(4-Benzyloxybutoxy)-4-(3-methoxycarbonylphenyl)-benzaldehyde

¹H-NMR (CDCl₃) δ ppm:
1.60-1.95 (4H, m), 3.47 (2H, t, J=6.2Hz), 3.91 (3H, s), 4.09 (2H, t, J=6.2Hz), 4.45 (2H, s), 6.95-7.70 (9H, m), 7.76 (1H, d, J=7.6Hz), 8.04 (1H, d, J=7.6Hz), 8.25 (1H, s), 10.00 (1H, s)
3-Methoxy-4-phenylbenzaldehyde
¹H-NMR (CDCl₃) δ ppm:
3.89 (3H, s), 7.30-7.65 (8H, m), 10.01 (1H, s)
3-(4-Benzyloxybutoxy)-4-(3-methanesulfonylphenyl)benzaldehyde
¹H-NMR (CDCl₃) δ ppm:
1.60-1.95 (4H, m), 3.04 (3H, s), 3.49 (2H, t, J=6.1Hz), 4.10 (2H, t, J=6.3Hz), 4.46 (2H, s), 7.20-7.70 (9H, m), 7.83 (1H, d, J=8.0Hz), 7.93 (1H, d, J=8.0Hz), 8.19 (1H, s), 10.01 (1H, s)
4-(3-Hydroxyphenyl)benzaldehyde
¹H-NMR (CDCl₃) δ ppm:
6.60-8.00 (8H, m), 10.06 (1H, s)

### Reference Example 28

### 3-Hydroxy-4-phenylbenzaldehyde

3-Methoxy-4-phenylbenzaldehyde (23.0g) was dissolved in dichloromethane (150mL). To the stirred mixture was added dropwise boron tribromide (15.4mL) under ice-cooling, and the mixture was stirred at room temperature for 2 hours. To the stirred reaction mixture was added dropwise water (20mL) under ice-cooling, and the resulting mixture was extracted with ethyl acetate. The organic layer was washed with brine and dried over anhydrous magnesium sulfate. The solvent was removed under reduced pressure. The obtained residue was suspended in methanol (100mL) and 2mol/L hydrochloric acid (50 mL), and the mixture was stirred at 50°C for 2 hours. To the reaction mixture was added brine, and the resulting mixture was extracted with ethyl acetate. The organic layer was washed with brine and dried over anhydrous magnesium sulfate. The solvent was removed under reduced pressure, and the obtained residue was purified by column chromatography on silica gel (eluent:ethyl acetate/hexane=1/5) to give the title compound (13.8g).
¹H-NMR (CDCl₃) δ ppm:
5.43 (1H, s), 7.30-7.65 (8H, m), 9.99 (1H, s)

### Reference Example 29

### 4-Fluoro-3-methoxymethoxybenzaldehyde

4-Fluoro-3-hydroxybenzaldehyde (11.9g) and N,N-diisopropylethylamine (44mL) were dissolved in dichloromethane (100mL). To the mixture was added chloromethylmethylether (13mL), and the mixture was stirred at room temperature for 3 hours. To the reaction mixture was added dropwise 1mol/L hydrochloric acid (250mL), and the resulting mixture was extracted with ethyl acetate. The organic layer was washed with brine and dried over anhydrous magnesium sulfate. The solvent was removed under reduced pressure to give the title compound (14.5g).
¹H-NMR (CDCl₃) δ ppm:
3.54 (3H, s), 5.29 (2H, s), 7.20-7.30 (1H, m), 7.50-7.60 (1H, m), 7.70-7.80 (1H, m), 9.92 (1H, s)

### Reference Example 30

### 3-Methoxymethoxy-4-(morpholin-4-yl)benzaldehyde

4-Fluoro-3-methoxymethoxybenzaldehyde (1.12g), morpholine (0.8mL), potassium carbonate (1.26g) and water (3mL) were suspended in dimethylsulfoxide (10mL), and the mixture was stirred at 100°C for 18 hours. To the reaction mixture was added a saturated aqueous sodium hydrogen carbonate solution, and the resulting mixture was extracted with ethyl acetate. The organic layer was washed with brine and dried over anhydrous magnesium sulfate. The solvent was removed under reduced pressure, and the obtained residue was purified by column chromatography on silica gel (eluent: ethyl acetate/hexane=1/5) to give the title compound (0.8g).
¹H-NMR (CDCl₃) δ ppm:
3.15-3.30 (4H, m), 3.53 (3H, s), 3.80-3.95 (4H, m), 5.27 (2H, s), 6.98 (1H, d, J=8. 2Hz), 7.51 (1H, dd, J=2.0Hz, 8.2Hz), 7.59 (1H, d, J=2.0Hz), 9.85 (1H, s)

### Reference Example 31

### 3-Hydroxy-4-(morpholin-4-yl)benzaldehyde

3-Methoxymethoxy-4-(morpholin-4-yl)benzaldehyde (0.3.5g) was dissolved in methanol (10 mL). To the mixture was added 2mol/L hydrochloric acid (5mL), and the mixture was stirred at 60°C for 18 hours. To the reaction mixture was added a saturated aqueous sodium hydrogen carbonate solution, and the resulting mixture was extracted with ethyl acetate. The organic layer was washed with brine and dried over anhydrous magnesium sulfate. The solvent was removed under reduced pressure to give the title compound (0.29g).
¹H-NMR (CDCl₃) δ ppm:
2.90-3.02 (4H, m), 3.80-3.95 (4H, m), 6.55-6.75 (1H, m), 7.20-7.30 (1H, m), 7.40-7.50 (2H, m), 9.91 (1H, s)

### Reference Example 32

### 3-(4-Benzyloxybutoxy)-4-(morpholin-4-yl)benzaldehyde

3-Hydroxy-4-(morpholin-4-yl)benzaldehyde (0.28g) and potassium carbonate (0.38g) were suspended in *N,N*-dimethylformamide (2 mL). To the reaction mixture was added benzyl 4-bromobutylether (0.36g), and the mixture was stirred at 50°C for 16 hours. To the reaction mixture was added water, and the resulting mixture was extracted with ethyl acetate. The organic layer was washed with brine and dried over anhydrous magnesium sulfate. The filtrate was concentrated under reduced pressure, and the obtained residue was purified by column chromatography onsilicagel(ethylacetate/hexane=1/5) to give the title compound (0.50g).
¹H-NMR (CDCl₃) δ ppm:
1.70-1.85 (2H, m), 1.90-2.05 (2H, m), 3.15-3.30 (4H, m), 3.55 (2H, t, J=6.3Hz), 3.80-3.95 (4H, m), 4.09 (2H, t, J=6.5Hz), 4.09 (2H, t, J=6.5Hz), 4.53 (2H, s), 6.94 (1H, d, J=8.3Hz), 7.20-7.50 (7H, m), 9.84 (1H, s)

### Reference Example 33

### 3-(N-t-Butoxycarbonylpiperidin-4-yloxy)benzaldehyde

3-Hydroxybenzaldehyde (0.98g), 1-*t*-butoxycarbonyl-4-hydroxypiperidine (2.41g) and triphenylphosphine (3.15g) were suspended in tetrahydrofuran (10mL) . To the mixture was added dropwise 40% azodicarboxylic acid diisopropyl ester-toluene solution (6.1mL) under ice-cooling, and the mixture was stirred at room temperature for 1hour. The solvent was removed under reduced pressure, and the obtained residue was purified by column chromatography on silica gel (eluent: ethyl acetate/hexane=1/3) to give the title compound (0.74g).
¹H-NMR (CDCl₃) δ ppm:
1.47 (9H, s), 1.70-1.82 (2H, m), 1.88-2.00 (2H, m), 3.30-3.40 (2H, m), 3.65-3.80 (2H, m), 4.50-4.60 (1H, m), 7.13-7.23 (1H, m), 7.35-7.50 (3H, m), 9.97 (1H, s)

### Reference Example 34

### 4-Ethoxy-3-hydroxybenzaldehyde

3,4-Dihydroxybenzaldehyde (101.6g) and potassium carbonate (101.7g) were suspended in *N,N*-dimethylformamide (500mL). To the mixture was added dropwise ethyl iodide (58.8mL) under ice-cooling, and the mixture was stirred at room temperature for 16 hours. To the reaction mixture was added dropwise 2mol/L hydrochloric acid (500mL), and the resulting mixture was extracted with ethyl acetate. The organic layer was washed with brine and dried over anhydrous magnesium sulfate. The solvent was removed under reduced pressure, and the obtained residue was purified by column chromatography on silica gel (eluent:ethyl acetate/hexane=1/1) to give the title compound (74.3g).
¹H-NMR (CDCl₃) δ ppm:
1.50 (3H, t, J=7.0Hz), 4.22 (2H, q, J=7.0Hz), 5.76 (1H, s), 6.95 (1H, d, J=8.1Hz), 7.35-7.50 (2H, m), 9.84 (1H, s)

### Reference Example 35

The following compound was prepared in a similar manner to that described in Reference Example 34 using the corresponding materials.
3-Hydroxy-4-propoxybenzaldehyde

### Reference Example 36

### 3-(3-Chloropropoxy)-4-ethoxybenzaldehyde

4-Ethoxy-3-hydroxybenzaldehyde (74.3g) and potassium carbonate (111.3g) were suspended in *N,N*-dimethylformamide (350mL). To the mixture was added dropwise 1-bromo-3-chloropropane (79.6mL) under ice-cooling, and the mixture was stirred at room temperature for 16 hours. To the reaction mixture was added dropwise 2mol/L hydrochloric acid (300mL), and the resulting mixture was extracted with ethyl acetate. The organic layer was washed with brine and dried over anhydrous magnesium sulfate. The solvent was removed under reduced pressure, and the obtained residue was purified by column chromatography on silica gel (eluent:ethyl acetate/hexane=1/5) to give the title compound (69.8g).
¹H-NMR (CDCl₃) δ ppm:
1.49 (3H, t, J=7.0Hz), 2.20-2.40 (2H, m), 3.78 (2H, t, J=6.3Hz), 4.17 (2H, t, J=7.0Hz), 4.22 (2H, t, J=5.8Hz), 6.96 (1H, d, J=8.1Hz), 7.38-7.52 (2H, m), 9.84 (1H, s)

### Reference Example 37

The following compounds were prepared in a similar manner to that described in Reference Example 36 using the corresponding materials.
3-(3-Chloropropoxy)-4-phenylbenzaldehyde
¹H-NMR (CDCl₃) δ ppm:
2.10-2.25 (2H, m), 3.50-3.70 (2H, m), 4.10-4.30 (2H, m), 7-30-7.70 (8H, m), 10.01 (1H, s)
4-Benzyloxy-3-(3-chloropropoxy)benzaldehyde
¹H-NMR (CDCl₃) δ ppm:
2.15-2.40 (2H, m), 3.65-3.85 (2H, m), 4.15-4.35 (2H, m), 5.21 (2H, s), 6.95-7.55 (8H, m), 9.84 (1H, s)
3-(3-Chloropropoxy)-4-methoxybenzaldehyde
3-(3-Chloropropoxy)benzaldehyde
3-(3-Chloropropoxy)-4-propoxybenzaldehyde
4-(3-Chloropropoxyphenyl)benzaldehyde

### Reference Example 38

### 3,5-Bis(4-acetoxybutoxy)benzaldehyde

3,5-Dihydroxybenzaldehyde (0.49g) and potassium carbonate (1.48g) were suspended in *N,N*-dimethylformamide (5mL). To the mixture was added 4-bromobutyl acetate (1.46g) under ice-cooling, and the mixture was stirred at 50°C for 16 hours. To the reaction mixture was added 1mol/L hydrochloric acid, and the resulting mixture was extracted with diethyl ether. The organic layer was washed with a saturated aqueous sodium hydrogen carbonate solution and brine successively and dried over anhydrous magnesium sulfate. The filtrate was concentrated under reduced pressure, and the obtained residue was purified by column chromatography on silica gel (ethyl acetate/hexane= 1/5) to give the title compound (0.30g).
¹H-NMR (CDCl₃) δ ppm:
1.75-1.95 (8H, m), 2.05 (6H, s), 3.95-4.25 (8H, m); 6.69 (1H, s), 6.99 (2H, s), 9.89 (1H, s)

### Reference Example 39

### 3-(4-Benzyloxybutylamino)benzonitrile

3-Aminobenzonitrile (0.45g) and potassium carbonate (1.05g) were suspended in *N,N*-dimethylformamide (5 mL). To the reactionmixture was addedbenzyl 4-bromobutyl ether (0.63g), and the mixture was stirred at 50°C for 16 hours. To the reaction mixture was added lmol/L hydrochloric acid, and the resulting mixture was extracted with diethyl ether. The organic layer was washed with a saturated aqueous sodium hydrogen carbonate solution and brine successively and dried over anhydrous magnesium sulfate. The filtrate was concentrated under reduced pressure, and the obtained residue was purified by column chromatography on silica gel (ethyl acetate/hexane=1/1) to give the title compound (0.45g).
¹H-NMR (CDCl₃) δ ppm:
1.65-1.85 (4H, m), 3.05-3.20 (2H, m), 3.45-3.60 (2H, m), 4.00-4.10 (1H, m), 4.52 (2H, s), 6.65-6.75 (2H, m), 6.91 (1H, d, J=7.6Hz), 7.17 (1H, t, J=7.6Hz), 7.23-7.45 (5H, m)

### Reference Example 40

### 3-[N-(4-Benzyloxybutyl)-N-methylamino]benzonitrile

3-(4-Benzyloxybutylamino)benzonitrile (0.22g) and potassium carbonate (0.21g) were suspended in *N,N*-dimethylformamide (5 mL). To the reaction mixture was added methyl iodide (0.16g), and the mixture was stirred at 50°C for 16 hours. To the reaction mixture was added 1mol/L hydrochloric acid, and the resulting mixture was extracted with diethyl ether. The organic layer was washed with a saturated aqueous sodium hydrogen carbonate solution and brine successively and dried over anhydrous magnesium sulfate. The filtrate was concentrated under reduced pressure, and the obtained residue was purified by column chromatography on silica gel (ethyl acetate/hexane=1/1) to give the title compound (0.22 g)
¹H-NMR (CDCl₃) δ ppm:
1.55-1.80 (9H, m), 2.93 (3H, s), 3.25-3.40 (2H, m), 3.95-3.55 (2H, m), 4.51 (2H, s), 6.75-6.95 (3H, m), 7.15-7.40 (6H, m)

### Reference Example 41

### 3-(4-Acetoxybutylsulfanyl)benzonitrile

3-Mercaptobenzonitrile (0.40g) and potassium carbonate (0.61g) were suspended in *N,N*-dimethylformamide (5 mL). To the reaction mixture was added 4-bromobutyl acetate (0.63g), and the mixture was stirred at room temperature for 16 hours. To the reaction mixture was added 1mol/L hydrochloric acid, and the resulting mixture was extracted with diethyl ether. The organic layer was washed with a saturated aqueous sodium hydrogen carbonate solution and brine successively and dried over anhydrous magnesium sulfate. The filtrate was concentrated under reduced pressure, and the obtained residue was purified by column chromatography on silica gel (ethyl acetate/hexane=1/5) to give the title compound (0.75g).
¹H-NMR (CDCl₃) δ ppm:
1.65-1.90 (4H, m), 2.05 (3H, s), 2.94-3.05 (2H, m), 4.03-4.20 (2H, m), 7.30-7.60 (4H, m)

### Reference Example 42

### 3-(4-Hydroxybutylsulfanyl)benzylamine

Lithium aluminum hydride (0.20g) was suspended in tetrahydrofuran (15mL). To the mixture was added 3-(4-acetoxybutylsulfanyl)benzonitrile (0.75g) under ice-cooling. The mixture was stirred at 60°C for 2 hours, and the mixture was cooled under ice-cooling. To the reaction mixture were added dropwise ethanol and water successively, and then added diethyl ether. To the reaction mixture was added anhydrous sodium sulfate, and the insoluble material was removed by filtration. The filtrate was concentrated under reduced pressure to give the title compound (0.22g).
¹H-NMR (CDCl₃) δ ppm:
1.60-1.85 (4H, m), 2.97 (2H, t, J=7.1Hz), 3.67 (2H, t, J=6.1Hz), 3.8-5 (2H, s), 7.05-7.40 (4H, m)

### Reference Example 43

The following compounds were prepared in a similar manner to that described in Reference Example 42 using the corresponding materials.
3-(4-Benzyloxybutylamino)benzylamine
¹H-NMR (CDCl₃) δ ppm:
1.60-1.80 (4H, m), 3.05-3.25 (2H, m), 3.44-3.60 (2H, m), 3.77 (2H, s), 4.51 (2H, s), 6.46 (1H, d, J=7.9Hz), 6.52 (1H, s), 6.62 (1H, d, J=7.6Hz), 7.14 (1H, dd, J=7.6Hz, 7.9Hz), 7.29-7.45 (5H, m)
3-[*N*-(4-Benzyloxybutyl)-*N*-methylamino]benzylamine
¹H-NMR (CDCl₃) δ ppm:
1.55-1.80 (4H, m), 2.92 (3H, s), 3.25-3.40 (2H, m), 3.43-3.58 (2H, s), 3.79 (2H, s), 4.50 (2H, s), 6.50-6.70 (3H, m), 7.10-7.45 (6H, m)

### Reference Example 44

### 3-Benzyloxy-4-formylbenzonitrile

4-Formyl-3-hydroxybenzonitrile (4.0g) and potassium carbonate (3.76g) were suspended in *N,N*-dimethylformamide (20 mL). To the reaction mixture was added benzylbromide (3.6mL), and the mixture was stirred at room temperature for 16 hours. To the reaction mixture was added lmol/L hydrochloric acid, and the resulting mixture was extracted with diethyl ether. The organic layer was washed with a saturated aqueous sodium hydrogen carbonate solution and brine successively and dried over anhydrous magnesium sulfate. The filtrate was concentrated under reduced pressure, and the obtained residue was purified by column chromatography on silica gel (ethyl acetate/hexane=1/1) to give the title compound (3.0g).
¹H-NMR (CDCl₃) δ ppm:
5.23 (2H, s), 7.30-7.50 (7H, m), 7.93 (1H, d, J=7.9Hz), 10.55 (1H, s)

### Reference Example 45

### 3-Benzyloxy-4-(3-hydroxypropyl)benzylamine

3-Benzyloxy-4-formylbenzonitrile (0.93g) and (carboethoxymethyl)triphenylphosphoniumbromide (2.52g) were suspended in *N,N*-dimethylformamide (10mL). To the reaction mixture was added potassium *t*-butoxide (0.66g), and the mixture was stirred at room temperature for 18 hours. To the reaction mixture was added 1mol/L hydrochloric acid, and the resulting mixture was extracted with ethyl acetate. The organic layer was washed with brine and dried over anhydrous magnesium sulfate. The filtrate was concentrated under reduced pressure. The obtained residue was dissolved in tetrahydrofuran (10 mL), and the mixture was added dropwise to a suspension of lithium aluminum hydride (0.20g) in tetrahydrofuran (15mL) under ice-cooling. The mixture was stirred at 60°C for 2 hours. After mixture was cooled in an ice bath, and to the reaction mixture were added dropwise ethanol and water successively, and then added diethyl ether. To the reaction mixture was added anhydrous sodium sulfate, and the insoluble material was removed by filtration. The filtrate was concentrated under reduced pressure to give the title compound (0.20g).
¹H-NMR (CDCl₃) δ ppm:
1.75-1.95 (2H, m), 2.75 (2H, t, J=7.3Hz), 3. 58 (2H, t, J=6.2Hz), 3.85 (2H, t, J=6.2Hz), 5.10 (2H, s), 6.88 (1H, d, J=7.6Hz), 6.96 (1H, s), 7.14 (1H, d, J=7.6Hz), 7.25-7.55 (5H, m)

### Reference Example 46

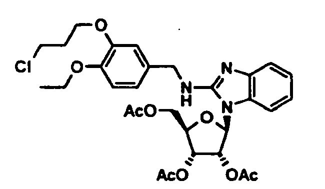

### 2-[3-(3-Chloropropoxy)-4-ethoxybenzylamino]-1-(2,3,5-tri-O-acetyl-β-D-ribofuranosyl)-1H-benzimidazole

2-Amino-1-(2, 3, 5-tri-*O*-acetyl-β-D-ribofuranosyl)-*1H-*benzimidazole (13.7g) and 3-(3-chloropropoxy)-4-ethoxybenzaldehyde (15.3g) were suspended in tetrahydrofuran (150 mL), and the mixture was stirred at 70°C for 20 hours. To the stirred reaction mixture was added sodium triacetoxyborohydride (21.7g) under ice-cooling, and the mixture was stirred at room temperature for 24 hours. To the reaction mixture was added water, and the resulting mixture was extracted with ethyl acetate. The organic layer was washed with brine and dried over anhydrous magnesium sulfate, and the filtrate was concentrated under reduced pressure. The obtained residue was purified by column chromatography on silica gel (eluent: ethyl acetate/hexane=1/3) to give the title compound (16.8g).
¹H-NMR (CDCl₃) δ ppm:
1.42 (3H, t, J=7.1Hz), 1.83 (3H, s), 1.97 (3H, s), 2.15 (3H, s), 2.20-2.30 (2H, m), 3.76 (2H, t, J=6.6Hz), 3.95-4.40 (6H, m), 4.47 (1H, dd, J=3.6Hz, 12.4Hz), 4.68 (2H, s), 5.20-5.45. (2H, m), 5.57 (1H, dd, J=6.8Hz, 7.4Hz), 6.01 (1H, d, J=7.4Hz), 6. 83 (1H, d, J=8.1Hz), 6.92 (1H, dd, J=2.1Hz, 8.1Hz), 6.99 (1H, d, J=2.1Hz), 7.02-7.25 (3H, m), 7.99 (1H, d, J=7.8Hz)

### Reference Example 47

The following compounds were prepared in a similar manner to that described in Reference Example 4 6 using the corresponding materials.
2-[3-(3-Chloropropoxy)benzylamino)-1-(2,3;5-tri-*O*-acetyl-β-D-ribofuranosyl)-*1H*-benzimidazole
¹H-NMR (CDCl₃) δ ppm:
1.81 (3H, s), 1.98 (3H, s), 2.15 (3H, s), 2.18-2.30 (2.H, m), 3.73 (2H, t, J=6.6Hz), 4.10 (2H, t, J=6.0Hz), 4.22 (1H, dd, J=2.4Hz, 12.5Hz), 4.30-4.38 (1H, m), 4.49 (1H, d, J=3.4Hz, 12.5Hz), 4.65-4.85 (2H, m), 5.30-5.45 (2H, m), 5.56 (1H, dd, J=6.4Hz, 7.6Hz), 6.03 (1H, d, J=7.6Hz), 6.81 (1H, dd, J=2.2Hz, 8.1Hz), 6.90-7.00 (2H, m), 7.03-7.30 (4H, m), 7.49 (1H, d, J=7.6Hz)
2-[4-Benzyloxy-3-(3-chloropropoxy)benzylamino]-1-(2,3,5-tri-*O*-acetyl-β-D-ribofuranosyl)-*1H*-benzimidazole
¹H-NMR (CDCl₃) δ ppm:
1.80 (3H, s), 1.97 (3H, s), 2.15 (3H, s), 2.18-2.30 (2H, m), 3.74 (2H, t, J=6.5Hz), 4.05-4.40 (4H, m), 4.46 (1H, d, J=3.5Hz, 12.5Hz), 4.67 (2H, d, J=5.2Hz), 5.10 (2H, s), 5.25-5.40 (2H, m), 5.57 (1H, dd, J=6.5Hz, 7.3Hz), 6.00 (1H, d, J=7.3Hz), 6.80-7.60 (12H, m)
2-[3-(3-Chloropropoxy)-4-phenylbenzylamino]-1-(2, 3, 5-tri-*O*-acetyl-β-D-ribofuranosyl)-*1H*-benzimidazole
¹H-NMR (CDCl₃) δ ppm:
1.83 (3H, s), 1.98 (3H, s), 2.05-2.25 (5H, m), 3.55-3.65 (2H, m), 4.00-4.40 (4H,m), 4.52 (1H, d, J=3.4Hz, 12.5Hz), 4.70-4.90 (2H, m), 5.30-5.50 (2H, m), 5.50 (1H, dd, J=6.5Hz, 7.8Hz), 6.04 (1H, d, J=7.8Hz), 7.00-7.60 (12H, m)
2-{4-[3-(3-Chloropropoxy)phenyl]benzylamino}-1-(2,3,5-tri-*O*-acetyl-β-ribofuranosyl)-*1H*-benzimidazole
¹H-NMR (CDCl₃) δ ppm:
1.77 (3H, s), 2.00 (3H, s), 2.16 (3H, s), 2.20-2.35 (2H, m), 3.60-3.85 (2H, m), 4.10-4.30 (4H, m), 4.50 (1H, d, J=3.5Hz, 12.7Hz), 4.75-4.90 (2H, m), 5.30-5.50 (2H, m), 5.57 (1H, dd, J=6.5Hz, 7.7Hz), 6.04 (1H, d, J=7.7Hz), 6.85-7.60 (12H, m)
2-[3-(3-Chloropropoxy)-4-methoxybenzylamino]-1-(2, 3, 5-tri-*O*-acetyl-β-D-ribofuranosyl)-*1H*-benzimidazole
2-[3-(3-Chloropropoxy)-4-propoxybenzylamino]-1-(2,3,5-tri-*O*-acetyl-β-D-ribofuranosyl)-*1H*-benzimidazole

### Example 1

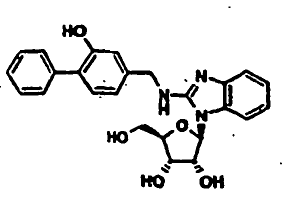

### 2-(3-Hydroxy-4-phenylbenzylamino)-1-(β-D-ribofuranosyl)-1H-benzimidazole

2-Chloro-1-(β-D-ribofuranosyl)-*1H*-benzimidazole (0.21g) and 3-hydroxy-4-phenylbenzylamine (0.19g) were suspended in isobutanol (5 mL). To the mixture was added triethylamine (0.36mL), and the mixture was stirred for reflux for 16 hours. The reaction mixture was concentrated under reduced pressure, and the obtained residue was purified by column chromatography on silica gel (eluent: methanol/ethyl acetate=1/20) to give the title compound (0.1g).
¹H-NMR (DMSO-d₆) δ ppm:
3.60-3.80 (2H, m), 3.90-4.20 (2H, m), 4.35-4.65 (3H, m), 5.21 (1H, d, J=4.4Hz), 5.25 (1H, d, J=7.4Hz), 5.56 (1H, t, J=4.0Hz), 5.82 (1H, d, J=7.4Hz), 6.80-7.01 (4H, m), 7.10-7.60 (9H, m), 9.44 (1H, s)

### Examples 2-11 and Reference Example 48

The compounds of Tables 1 and 2 were prepared in a similar manner to that described in Example 1 using the corresponding materials.

**[Table 1]**

| Example No. | Structure | ¹H-NMR δ ppm: |
|---|---|---|
| Example 2 | 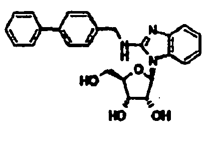 | (DMSO-d₆)3.60-3.80 (2H, m), 3.95-4.03 (1H, m). 4.06-4.18 (1H, m), 4.35-4.50 (1H, m), 4.55-4.70 (2H, m), 5.19 (1H, d, J=4.4Hz), 5.29 (1H, d, J=7.4Hz), 5.60 (1H, t, J=4.5Hz), 5.83 (1H, d, J=7.3Hz), 6.80-7.03 (2H, m), 7.17 (1H, d, J=7.3Hz), 7.29 (1H, d, J=7.3Hz), 7.34 (1H, t, J=7.4Hz), 7.40-7.48 (4H, m), 7.50 (1H, t,J=6.1Hz), 7.57-7.67 (4H, m) |
| Example 3 | 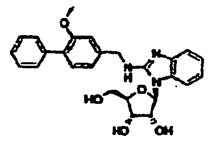 | (DMSO-d₆)3.60-3.80 (5H, m), 3.95-4.20 (2H, m), 4.40-4.50 (1H, m), 4.61 (2H, d, J=6.2Hz), 5.21 (1H, d, J=4.4Hz), 5.31 (1H, d, J=7.6Hz), 5.63 (1H, t, J=4.4Hz), 5.84 (1H, d, J=7.8Hz), 6.80-7.07 (3H, m), 7.10-7.46 (9H, m), 7.50 (1H, t,J=6.2Hz) |
| Example 4 | 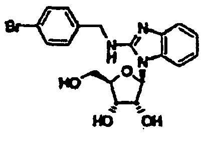 | (DMSO-d₆)3.60-3.80 (2H, m), 3.95-4.02 (1H, m), 4.05-4.15 (1H, m), 4.30-4.45 (1H, m), 4.46-4.64 (2H, m), 5.19 (1H, d, J=4.2Hz), 5.29 (1H, d, J=7.5Hz), 5.57 (1H, t, J=4.3Hz), 5.80 (1H, d, J=7.8Hz), 6.88 (1H, t, J=7.5Hz), 6.93 (1H, t, J=7.5Hz), 7.16 (1H, d, J=7.5Hz), 7.28 (1H, d, J=7.5Hz), 7.32 (1H, d, J=8.4Hz), 7.40-7.60 (3H, m) |
| Example 5 | 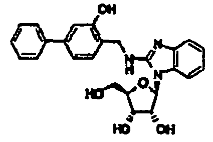 | (DMSO-d₆)3.60-3.80 (2H, m), 3.96-4.04 (1H, m), 4.07-4.16 (1H, m), 4.30-4.60 (3H, m), 5.22 (1H, d, J=4.4Hz), 5.35 (1H, d, J=7.6Hz), 5.78 (1H, t, J=4,1Hz), 5.80 (1H, d, J=7.7Hz), 6.90-7.10 (4H, m), 7.20-7.37 (4H, m), 7.39-7.47 (2H, m), 7.55-7.62 (2H, m), 11.74 (1H, s) |
| Example 6 | 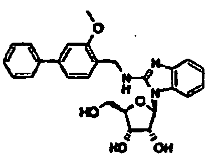 | (DMSO-d₆)3.60-3.75 (2H, m), 3.94 (3H, s), 3.96-4.04 (1H, m), 4.08-4.16 (1H, m), 4.40-4.70 (3H, m), 5.22 (1H, d, J=4.4Hz), 5.36 (1H, d, J=7.6Hz), 5.55 (1H, t, J=4.4Hz), 5.85 (1H, d, J=7.6Hz), 6-88 (1H, t, J=7.6Hz), 6.94 (1H, t, J=7.6Hz), 7.10-7.50 (9H, m), 7.60-7.70 (2H, m) |
| Example 7 | 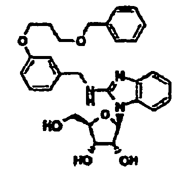 | (DMSO-d₆)1.85-2.05 (2H, m), 3.57 (2H, t, J=6.3Hz), 3.60-3.80 (2H, m), 3.95-4.15 (4H, m), 4.35-4.60 (5H, m), 5.81 (1H, d, J=7.4Hz), 6.70-7.50 (14H, m) |

**[Table 2]**

| ExampleNo. | Structure | ¹H-NMR δ ppm: |
|---|---|---|
| Example 8 | 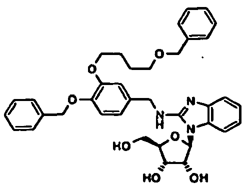 | (DMSO-d₆)1.60-1.85 (4H, m), 3.46 (2H, t, J=6.2Hz), 3.60-3.75 (2H, m), 3.90-4.02 (3H, m), 4.06-4.14 (1H, m), 4.35-4.53 (5H, m), 5.04 (2H, s), 5.18 (1H, d, J=4.5Hz), 5.26 (1H, d, J=7.5Hz), 5.56 (1H, t, J=4.6Hz), 5.80 (1H, d, J=7.4Hz), 6.80-7.06 (5H, m), 7.10-7.50 (13H, m) |
| Reference Example 48 | 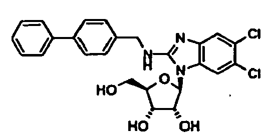 | (DMSO-d₆)3.60-3.80 (2H, m), 3.95-4.15 (2H, m), 4.30-4.45 (1H, m), 4.61 (2H, d, J=6.0Hz), 5.25 (1H, d, J=4.5Hz), 5.32 (1H, d, J=7.6Hz), 5.68 (1H, t, J=4.5Hz), 5.83 (1H, d, J=7.7Hz), 7.34 (1H, t, J=7.4Hz), 7.37 (1H, s), 7.40-7.49 (4H, m), 7.55-7.66 (4H, m), 7.70 (1H, s), 7.83(1H, t, J=6.0Hz) |
| Example 9 | 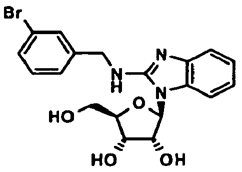 | (CD₃OD)3.75-3.90 (2H, m), 4.05-4.15 (1H, m), 4.26 (1H, dd, J=2.4Hz, 5.9Hz), 4.58 (1H, dd, J=5.9Hz, 7.3Hz), 4.61 (2H, s), 5.97 (1H, d, J=7.3Hz), 6.90-7.10 (2H, m), 7.15-7.45 (5H, m), 7.57 (1H, s) |
| Example 10 | 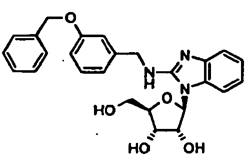 | (CDCl₃)3.60-3.80 (2H, m), 4.05-4.15 (4H, m), 4.55-4.65 (1H, m), 5.01 (2H, s), 5.80 (1H, d, J=6.9Hz), 6.08 (1H, brs), 6.70-6.90 (3H, m), 6.95-7.50 (10H, m) |
| Example 11 | 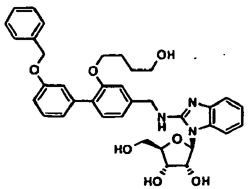 | (DMSO-d₆)1.43-1.55 (2H, m), 1.60-1.75 (2H, m), 3.33-3.45 (2H, m), 3.60-3.80 (2H, m), 3.90-4.20 (4H, m), 4.35-4.50 (2H, m), 4.59 (2H, d, J=6.0Hz), 5.11 (2H, s); 5.21 (1H, d, J=4.5Hz), 5.29 (1H, d, J=7.5Hz), 5.63 (1H, t, J=4.5Hz), 5.83 (1H, d, J=7.2Hz), 6.80-7.60 (17H, m) |

### Example 12

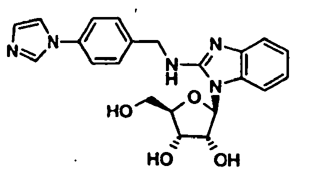

### 2-[4-(1H-Imidazol-1-yl)benzylamino]-1-(β-D-ribofuranosyl)-1H-benzimidazole

2-Amino-1-(2,3,5-tri-*O*-acetyl-β-D-ribofuranosyl)-1H-benzimidazole (94mg) and 4-(1H-imidazol-1-yl)benzaldehyde (41mg) were suspended in tetrahydrofuran (3mL), and the mixture stirred at room temperature for 2 hours. To the reaction mixture was added acetic acid (200µL), and then was added sodium triacetoxyborohydride (56mg). The mixture was stirred at room temperature for 24 hours. After adding water to the reaction mixture, and the mixture was concentrated under reduced pressure. The obtained residue was dissolved in ethanol (2mL). To the mixture was added 5mol/L aqueous sodium hydroxide solution (0.5mL), and the mixture was stirred at room temperature for 1 hour. To the reaction mixture was added acetic acid (1mL), and the mixture was concentrated under reduced pressure. The obtained residue was purified by preparative reverse phase column chromatography (Shiseido CAPSELL PAC C18UG80, 5µm, 20×50mm, flow rate 30mL/minutes linear gradient, water/methanol=70/30-10/90)to give the title compound (67mg) .
¹H-NMR (CD₃OD) δ ppm:
3.75-3.90 (2H, m), 4.08-4.18 (1H, m), 4.26 (1H, dd, J=2.1Hz, 5.6Hz), 4.60 (1H, dd, J=5.6Hz, 7.6Hz), 4.66 (1H, d, J=15.8Hz), 4.71 (1H, d, J=15.8Hz), 5.97 (1H, d, J=7.6Hz), 6.94-7.07 (2H, m), 7.12 (1H, s), 7.20-7.32 (2H, m), 7.45-7.60 (5H, m), 8.51 (1H, s)

### Example 13

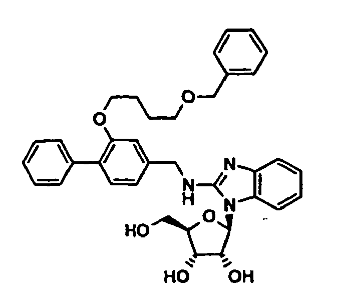

2-[3-(4-Benzyloxybutoxy)-4-phenylbenzylamino]-1-(β-D-ribofuranosyl)-1*H*-benzimidazole 2-(3-Hydroxy-4-phenylbenzylamino)-1-(β-D-ribofuranosyl)-1*H*-benzimidazole (70mg) and potassium carbonate (65mg) were suspended in *N,N*-dimethylformamide (1mL). To the mixture was added benzyl 4-bromobutylether (45µL), and the mixture was stirred at 50°C for 16 hours. The insoluble material was removed by filtration, and the reaction mixture was concentrated under reduced pressure. The obtained residue was purified by column chromatography on silica gel (eluent :methanol/ethyl acetate=1/20) to give the title compound (54mg).
¹H-NMR (DMSO-d₆) δ ppm:
1.50-1.80 (4H, m), 3.39 (2H, t, J=6.3Hz), 3.60-3.80 (2H, m), 3.90-4.20 (4H, m), 4.39 (2H, s), 9.41-4.50 (1H, m), 4.59 (2H, d, J=5.9Hz), 5 . 21 (1H, d, J=4.4Hz), 5.31 (1H, d, J=7.1Hz), 5.62 (1H, t, J=4.4Hz), 5.83 (1H, d, J=7.5Hz), 6.89 (1H, t, J=7.6Hz), 6.95 (1H, t, J=7.6Hz), 7.02 (1H, d, J=7.6Hz), 7.10-7.60 (15H, m)

### Examples 14-26

The compounds of Tables 3 to 6 were prepared in a similar manner to that described in Example 13 using the corresponding materials.

**[Table 3]**

| ExampleNo. | Structure | ¹H-NMR δ ppm: |
|---|---|---|
| Example 14 | 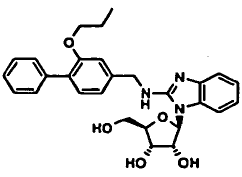 | (DMSO-d₆)0.89 (3H, t, J=7.5Hz), 1.50-1.70 (2H, m), 3.60-3.80 (2H, m), 3.92 (2H, t, J=6.1Hz), 3.98-4.05 (1H, m), 4.08-4.16 (1H, m), 4.40-4.50 (1H, m), 4.59 (2H, d, J=6.0Hz), 5.83 (1H, d, J=7.4Hz), 6.89 (1H, t, J=7.6Hz), 6.95 (1H, t, J=7.6Hz), 7.01 (1H, d, J=7.6Hz), 7.13 (1H, s), 7.17 (1H, d, J=7.6Hz), 7.22 (1H, d, J=7.3Hz), 7.24-7.55 (7H, m) |
| Example 15 | 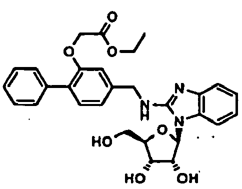 | (DMSO-d₆)1.16 (3H, t, J=7.1 Hz), 3.60-3.80 (2H, m), 3.95-4.20 (4H, m), 4.35-4.50 (1H, m), 4.58 (2H, d, J=7.0Hz), 4.75 (2H, s), 5.21 (1H, d, J=4.4Hz), 5.28 (1H, d, J=7.2Hz), 5.61 (1H, t, J=4.5Hz), 5.83 (1H, d, J=7.5Hz), 6.89 (1H, t, J=7.6Hz), 6.95 (1H, t, J=7.6Hz), 7.02 (1H, s), 7.05 (1H, d, J=7.6Hz), 7.17 (1H, d, J=7.6Hz), 7.25 (1H, d, J=7.8Hz), 7.26-7.58 (7H, m) |

**[Table 4]**

| ExampleNo. | Structure | ¹H-NMR δ ppm: |
|---|---|---|
| Example 16 | 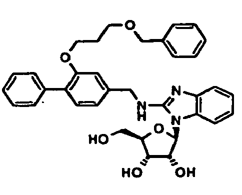 | (DMSO-d₆)1.80-2.00 (2H, m), 3.48 (2H, t, J=6.3Hz), 3.60-3.80 (2H, m), 3.95-4.02 (1H, m), 4.04 (2H, t J=6.0Hz), 4.09-4.15 (1H, m), 4.41 (2H, s), 4.40-4.48 (1H, m), 4.55-4.65 (2H, m), 5.20 (1H, d, J=4.4Hz), 5.29 (1H, d, J=7.1 Hz), 5.63 (1H, t, J=4.4Hz), 5.83 (1H, d, J=7.7Hz), 6.89 (1H, t, J=7.6Hz), 6.95 (1H, t, J=7.6Hz), 7.02 (1H, d, J=7.6Hz), 7.10-7.60 (15H, m) |
| Example 17 | 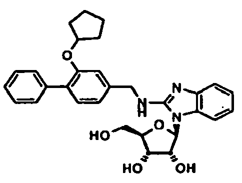 | (DMSO-d₆)1.40-1.90 (8H, m), 3.60-3.80 (2H, m), 3.95-4.05 (1H, m), 4.08-4.15 (1H, m), 4.40-4.50 (1H, m), 4.80 (2H, d, J=6.2Hz), 4.70-4.85 (1H, m), 5.21 (1H, d, J=4.4Hz), 5.29 (1H, d, J=7.7z). 5.64 (1H, t, J=4.4Hz), 5.83 (1H, d, J=7.6Hz), 6.89 (1H, t, J=7.6Hz), 6.95 (1H, t, J=7.6Hz), 6.99 (1H, d, J=7.6Hz), 7.07-7.60 (10H, m) |
| Example 18 | 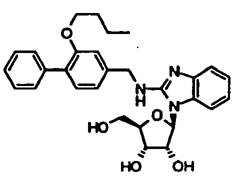 | (DMSO-d₆)0.85 (3H, t, J=7.4Hz), 1.25-1.43 (2H, m), 1.53-1.70 (2H, m), 3.60-3.80 (2H, m), 3.90-4.20 (4H, m), 4.40-4.50 (1H, m), 4.55-4.65 (2H, m), 5.21 (1H, d, J=4.4Hz), 5.29 (1H, d, J=7.1Hz), 5.63 (1H, t, J=4.4Hz), 5.83 (1H, d, J=7.6Hz), 6.89 (1H, t, J=7.6Hz), 6.95 (1H, t, J=7.6Hz), 7.01 (1H, d, J=7.6Hz), 7.08-7.60 (10H, m) |
| Example 19 | 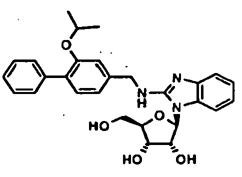 | (DMSO-d₆)1.10-1.30 (6H, m), 3.60-3.80 (2H, m), 3.95-4.05 (1H, m), 4.08-4.16 (1H, m), 4.35-4.65 (4H, m), 5.21 (1H, d, J=4.4Hz), 5.29 (1H, d, J=7.5Hz), 5.64 (1H, t, J=4.4Hz), 5.83 (1H, d, J=7.5Hz), 6.89 (1H, t, J=7.6Hz), 6.95 (1H, t, J=7.6Hz), 6.99 (1H, d, J=7.6Hz), 7.10-7.60 (10H, m) |
| Example 20 | 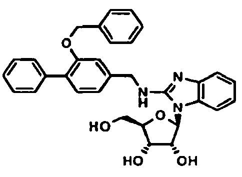 | (DMSO-d₆)3.60-3.80 (2H, m), 3.95-4.05 (1H, m), 4.08-4.16 (1H, m), 4.40-4.52 (1H, m), 4.56-4.70 (2H, m), 5.09 (2H, s), 5.22 (1H, d, J=4.4Hz), 5.31 (1H, d, J=7.4Hz), 5.63 (1H, t, J=4.4Hz), 5.85 (1H, d, J=7.5Hz), 6.89 (1H, t, J=7.6Hz), 6.96 (1H, t, J=7.6Hz), 7.04 (1H, d, J=7.6Hz), 7.15-7.60 (15H, m) |

**[Table 5]**

| ExampleNo. | Structure | ¹H-NMR δ ppm: |
|---|---|---|
| Example 21 | 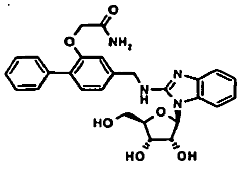 | (DMSO-d₆)3.60-3.80 (2H, m), 3.96-4.04 (1H, m), 4.08-4.16 (1H, m), 4.35-4.50 (3H, m), 4.58 (2H, d, J=6.0Hz), 5.23 (1H, d, J=4.5Hz), 5.32 (1H, d, J=7.3Hz), 5.62 (1H, t, J=4.4Hz), 5.83 (1H, d, J=7.6Hz), 6.89 (1H, t, J=7.6Hz), 6.95 (1H, t, J=7.6Hz), 7.00-7.60 (13H, m) |
| Example 22 | 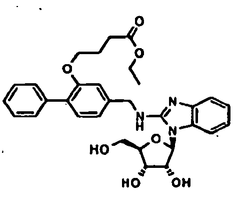 | (DMSO-d₆)1.15 (3H, t, J=7.0Hz), 1.80-1.95 (2H, m), 2.30-2.40 (2H, m), 3.60-3.80 (2H, m), 3.90-4.20 (6H, m), 4.35-4.50 (1H, m), 4.58 (2H, d, J=7.0Hz), 5.21 (1H, d, J=4.4Hz), 5.28 (1H, d, J=7.2Hz), 5.61 (1H, t, J=4.5Hz), 5.83 (1H, d, J=7.5Hz), 6.89 (1H, t, J=7.6Hz), 6.95 (1H, t, J=7.6Hz), 7.02 (1H, d, J=7.6Hz), 7.12 (1H, s), 7.17 (1H, d, J=7.6Hz), 7.22 (1H, d, J=7.6Hz), 7.26-7.58 (7H, m) |
| Example 23. | 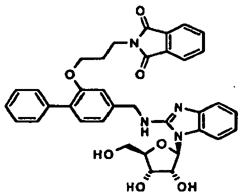 | (DMSO-d₆)1.90-2.10 (2H, m), 3.60-3.80 (4H, m), 3.90-4.20 (4H, m), 4.35-4.50 (1H, m), 4.58 (2H, d, J=7.0Hz), 5.21 (1H, d, J=4.4Hz), 5.28 (1H, d, J=7.2Hz), 5.63 (1H, t, J=4.5Hz), 5.82 (1H, d, J=7.5Hz), 6.89 (1H, t, J=7.6Hz), 6.95 (1H, t, J=7.6Hz), 7.02 (1H, d, J=7.6Hz), 7.08 (1H, s), 7.16 (1H, d, J=7.6Hz), 7.18-7.40 (5H, m), 7.45-7.60 (3H, m), 7.75-7.90 (4H, m) |
| Example 24 | 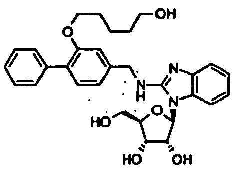 | (DMSO-d₆)1.25-1.45 (4H, m), 1.55-1.70 (2H, m), 3.25-3.40 (2H, m), 3.60-3.80 (2H, m), 3.90-4.20 (4H, m), 4.35-4.50 (1H, m), 4.59 (2H, d, J=6.3Hz), 5.21 (1H, d, J=4.4Hz), 5.30 (1H, d, J=7.5Hz), 5.63 (1H, t, J=4.4Hz), 5.83 (1H, d, J=7.5Hz), 6.88 (1H, t, J=7.6Hz), 6.95 (1H, t, J=7.6Hz), 7.01 (1H, d, J=7.6Hz), 7.10-7.60 (10H, m) |
| Example 25 | 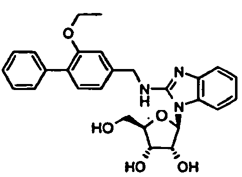 | (DMSO-d₆)1.24 (3H, t, J=6.9Hz), 3.60-3.80 (2H, m), 3.95-4.20 (4H, m), 4.35-4.50 (1H, m), 4.59 (2H, d, J=5.8Hz), 5.83 (1H, d, J=7.8Hz), 6.88 (1H, t, J=7.6Hz), 6.95 (1H, t, J=7.6Hz), 7.01 (1H, d, J=7.6Hz), 7.12 (1H, s), 7.17 (1H, d, J=7.6Hz), 7.21 (1H, d, J=7.9Hz), 7.24-7.55 (7H, m) |

**[Table 6]**

| ExampleNo. | Structure | ¹H-NMR δ ppm: |
|---|---|---|
| Example 26 | 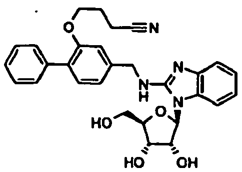 | (CD₃OD)1.90-2.02 (2H, m), 2.40 (2H, t, J=7.2Hz), 3.78-3.90 (2H, m), 4.04 (2H, t, J=5.7Hz), 4.10-4.20 (1H, m), 4.28 (1H, dd, J=2.1 Hz, 5.6Hz), 4.55-4.75 (3H, m), 5.98 (1H, d, J=7.0Hz), 6.90-7.50 (13H, m) |

### Example 27

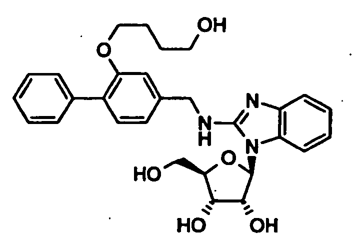

### 2-[3-(4-Hydroxybutoxy)-4-phenylbenzylamino]-1-(β-D-ribofuranosyl)-1H-benzimidazole

2-[3-(4-Benzyloxybutoxy)-4-phenylbenzylamino]-1-(β-D-ribofuranosyl)-1*H*-benzimidazole (35mg) was dissolved in ethanol (5mL). To the solution was added a catalytic amount of 10% palladium-carbon powder, and the mixture was stirred at 60°C under a hydrogen atmosphere for 24 hour. The insoluble material was removed by filtration, and the solvent of the filtrate was removed under reduced pressure to give the title compound (21mg).
¹H-NMR (DMSO-d₆) δ ppm:
1.40-1.55 (2H, m), 1.60-1.75 (2H, m), 3.30-3.45 (2H, m), 3.60-3.80 (2H, m), 3.90-4.20 (4H, m), 4.35-4.50 (2H, m), 4.59 (2H, d, J=6.3Hz), 5.21 (1H, d, J=4.4Hz), 5.30 (1H, d, J=7.5Hz), 5.63 (1H, t, J=4.4Hz), 5.83 (1H, d, J=7.5Hz), 6.88 (1H, t, J=7. 6Hz), 6.95 (1H, t, J=7. 6Hz), 7.01 (1H, d, J=7.6Hz), 7.10-7.60 (10H, m)

### Examples 28-32

The compounds of Table 7 were prepared in a similar manner to that described in Example 27 using the corresponding materials.

**[Table 7]**

| ExampleNo. | Structure | ¹H-NMR δ ppm: |
|---|---|---|
| Example 28 | 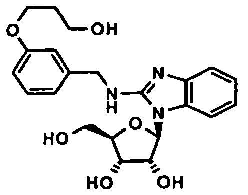 | (DMSO-d₆)1.75-1.95 (2H, m), 3.48-3.58 (2H, m), 3.62-3.76 (2H, m), 3.96-4.03 (3H, m), 4.07-4.14 (1H, m), 4.36-4.46 (1H, m), 4.49-4.57 (2H, m), 5.21 (1H, d, J=4.4Hz), 5.30 (1H, d, J=7.4Hz), 5.60 (1H, t, J=4.4Hz), 5.81 (1H, d, J=7.4Hz), 6.70-7.00 (5H, m), 7.16 (1H, d, J=7.8Hz), 7.19 (1H, t, J=7.8Hz), 7.28 (1H, d, J=7.8Hz), 7.45 (1H, t, J=6.5Hz) |
| Example 29 | 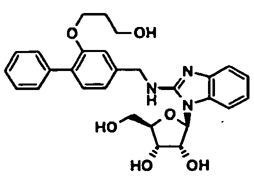 | (DMSO-d₆)1.70-1.85 (2H, m), 3.40-3.55 (2H, m), 3.65-3.80 (2H, m), 3.95-4.20 (4H, m), 4.35-4.42 (2H, m), 4.59 (2H, d, J=6.3Hz), 5.21 (1H, d, J=4.4Hz), 5.30 (1H, d, J=7.4Hz), 5.63 (1H, t, J=4.4Hz), 5.83 (1H, d, J=7.5Hz), 6.88 (1H, t, J=7.6Hz), 6.95 (1H, t, J=7.6Hz), 7.01 (1H, d, J=7.6Hz), 7.10-7.60 (10H, m) |
| Example 30 | 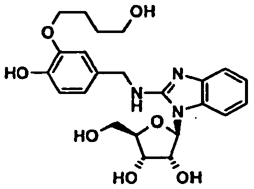 | (DMSO-d₆)1.47-1.62 (2H, m), 1.66-1.80 (2H, m), 3.43 (2H, t, J=6.8Hz), 3.60-3.75 (2H, m), 3.85-4.02 (3H, m), 4.05-4.15 (1H, m), 4.30-4.50 (3H, m), 5-79 (1H, d, J=7.7Hz), 6.69 (1H, d, J=7.9Hz), 6.75 (1H, dd, J=1.8Hz, 7.9Hz), 6.80-7.00 (3H, m), 7.10-7.40 (3H, m) |
| Example 31 | 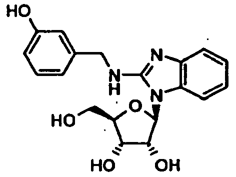 | (CD₃OD)3.70-3.90 (2H, m), 4.05-4.15 (1H, m), 4.25 (1H, dd, J=2.3Hz, 5.7Hz), 4.45-4.70 (3H, m), 5.95 (1H, d, J=7.8Hz), 6.63 (1H, dd, J=2.0Hz, 8.0Hz), 6.75-6.90 (2H, m), 6.94-7.15 (3H, m), 7.10-7.22 (2H, m) |
| Example 32 | 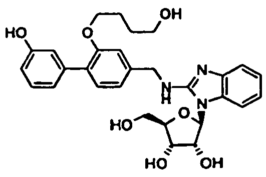 | (DMSO-d₆)1.40-1.55 (2H, m), 1.60-1.75 (2H, m), 3.33-3.45 (2H, m), 3.60-3.80 (2H, m), 3.90-4.20 (4H, m), 4.30-4.50 (2H, m), 4.59 (2H, d, J=5.9Hz), 5.22 (1H, d, J=3.9Hz), 5.31 (1H, d, J=7.4Hz), 5.63 (1H, t, J=4.0Hz), 5.83 (1H, d, J=7.7Hz), 6.67 (1H, dd, J=1.8Hz, 7.8Hz), 6.80-7.03 (5H, m), 7.08-7.25 (4H, m), 7.29 (1H, d, J=7.8Hz), 7.45 (1H, t, J=5.9Hz), 9.29 (1H, s) |

### Example 33

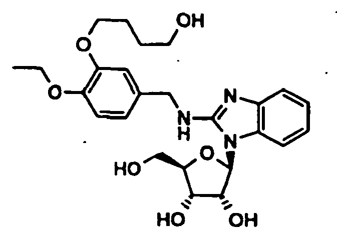

2-[4-Ethoxy-3-(4-hydroxybutoxy)benzylamino]-1-(β-D-ribofuranosyl)-1*H*-benzimidazole 2-[4-Hydroxy-3-(4-hydroxybutoxy)benzylamino]-1-(β-D-ribofuranosyl)-1*H*-benzimidazole (30mg) and potassium carbonate (18mg) was suspended in *N,N*-dimethylformamide (0.7mL). To the mixture was added ethyl iodide (20µL), and the mixture was stirred at 55°C for 16 hour. The insoluble material was removed by filtration, and the reaction mixture was concentrated under reduced pressure. The obtained residue was purified by preparative reverse phase column chromatography (Shiseido CAPSELL PAC C18UG80, 5µm, 20×50mm; flow rate 30mL/minutes linear gradient, water/methanol =90/10-10/90) to give the title compound (13 mg).
¹H-NMR (CD₃OD) δ ppm:
1.36 (3H, t, J=7.1Hz), 1.56-1.90 (4H, m), 3.58 (2H, t, J=6.5Hz), 3.73-3.90 (2H, m), 3.94-4.16 (5H, m), 4.24 (1H, dd, J=2.3Hz, 5.7Hz), 4.43-4.65 (3H, m), 5.94 (1H, d, J=7.6Hz), 6.80-7.10. (5H, m), 7.20-7.35 (2H, m)

### Examples 34-47

The compounds of Tables 8 to 11 were prepared in a similar manner to that described in Example 33 using the corresponding materials.

**[Table 8]**

| ExampleNo. | Structure | ¹H-NMR δ ppm: |
|---|---|---|
| Example 34 | 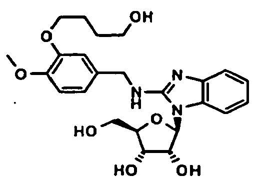 | (CD₃OD)1.56-1.90 (4H, m), 3.57 (2H, t, J=6.5Hz), 3.70-3.85 (5H, m), 3.94-4.05 (2H, m), 4.08-4.15 (1H, m), 4.24 (1H, dd, J=2.3Hz, 5.7Hz), 4.45-4.65 (3H, m), 5.94 (1H, d, J=7.8Hz), 6.80-7.10 (5H, m), 7.20-7.35. (2H, m) |

**[Table 9]**

| ExampleNo. | Structure | ¹H-NMR δ ppm: |
|---|---|---|
| Example 35 | 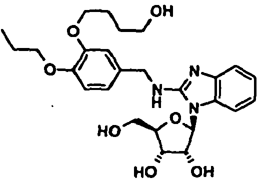 | (CD₃OD)1.02 (3H, t, J=7.4Hz), 1.56-1.90 (6H, m), 3.58 (2H, t, J=6.5Hz), 3.73-3.85 (2H, m), 3.91 (2H, t, J=6.5Hz), 3.99 (2H, t, J=6.5Hz), 4.05-4.15 (1H, m), 4.24 (1H, dd, J=2.3Hz, 5.8Hz), 4.43-4.65 (3H, m), 5.94 (1H, d, J=7.7Hz), 6.80-7.10 (5H, m), 7.15-7.35 (2H, m) |
| Example 36 | 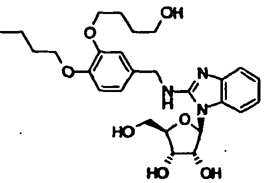 | (CD₃OD)0.97 (3H, t, J=7.4Hz), 1.40-1.55 (2H, m), 1.60-1.90 (6H, m), 3.58 (2H, t, J=6.6Hz), 3.55-3.85 (2H, m), 3.96 (2H, t, J=6.5Hz), 4.00 (2H, t, J=6.4Hz), 4.05-4.15 (1H, m), 4.25 (1H, dd, J=2.4Hz, 5.9Hz), 4.45-4.65 (3H, m), 5.95 (1H, d, J=7.5Hz), 6.80-7.10 (5H, m), 7.20-7.35 (2H, m) |
| Example 37 | 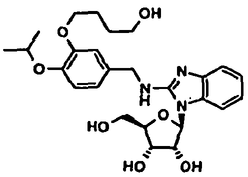 | (CD₃OD)1.26 (6H, d, J=7.8Hz), 1.60-1.90 (4H, m), 3.58 (2H, t, J=6.4Hz), 3.75-3.85 (2H, m), 4.00 (2H, t, J=6.3Hz), 4.05-4.15 (1H, m), 4.25 (1H, dd, J=2.3Hz, 5.7Hz), 4.35-4.65 (4H, m), 5.95 (1H, d, J=7.4Hz), 6.80-7.10 (5H, m), 7.20-7.35 (2H, m) |
| Example 38 | 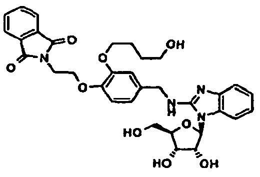 | (CD₃OD)1.45-1.75 (4H, m), 3.50 (2H, t, J=6.5Hz), 3.70-3.95 (4H, m), 4.00-4.40 (6H, m), 4.43-4.65 (3H, m), 5.94 (1H, d, J=7.7Hz), 6.80-7.10 (5H, m), 7.15-7.35 (2H, m), 7.50-7.70 (4H, m) |
| Example 39 | 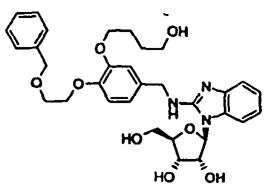 | (CD₃OD)1.55-1.90 (4H, m), 3.55 (2H, t, J=6.4Hz), 3.70-3.90 (4H, m), 4.01 (2H, t, J=6.4Hz), 4.05-4.20 (3H, m), 4.24 (1H, dd, J=2.3Hz, 5.9Hz), 4.40-4.70 (5H, m), 5.94 (1H, d, J=7.4Hz), 6.80-7.10 (5H, m), 7.15-7.45 (7H, m) |
| Example 40 | 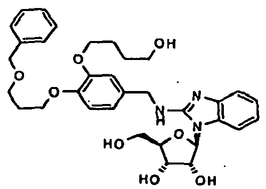 | (CD₃OD)1.55-1.85 (4H, m), 1.90-2.10 (2H, m), 3.56 (2H, t, J=6.5Hz), 3.67 (2H, t, J=6.2Hz), 3.72-3.88 (2H, m), 3.92 (2H, t, J=6.4Hz), 4.07 (2H, t, J=6.1Hz), 4.09-4.15 (1H, m), 4.24 (1H, dd, J=2.4Hz, 5.9Hz), 4.40-4.70 (5H, m), 5.95 (1H, d, J=7.5Hz), 6.80-7.10 (5H, m), 7.15-7.45 (7H, m) |

**[Table 10]**

| ExampleNo. | Structure | ¹H-NMR δ ppm: |
|---|---|---|
| Example 41 | 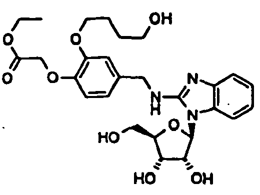 | (CD₃OD) 1.25 (3H, t, J=7.0Hz), 1.55-1.90 (4H, m), 3.58 (2H, t, J=6.4Hz), 3.73-3.85 (2H, m), 4.03 (2H, t, J=6.4Hz), 4.05-4.15 (1H, m), 4.21 (2H, q, J=7.0Hz), 4.25 (1H, dd, J=2.4Hz, 5.9Hz), 4.43-4.70 (5H, m), 5.96 (1H, d, J=7.3Hz), 6.80-7.15 (5H, m), 7.20-7.40 (2H, m) |
| Example 42 | 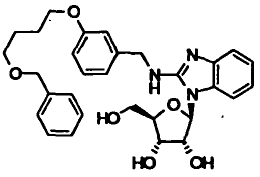 | (CD₃OD) 1.65-1.90 (4H, m), 3.49 (2H, t, J=6.3Hz), 3.73-3.88 (2H, m), 3.94 (2H, t, J=6.3Hz), 4.05-4.17 (1H, m), 4.25 (1H, dd, J=2.2Hz, 5.7Hz), 4.45 (2H, s), 4.50-4.70 (3H, m), 5.95 (1H, d, J=7.0Hz), 6.73 (1H, dd, J=1.7Hz, 8.9Hz), 6.90-7.08 (4H, m), 7.13-7.38 (8H, m) |
| Example 43 | 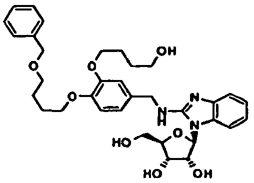 | (CD₃OD) 1.60-1.90 (8H, m), 3.50-3.65 (4H, m), 3.75-3.85 (2H, m), 3.93-4.15 (5H, m), 4.24 (1H, dd, J=2.4Hz, 5.8Hz), 4.45-4.65 (5H, m), 5.94 (1H, d, J=7.7Hz), 6.80-7.10 (5H, m), 7.19-7.50 (7H, m) |
| Example 44 | 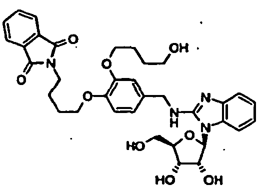 | (DMSO-d₆)1.44-1.58 (2H, m), 1.62-1.83 (6H, m), 3.30-3.45 (2H, m), 3.57-3.75 (4H, m), 3.85-4.00 (5H, m), 4.05-4.15 (1H, m), 4.30-4.52 (4H, m), 5.18 (1H, d, J=3.9Hz), 5.26 (1H, d, J=7.2Hz), 5.56 (1H, t, J=4.3Hz), 5.79 (1H, d, J=7.4Hz), 6.75-7.01 (5H, m), 7.16 (1H, d, J=7.5Hz), 7.26 (1H, d, J=7.5Hz), 7.33 (1H, t, J=6.0Hz), 7.75-7.95 (4H, m) |
| Example 45 | 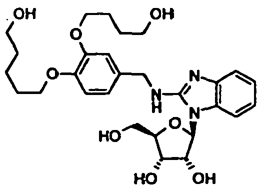 | (DMSO-d₆)1.35-1.80 (10H, m), 3.30-3.50 (4H, m), 3.60-3.76 (2H, m), 3.85-4.02 (5H, m), 4.05-4.15 (1H, m), 4.30-4.55 (5H, m), 5.19 (1H, d, J=3.9Hz), 5.26 (1H, d, J=7.2Hz), 5.56 (1H, t, J=4.3Hz), 5.79 (1H, d, J=7.6Hz), 6.80-7.05 (5H, m), 7.16 (1H, d, J=7.9Hz), 7.26 (1H, d, J=7.9Hz), 7.34 (1H, t, J=6.1 Hz) |

**[Table 11]**

| Example No. | Structure | ¹H-NMR δ ppm: |
|---|---|---|
| Example 46 | 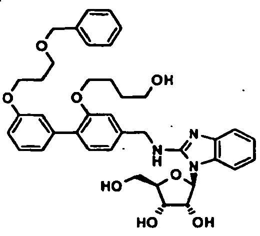 | (DMSO-d₆) 1.40-1.55 (2H, m), 1.60-1.75 (2H, m), 1.90-2.05 (2H, m), 3.30-3.45 (2H, m), 3.53-3.63 (2H, m), 3.65-3.80 (2H, m), 3.90-4.20 (6H, m), 4.37 (1H, t, J=5.1 Hz), 4.40-4.52 (3H, m), 4.59 (2H, d, J=6.0Hz), 5.22 (1H, d, J=3.9Hz), 5.30 (1H, d, J=7.4Hz), 5.63 (1H, t, J=4.0Hz), 5.83 (1H, d, J=7.6Hz), 6.80-7.40 (16H, m), 7.49 (1H, t, J=6.0Hz) |
| Example 47 | 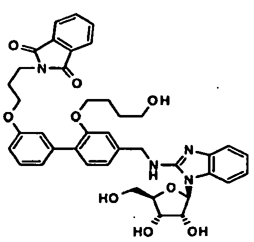 | (DMSO-d₆) 1.40-1.53 (2H, m), 1.58-1.73 (2H, m), 1.98-2.12 (2H, m), 3.30-3.43 (2H, m), 3.63-3.82 (4H, m), 3.88-4.18 (6H, m), 4.36 (1H, t, J=5.1 Hz), 4.40-4.50 (1H, m), 4.59 (2H, d, J=6.1 Hz), 5.22 (1H, d, J=3.9Hz), 5.31 (1H, d, J=7.4Hz), 5.63 (1H, t, J=4.0Hz), 5.83 (1H, d, J=7.4Hz), 6.71 (1H, dd, J=1.7Hz, 8.0Hz), 6.80-7.35 (10H, m), 7.49 (1H, t, J=6.1 Hz), 7.70-7.90 (4H, m) |

### Example 48

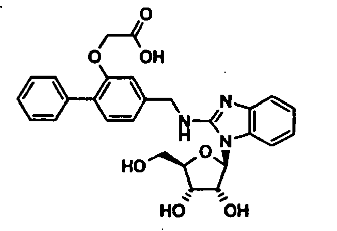

### 2-(3-Carboxymethyloxy-4-phenylbenzylamino)-1-(β-D-ribofuranosyl)-1H-benzimidazole

2-(3-Ethoxycarbonylmethyloxy-4-phenylbenzylamino)-1-(β-D-ribofuranosyl)-1*H-*benzimidazole (53mg) was dissolved in tetrahydrofuran (5mL). To the reaction mixture was added 2mol/L aqueous sodium hydroxide solution (1mL), and the mixture was stirred 60°C for 1 hour. To the reaction mixture was added 2mol/L hydrochloric acid (1mL), and the solvent was removed under reduced pressure. The obtained residue was purified by preparative reverse phase column chromatography (Shiseido CAPSELL PAC C18UG80, 5µm, 20×50mm, flow rate 30mL/minutes linear gradient, water/methanol =70/30-10/90) to give the title compound (20mg).
¹H-NMR (DMSO-d₆) δ ppm:
3.60-3.80 (2H, m), 3.96-4.04 (1H, m), 4.08-4.16 (1H, m), 4.35-4.50 (1H, m), 4.58 (2H, d, J=6.0Hz), 4.66 (2H, s,), 5.83 (1H, d, J=7.6Hz), 6.89 (1H, t, J=7.6Hz), 6.95 (1H, t, J=7.6Hz), 7.00-7.60 (11H, m)

### Example 49

The compound of Table 12 was prepared in a similar manner to that described in Example 48 using the corresponding materials.

**[Table 12]**

| ExampleNo. | Structure | ¹H-NMR δ ppm: |
|---|---|---|
| Example 49 | 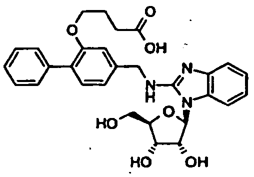 | (DMDO-d₆)1.75-1.95 (2H, m), 2.20-2.40 (2H, m), 3.60-3.80 (2H, m), 3.90-4.20 (4H, m), 4.35-4.50 (1H, m), 4.58 (2H, d, J=7.0Hz), 5.83 (1H, d, J=7.5Hz), 6.89 (1H, t, J=7.6Hz), 6.95 (1H, t, J=7.6Hz), 7.02 (1H, d, J=7.6Hz), 7.12 (1H, s), 7.17 (1H, d, J=7.6Hz), 7.22 (1H, d, J=7.6Hz), 7.26-7.58 (7H, m), 11.70-12.40 (1H, m) |

### Example 50

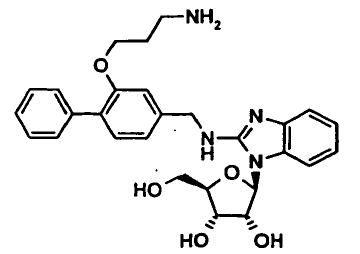

### 2-[3-(3-Aminopropoxy)-4-phenylbenzylamino]-1-(β-D-ribofuranosyl)-1H-benzimidazole

2-[3-(3-Phthalimidepropoxy)-4-phenylbenzylamino]-1-(β-D-ribofuranosyl)-1*H-*benzimidazole (53mg) was dissolved in methanol (5mL). To the mixture was added hydrazine monohydrate (0.5mL), and the mixture was stirred at 90°C for 6hours. The solvent was remove under reduced pressure, and the obtained residue was purified by preparative reverse phase column chromatography (Shiseido CAPSELL PAC C18UG80, 5µm, 20×50mm, flow rate 30mL/minutes linear gradient, water/methanol =70/30-10/90) to give the title compound (22mg).
¹H-NMR (DMSO-d₆) δ ppm:
1.60-1.88 (2H, m), 2.50-2.70 (2H, m), 3.60-3.80 (2H, m), 3.95-4.20 (4H, m), 4.40-4.50 (1H, m), 4.55-4.65 (2H, m), 5.84 (1H, d, J=7.5Hz), 6.88 (1H, t, J=7.6Hz), 6.95 (1H, t, J=7.6Hz), 7.01 (1H, d, J=7.6Hz), 7.10-7.60 (10H, m)

### Examples 51-52

The compounds of Table 13 were prepared in a similar manner to that described in Example 50 using the corresponding materials.

**[Table 13]**

| ExampleNo. | Structure | ¹H-NMR δ ppm: |
|---|---|---|
| Example 51 | 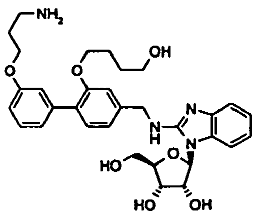 | (DMSO-d₆) 1.40-1.55 (2H, m), 1.60-1.83 (4H, m), 2.68 (2H, t, J=6.5Hz), 3.38 (2H, t, J=6.5Hz), 3.60-3.80 (2H, m), 3.90-4.06 (5H, m), 4.08-4.16 (1H, m), 4.38-4.49 (1H, m), 4.59 (2H, d, J=6.0Hz), 5.22 (1H, d, J=3.9Hz), 5.30 (1H, d, J=7.4Hz), 5.63 (1H, t, J=4.0Hz), 5.83 (1H, d, J=7.5Hz), 6.80-7.32 (11H, m), 7.49 (1H, t, J=6.0Hz) |
| Example 52 | 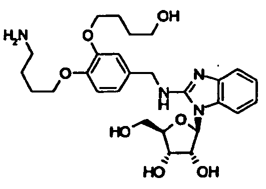 | (DMSO-d₆) 1.40-1.80 (8H, m), 2.57 (2H, t, J=6.5Hz), 3.44 (2H, t, J=6.5Hz), 3.60-3.75 (2H, m), 3.85-4.02 (5H, m), 4.05-4.15 (1H, m), 4.35-4.45 (1H, m), 4.47 (2H, d, J=5.9Hz), 5.19 (1H, d, J=3.9Hz), 5.27 (1H, d, J=7.2Hz), 5.57 (1H, t, J=4.3Hz), 5.79 (1H, d, J=7.2Hz), 6.80-7.05 (5H, m), 7.16 (1H, d, J=7.9Hz), 7.27 (1H, d, J=7.9Hz), 7.34 (1H, t, J=5.9Hz) |

### Example 53

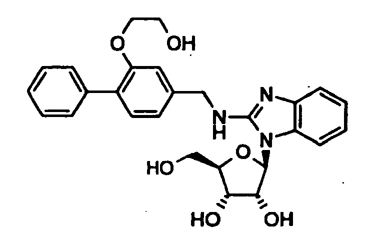

### 2-[3-(2-Hydroxyethyloxy)-4-phenylbenzylamino]-1-(β-D-ribofuranosyl)-1H-benzimidazole

2-(3-Ethoxycarbonylmethyloxy-4-phenylbenzylamino)-1-(β-D-ribofuranosyl)-1*H-*benzimidazole (53mg) was dissolved in methanol (5mL). To the mixture was added sodium tetrahydroborate (8mg), and the mixture was stirred at room temperature for 1 hours. To the reaction mixture was added 1mol/L hydrochloric acid (0.5mL), and the solvent was removed under reduced pressure. The obtained residue was purified by preparative reverse phase column chromatography (Shiseido CAPSELL PACC18UG80, 5µm, 20×50mm, flow rate 30mL/minutes linear gradient, water/methanol =70/30-10/90) to give the title compound (21mg).
¹H-NMR (DMSO-d₆) δ ppm:
3.60-3.80 (4H, m), 3.93-4.20 (4H, m), 4.38-4.50 (1H, m), 4.59 (2H, d, J=5.8Hz), 4.73 (1H, t, J=5.2Hz), 5.21 (1H, d, J=4.4Hz), 5.29 (1H, d, J=7.2Hz), 5.63 (1H, t, J=3.8Hz), 5.83 (1H, d, J=7.2Hz), 6.89 (1H, t, J=7.6Hz), 6.95 (1H, t, J=7.6Hz), 7.02 (1H, d, J=7.6Hz), 7.13 (1H, s), 7.18 (1H, d, J=7.6Hz), 7.23 (1H, d, J=7.6Hz), 7.25-7.60 (7H, m)

### Example 54

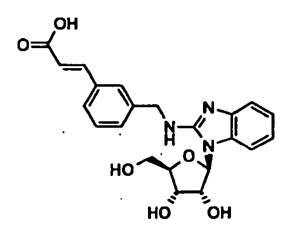

### 2-[3-(2-Carboxyvinyl)benzylamino]-1-(β-D-ribofuranosyl)-1H-benzimidazole

2-(3-Bromobenzylamino)-1-(β-D-ribofuranosyl)-1*H-*benzimidazole (200mg), acrylic acid (112mg), palladium acetate (10mg) and tri-o-tolylphosphine (28mg) were suspended in acetonitrile (2mL). To the mixture was added triethylamine (0.3mL), and the mixture was stirred.at 100°C for 10 hours. The insoluble material was removed by filtration, and the solvent was removed under reduced pressure. The obtained residue was purified by preparative reverse phase column chromatography (Shiseido CAPSELL PAC C18UG80, 5µm, 20×50mm, flow rate 30mL/minutes linear gradient, water/methanol =70/30-10/90) to give the title compound (52mg).
¹H-NMR (CD₃OD) δ ppm:
3.75-3.90 (2H, m), 4.10-4.20 (1H, m), 4.26 (1H, dd, J=2.2Hz, 5.7Hz), 4.60 (1H, dd, J=5.7Hz, 7.5Hz), 4.64 (1H, d, J=15.9Hz), 4.69 (1H, d, J=15.9Hz), 5.99 (1H, d, J=7.5Hz), 6.48 (1H, d, J=16.0Hz), 6.99-7.12 (2H, m), 7.20-7.52 (5H, m), 7.60 (1H, d, J=16.0Hz), 7.62 (1H, s)

### Example 55

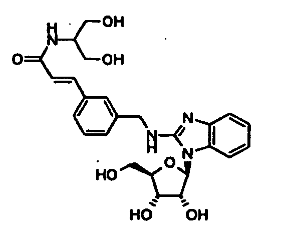

2-{3-[2-(2-Hydroxy-1-hydroxymethyethylcarbamoyl)vinyl]-benzylamino}-1-(β-D-ribofuranosyl)-1*H-*benzimidazole 2-[3-(2-Carboxyvinyl)benzylamino]-1-(β-D-ribofuranosyl)-1*H-*benzimidazole (50mg), 2-amino-1,3-propanediol (21mg), 1-hydroxybenzotriazole (36mg) and triethylamine (41µL) were suspended in tetrahydrofuran (2mL). To the mixture was added 1-ethyl-3-(3-dimethylaminopropyl) carbodiimide hydrochloride (45mg), and the mixture was stirred for 17 hours. The solvent was removed under reduced pressure, and the obtained residue was purified by preparative reverse phase column chromatography (Shiseido CAPSELL PAC C18UG80, 5µm, 20×50mm, flow rate 30mL/minutes linear gradient, water/methanol =70/30-10/90) togive the title compound (52mg)
¹H-NMR (CD₃OD) δ ppm:
3.66 (4H, d, J=5.4Hz), 3.75-3.90 (2H, m), 3.99-4.16 (2H, m), 4.27 (1H, dd, J=2.6Hz, 5.8Hz), 4.60 (1H, dd, J=5. 8Hz, 7.4Hz), 4.63 (1H, d, J=15.9Hz), 4.69 (1H, d, J=15.9Hz), 5.97 (1H, d, J=7.4Hz), 6.65 (1H, d, J=15.7Hz), 6.95-7.10 (2H, m), 7.20-7.47 (5H, m), 7.52 (1H, d, J=15.7Hz), 7.59 (1H, s)

### Example 56

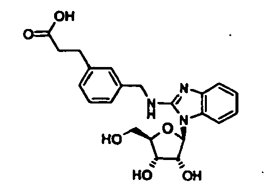

### 2-[3-(2-Carboxyethyl)benzylamino]-1-(β-D-ribofuranosyl)-1H-benzimidazole

2-[3-(2-Carboxyvinyl)benzylamino]-1-(β-D-ribofuranosyl)-1*H-*benzimidazole (30mg) was dissolved in methanol (2mL). To the solution was added a catalytic amount of 10% palladium-carbon powder, and the mixture was stirred at room temperature under a hydrogen atmosphere for 1 hour. The insoluble material was removed by filtration, and the solvent of the filtrate was removed under reduced pressure to give the title compound (25mg).
¹H-NMR (CD₃OD) β ppm:
2.54 (2H, t, J=7.6Hz), 2.89 (2H, t, J=7.6Hz), 3.70-3.90 (2H, m), 4.05-4.18 (1H, m), 4.25 (1H, dd, J=2.3Hz, 5.7Hz), 4.50-4.75 (3H, m), 5.97 (1H, d, J=7.3Hz), 7.00-7.15 (3H, m), 7.16-7.40 (5H, m)

### Example 57

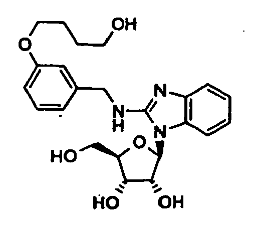

### 2-[3-(4-Hydroxybutyloxy)benzylamino]-1-(-β-D-ribofuranosyl)-1H-benzimidazole

2-[3-(4-Benzyloxybutyloxy)benzylamino)-1-(β-D-ribofuranosyl)-1*H-*benzimidazole (24mg) was dissolved in ethanol (2mL). To the solution was added a catalytic amount oaf 10% palladium-carbon powder, and the mixture was stirred at 60°C under a hydrogen atmosphere for 24 hour. The insoluble material was removed by filtration, and the solvent of the filtrate was removed under reduced pressure to give the title compound (20mg).
¹H-NMR (CD₃OD) δ ppm:
1.60-1.90 (4H, m), 3.57 (2H, t, J=6.6Hz), 3.75-3.90 (2H, m), 3.96 (2H, t, J=6.4Hz), 4.05-4.10 (1H, m), 4.25 (1H, dd, J=2.6Hz, 5.7Hz), 4.50-4.70 (3H, m), 5.95 (1H, d, J=7.3Hz), 6.75 (1H, dd, J=1.6Hz, 8.3Hz), 6.90-7.08 (4H, m), 7.13-7.35 (3H, m)

### Examples 58-61

The compounds of Table 14 were prepared in a similar manner to that described in Example 57 using the corresponding materials.

**[Table 14]**

| ExampleNo. | Structure | ¹H-NMR 6 ppm: |
|---|---|---|
| Example 58 | 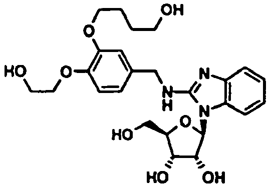 | (CD₃OD) 1.55-1.90 (4H, m), 3.58 (2H, t, J=6.4Hz), 3.74-3.90 (4H, m), 3.95-4.18 (5H, m), 4.24 (1H, dd, J=2.3Hz, 5.7Hz), 4.45-4.70 (3H, m), 5.95 (1H, d, J=7.7Hz), 6.85-7.15 (5H, m), 7.20-7.25 (2H, m) |
| Example 59 | 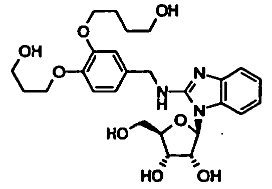 | (CD₃OD) 1.55-2.10 (6H, m), 3.58 (2H, t, J=6.5Hz), 3.60-3.90 (4H, m), 3.95-4.20 (5H, m), 4.24 (1H, dd, J=2.1Hz, 5.7Hz), 4.45-4.65 (3H, m), 5.95 (1H, d, J=7.4Hz), 6.80-7.15 (5H, m), 7.20-7.35 (2H, m) |
| Example 60 | 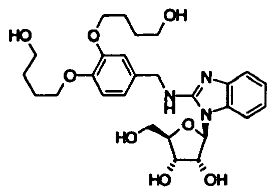 | (DMSO-d₆) 1.48-1.60 (4H, m), 1.65-1.80 (4H, m), 3.35-3.50 (4H, m), 3.60-3,75 (2H, m), 3.85-4.00 (5H, m), 4.05-4.15 (1H, m), 4.35-4.44 (3H, m), 4.47 (2H, d, J=6.0Hz), 5.19 (1H, d, J=3.9Hz), 5.27 (1H, d, J=7.2Hz), 5.57 (1H, t, J=4.3Hz), 5.79 (1H, d, J=7.4Hz), 6.82-6.90 (3H, m), 6.95 (1H, t, J=7.6Hz), 6.99 (1H, s), 7.17 (1H, d, J=7.6Hz), 7.27 (1H, d, J=7.6Hz), 7.34 (1H, t, J=6.0Hz) |
| Example 61 | 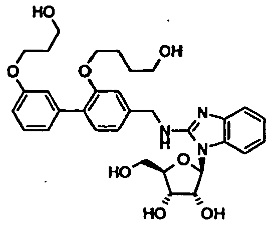 | (DMSO-d₆) 1.40-1.55 (2H, m), 1.60-1.75 (2H, m), 1.80-1.90 (2H, m), 3.33-3.44 (2H, m), 3.49-3.60 (2H, m), 3.64-3.80 (2H, m), 3.90-4.20 (6H, m), 4.35-4.55 (3H, m), 4.59 (2H, d, J=6.2Hz), 5.22 (1H, d, J=3.9Hz), 5.31 (1H, d, J=7.4Hz), 5.63 (1H, t, J=4.0Hz)_{,} 5.83 (1H, d, J=7.5Hz), 6.78-7.35 (11H, m), 7.49 (1H, t, J=6.2Hz) |

### Example 62

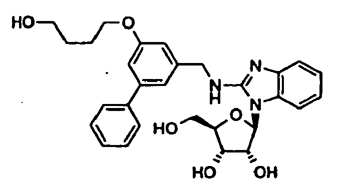

### 2-[3-(4-Hydroxybutoxy)-5-phenylbenzylamino]-1-(β-D-ribofuranosyl)-1H-benzimidazole

2-Amino-1-(2,3,5-tri-*O-*acetyl-β-D-ribofuranosyl)-1H-benzimidazole (0.20g) and 3-(4-acetoxybutoxy)-5-phenylbenzaldehyde (0.19g) were suspended in tetrahydrofuran (3mL), and the mixture was stirred at room temperature for 13 hours. To the reaction mixture was added sodium triacetoxyborohydride (0.21g), and the mixture was stirred at room temperature for 24 hours. After adding water to the reaction mixture, the mixture was concentrated under reduced pressure. The obtained residue was dissolved in methanol (2mL). To the reaction mixture was added 5mol/L aqueous sodium hydroxide solution (0.5mL), and the mixturewas stirredat roomtemperature for 1 hour. To the reaction mixture was added acetic acid (1mL), and the mixture was concentrated under reduced pressure. The obtained residue was purified by preparative reverse phase column chromatography (Shiseido CAPSELL PAC C18UG80, 5µm, 20×50mm, flow rate 30mL/minutes linear gradient, water/methanol =70/30-10/90) to give the title compound (0.08g).
¹H-NMR (DMSO-d₆) δ ppm:
1.45-1.85 (9H, m), 3.60-3.78 (2H, m), 3.93-4.15 (4H, m), 4.34-4.45 (1H, m), 4.50-4.65 (2H, m), 5.87 (1H, d, J=7.6Hz), 6.80-7.05 (4H, m), 7.10-7.50 (6H, m), 7.55-7.70 (3H, m)

### Examples 63-69

The compounds of Table 15 were prepared in a similar manner to that described in Example 62 using the corresponding materials.

**[Table 15]**

| Example No. | Structure | ¹H-NMR δ ppm |
|---|---|---|
| Example 63 | 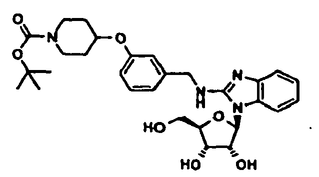 | (DMSO-d₆) 1.30-1.55 (11H, m), 1.78-1.95 (2H, m), 3.00-3.20 (2H, m), 3.55-3.80 (4H, m), 3.95-4.04 (1H, m), 4.08-4.16 (1H, m), 4.35-4.62 (4H, m), 5.82 (1H, d, J=7.7Hz), 6.75-7.02 (5H, m), 7.10-7.55 (4H, m) |
| Example 64 | 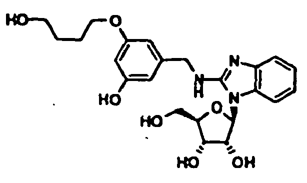 | (DMSO-d₆) 1.45-1.58 (2H, m), 1.62-1.75 (2H, m). 3.60-3.75 (2H, m). 3.86 (2H, t, J=6.6Hz). 3.95-4.03 (1H, m), 4.06-4.16 (1H, m). 4.35-4.55 (3H, m), 5.20-5.40 (2H, m), 5.61 (1H, t, J=4.0Hz). 5.81 (1H, d. J=7.5Hz), 6.15 (1H, s). 6.35 (2H, s). 6.87 (1H, t J=7.6Hz), 6.95 (1H, t, J=7.6Hz), 7.16 (1H, d, J=7.6Hz), 7.28 (1H. d J=7.6Hz), 7.41 (1H, t, J=6.1Hz), 9.32 (1H, s) |
| Example 65 | 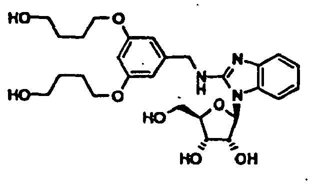 | (DMSO-d₆) 1.45-1.60 (4H, m), 1.62-1.80 (4H, m). 3.35-3.50 (4H, m), 3.60-3.78 (4H, m), 3.85-4.04 (5H, m), 4.06-4.15 (1H, m). 4.35-4.55 (5H. m), 527 (1H, d, J=4.0Hz), 5.34 (1H, d, J=7.1 Hz), 5.62 (1H, t, J=4.1Hz), 5.81 (1H, d, J=7.5Hz), 6.31 (1H, t, J=2.2Hz), 6.86 (1H, d, J=2.2Hz), 6.88 (1H, d, J=7.6Hz), 6.95 (1H, t, J=7.6Hz), 7.16 (1H, d, J=7.6Hz), 7.28 (1H, d J=7.6Hz), 7.43 (1H, t, J=6.2Hz) |
| Example 66 | 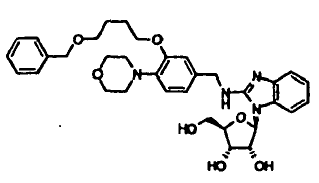 | (DMSO-d₆) 1.65-1.85 (4H, m), 2.84-3.00 (4H, m). 3.40-3.55 (2H, m). 3.60-3.80 (6H, m). 3.90-4.15 (4H, m), 4.35-4.60 (5H, m), 5.80 (2H, d, J=6.3Hz), 6.75-7.05 (5H, m), 7.10-7.50 (8H, m) |
| Example 67 | 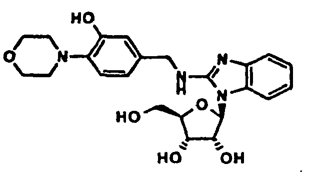 | (DMSO-d₆) 2.80-2.95 (4H, m), 3.60-3.80 (6H, m), 3.93-4.03 (1H, m), 4.05-4.13 (1H, m), 4.30-4.55 (3H, m). 5.79 (1H, d, J=7.5Hz). 6.70-7.00 (5H, m), 7.16 (1H, d. J=7.8Hz), 7.28 (1H, d. J=7.8Hz), 7.36 (1H, t, J=6.1Hz). 8.94 (1H. s) |
| Example 68 | 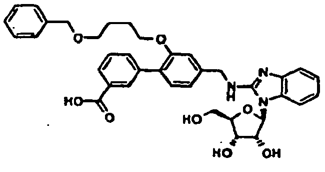 | (DMSO-d₆) 1.50-1.80 (4H, m), 3.65-3.78 (2H, m), 3.90-4.05 (3H, m), 4.10-4.18 (1H, m), 4.37 (2H, s), 4.40-4.50 (1H, m), 4.40-4.50 (1H, m), 4.61 (2H, d, J=6.1 Hz), 5.84 (1H, d, J=7.5Hz), 6.80-7.40 (12H, m), 7.43-7.55 (2H, m), 7.63-7.74 (1H, m), 7.80-7.90 (1H, m), 8.09 (1H, s) |
| Example 69 | 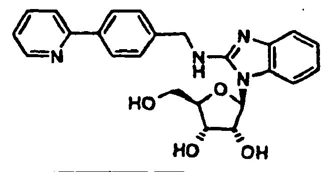 | (CD₃OD) 3.75-3.90 (2H, m), 4.05-4.30 (2H, m), 4.55-4.80 (3H, m), 5.97 (1H, d, J=7.3Hz), 6.90-7.10 (2H, m), 7.20-7.40 (3H, m), 7.45-7.60 (2H, m), 7.75-8.00 (4H, m), 8.50-8.65 (1H, m) |

### Example 70

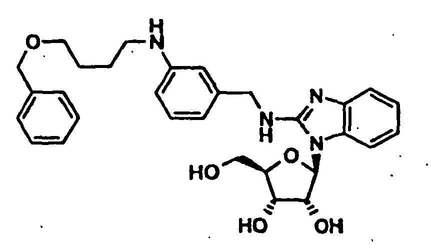

### 2-[3-(4-Benzyloxybutylamino)benzylamino]-1-(β-D-ribofuranosyl)-1H-benzimidazole

2-Chloro-1-(β-D-ribofuranosyl)-1*H-*benzimidazole (0.14g) and 3-(4-benzyloxybutylamino)benzylamine (0.36g) were suspended in isobutanol (5mL). To the mixture was added triethylamine (0.56mL), and the mixture was refluxed for 16 hours. The reaction mixture was concentrated under reduced pressure, and the obtained residue was purified by preparative reverse phase column chromatography (Shiseido CAPSELL PAC C18UG80, 5µm, 20×50mm, flow rate 30mL/minutes linear gradient, water/methanol =70/30-10/90) to give the title compound (0.08g).
¹H-NMR (DMSO-d₆) δ ppm:
1.50-1.70 (4H, m), 2.90-3.05 (2H, m), 3.43 (2H, t, J=6.1Hz), 3.60-3.75 (2H, m), 3.95-4.02 (1H, m), 4.05-4.15 (1H, m), 4.35-4.55 (5H, m), 5.21 (1H, d, J=4.1Hz), 5.27 (1H, d, J=7. 5Hz), 5.40-5.53 (1H, m), 5.57 (1H, t, J=4.4Hz), 5.80 (1H, d, J=7.5Hz), 6.38 (1H, d, J=7.7Hz), 6.45-6.60 (2H, m), 6.80-7.02 (3H, m), 7.15 (1H, d, J=7.5Hz), 7.20-7.40 (7H, m)

### Examples 71-74

The compounds of Table16 were prepared in a similar manner to that described in Example 70 using the corresponding materials.

**[Table 16]**

| Example No. | Structure | ¹H-NMR δ ppm |
|---|---|---|
| Example 71 | 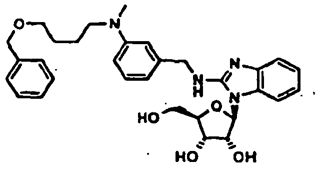 | (DMSO-d₆) 1.40-1.60 (4H, m), 2.83 (3H, s), 3.60-3.80 (2H, m), 3.90-4.00 (1H, m), 4.05-4.20 (1H, m), 4.35-4.60 (5H, m), 5.80 (1H, d, J=7.6Hz), 6.45-6.75 (3H, m), 6.80-7.50 (11H, m) |
| Example 72 | 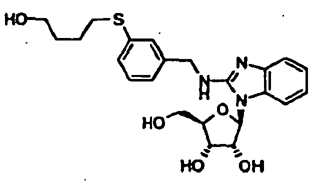 | (DMSO-d₆) 1.40-1.65 (4H, m), 2.93 (2H, t, J=7.2Hz), 3.30-3.43 (2H, m), 3.60-3.77 (2H, m), 3.95-4.03 (1H, m), 4.08-4.15 (1H, m), 4.35-4.47 (2H, m), 4.54 (2H, d, J=6.2Hz), 5.19 (1H, d, J=4.4Hz), 5.28 (1H, d, J=7.5Hz), 5.60 (1H, t, J=4.5Hz), 5.81 (1H, d, J=7.4Hz), 6.88 (1H, d, J=7.6Hz), 6.95 (1H, d, J=7.6Hz), 7.10-7.35 (6H, m), 7.47 (1H, t, J=6.2Hz) |
| Example 73 | 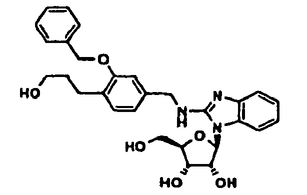 | (DMSO-d₆) 1.58-1.73 (2H, m), 2.48-2.63 (2H, m), 3.25-3.45 (2H, m), 3.60-3.75 (2H, m), 3.95-4.15 (2H, m), 4.35-4.60 (4H, m), 5.07 (2H, s), 5.15-5.40 (2H, m), 5.55-5.70 (1H, m), 5.83 (1H, d, J=7.0Hz), 6.80-7.50 (10H, m) |
| Example 74 | 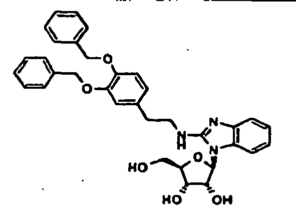 | (DMSO-d₆) 2.82 (2H, t J=7.7Hz), 3.40-3.75 (4H, m), 3.90-4.00 (1H, m), 4.03-4.14 (1H, m), 4.30-4.45 (1H, m), 5.08 (2H, s), 5.09 (2H, s), 5.17 (1H, d, J=4.7Hz), 5.22 (1H, d, J=7.4Hz), 5.57 (1H, t, J=4.4Hz), 5.76 (1H, d. J=7.8Hz), 6.70-7.05 (6H, m). 7.15-7.50 (12H, m) |

### Example 75

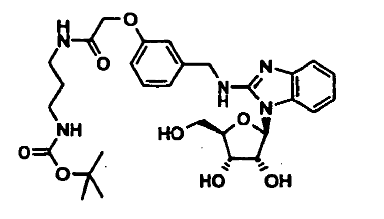

### 2-{3-[(3-t-Butoxycarbonylaminopropylcarbamoyl)methoxy]-benzylamino}-1-(β-D-ribofuranosyl)-1H-benzimidazole

*t-*Butyl *N-*(3-aminopropyl)carbamate (0.37g) and pyridine (0.51mL) were dissolved in dichloromethane (5mL). To the stirred mixture was added dropwise bromoacetylchloride (0.19mL) under ice-cooling, and the mixture was stirred at room temperature for 3 hours. To the reaction mixture was added 1mol/L hydrochloric acid, and the resulting mixture was extracted with ethyl acetate. The organic layer was washed with brine and dried over anhydrous magnesium sulfate. The filtrate was concentrated under reduced pressure, and the obtained residue was dissolved in *N*,*N-*dimethylformamide (2mL). To the mixture were added 2-(3-hydroxybenzylamino)-1-(β-D-ribofuranosyl)-1*H-*benzimidazole (0.09g) and potassium carbonate (0.14g), and the mixture was stirred at 50°C for 16 hours. After the insoluble material was removed by filtration, the reaction mixture was concentrated under reduced pressure. The obtained residue was purified by preparative reverse phase column chromatography (Shiseido CAPSELL PAC C18UG80, 5µ, 20×50mm, flow rate 30mL/minutes linear gradient, water/methanol =70/30-10/90) to give the title compound (0.02g).
¹H-NMR (DMSO-d₆) δ ppm:
1.37 (9H, s), 1.45-1.60 (2H, m), 2.80-2.95 (2H, m), 3.03-3.15 (2H, m), 3.60-3.86 (2H, m), 3.95-4.03 (1H, m), 4.08-4.15 (1H, m), 4.38-4.47 (3H, m), 4.50-4.60(2H, m), 5.22 (1H, d, J=4.6Hz), 5.31 (1H, d, J=7.3Hz), 5.60 (1H, t, J=4.5Hz), 5.81 (1H, d, J=7.6Hz), 6.70-7.00 (6H, m), 7.16 (1H, d, J=7.4Hz), 7.23 (1H, dd, J=7.4Hz, 8.1Hz), 7.29 (1H, d, J=8.1Hz), 7.40-7.50 (1H, m), 8.00-8.13 (1H, m)

### Example 76

The compound of Table 17 was prepared in a similar manner to that described in Example 75 using the corresponding materials.

**[Table 17]**

| Example No. | Structure | ¹H-NMR δ ppm |
|---|---|---|
| Example 76 | 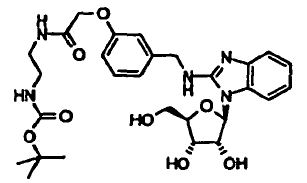 | (DMSO-d₆) 1.36 (9H, s), 2.90-3.20 (4H, m), 3.60-3.80 (2H. m), 3.95-4.15 (2H, m), 4.35-4.45 (3H, m), 4.47-4.62 (2H, m), 5.83 (1H, d, J=7.6Hz), 6.70-7.05 (6H, m), 7.10-7.60 (4H, m), 8.00-8.13 (1H, m) |

### Example 77

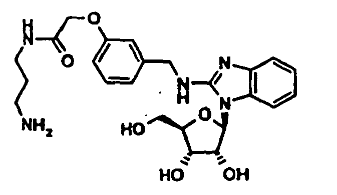

### 2-{3-[(3-Aminopropylcarbamoyl)methoxy]benzylamino}-1-(β-D-ribofuranosyl)-1H-benzimidazole

2-{3-[(3-*t-*Butoxycarbonylaminopropylcarbamoyl)-methoxy]benzylamino}-1-(β-D-ribofuranosyl)-1*H-*benzimidazole (15mg) was dissolved in 22% hydrochlorideethanol solution, and the mixture was stirred at room temperature for 30minutes. The reaction mixture was concentrated under reduced pressure, and the obtained residue was purified by preparative reverse phase column chromatography (Shiseido CAPSELL PAC C18UG80, 5µm, 20×50mm, flow rate 30mL/minutes linear gradient, water/methanol =90/10-10/90) to give the title compound (9 mg).
¹H-NMR (DMSO-d₆) δ ppm:
1.40-1.55 (2H, m), 3.08-3.23 (2H, m), 3.60-3.75 (2H, m), 3.95-4.15 (2H, m), 4.35-4.45 (3H, m), 4.46-4.63 (2H, m), 5.81 (1H, d, J=7.6Hz), 6.70-7.05(5H, m), 7.10-7.35(3H, m), 7.40-7.55 (1H, m), 8.05-8.20 (1H, m)

### Examples 78-79

The compounds of Table18 were prepared in a similar manner to that described in Example 77 using the corresponding materials.

**[Table 18]**

| Example No. | Structure | ¹H-NMR δ ppm |
|---|---|---|
| Example 78 | 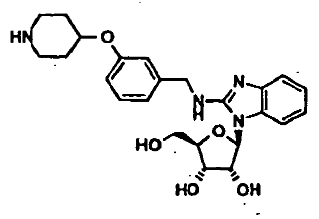 | (DMSO-d₆) 1.30-1.46 (2H, m), 1.80-1.95 (2H, m), 2.83-2.96 (2H, m), 3.60-3.75 (2H, m), 3.95-4.02 (1H, m), 4.06-4.15 (tH, m), 4.25-4.46 (3H, m), 4.52 (2H, d, J=6.1Hz), 5.20 (1H, d, J=4.3Hz), 5.27 (1H, d, J=7.5Hz), 5.60 (1H, t, J=4.4Hz), 5.81 (1H, d, J=7.7Hz), 6.76 (1H, dd, J=2.0Hz, 8.0Hz), 6.80-7.00 (4H, m), 7.10-7.23 (2H, m), 7.27 (1H, d J=7.8Hz), 7.43 (1H, t, J=6.1Hz) |
| Example 79 | 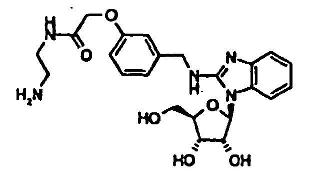 | (DMSO-d₆) 2.53-2.65 (2H, m), 3.08-3.18 (2H, m), 3.60-3.75 (2H, m), 3.95-4.15 (2H, m), 4.35-4.45 (3H, m), 4.50-4.60 (2H, m), 5.81 (1H, d, J=7.1Hz), 6.75-7.05 (5H, m), 7.10-7.35 (3H, m), 7.40-7.50 (1H, m), 7.95-8.05 (1H, m) |

### Example 80

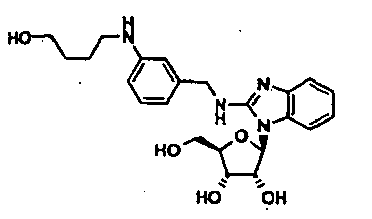

### 2-[3-(4-Hydroxybutylamino)benzylamino]-1-(β-D-ribofuranosyl)-1H-benzimidazole

2-[3-(4-Benzyloxybutylamino)benzylamino]-1-(β-D-ribofuranosyl)-1*H-*benzimidazole (44mg) was dissolved in ethanol (5mL). To the solution was added a catalytic amount of 10% palladium-carbon powder, and the mixture was stirred at 60°C under a hydrogen atmosphere for 24 hour. The insoluble material was removed by filtration, and the solvent of the filtrate was removed under reduced pressure to give the title compound (22mg).
¹H-NMR (DMSO-d₆) δ ppm:
1.40-1.60 (4H, m), 2.90-3.02 (2H, m), 3.60-3.75 (2H, m), 3.94-9.02 (1H, m), 4.08-4.15 (1H, m), 9.30-4.55 (4H, m), 5.21 (1H, d, J=4.3Hz), 5.28 (1H, d, J=7.8Hz), 5.43-5.52 (1H, m), 5.57 (1H, t, J=4.5Hz), 5.80 (1H, d, J=7.6Hz), 6.39 (1H, d, J=7.8Hz),6.51 (1H, d, J=7.1Hz), 6.55 (1H, s), 6.80-7.05 (3H, m), 7.15 (1H, d, J=7.7Hz), 7.27 (1H, d J=7.7Hz), 7.35 (1H, t, J=6.1Hz)

### Examples 81-83

The compounds of Table 19 were prepared in a similar manner to that described in Example 80 using the corresponding materials.

**[Table 19]**

| Example | No. Structure | ¹H-NMR δ ppm |
|---|---|---|
| Example 81 | 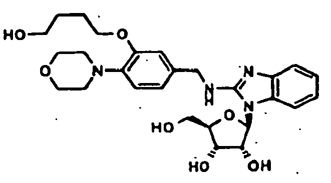 | (DMSO-d₆) 1.50-1.62 (2H, m), 1.67-1.82 (2H, m), 2.85-3.00 (4H, m), 3.40-3.50 (2H, m), 3.60-3.80 (6H, m), 3.90-4.15 (5H, m), 4.30-4.55 (4H, m), 5.20 (1H, d, J=4.4Hz), 5.30 (1H, d, J=7.4Hz), 5.50-5.70 (1H, m), 5.80 (1H, d. J=7.7Hz), 6.75-7.05 (5H, m). 7.10-7.50 (3H, m) |
| Example 82 | 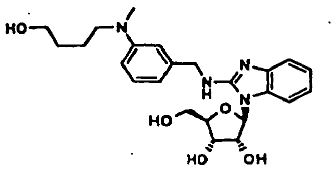 | (DMSO-d₆) 1.30-1.55-(4H, m), 2.84 (3H, s), 3.20-3.45 (4H, m), 3.60-3.75 (2H, m), 3.95-4.02 (1H, m), 4.06-4.15 (1H, m), 4.35-4.46 (1H, m), 4.50 (2H, d, J=6.1 Hz), 5.82 (1H d, J=8.1 Hz), 6.53 (1H, d, J=8.2Hz), 6.60 (1H, d, J=7.4Hz), 6.71 (1H, s). 6.80-7.50 (6H, m) |
| Example 83 | 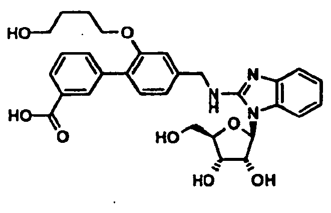 | (CD₃OD) 1.50-1.63 (2H, m), 1.68-1.80 (2H, m), 3.49 (2H, t, J=6.5Hz), 3.80-3.90 (2H, m), 4.01 (2H, t, J=6.1 Hz), 4.10-4.20 (1H, m), 4.25-4.35 (1H, m), 4.57-4.78 (3H, m), 6.00 (1H, d, J=7.3Hz), 7.00-7.17 (4H, m), 7.24-7.35 (3H, m), 7.43 (1H, t, J=7.7Hz), 7.67 (1H, d, J=7.6Hz), 7.92 (1H, d, J=7.6Hz), 8.16 (1H, s) |

### Example 84

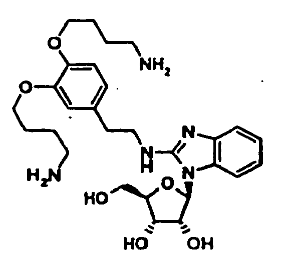

### 2-{2-[3,4-Bis(4-aminobutoxy)phenyl]ethylamino}-1-(β-D-ribofuranosyl)-1H-benzimidazole

2-{2-[3,4-Bis(benzyloxy)phenyl]ethylamino}-1-(β-D-ribofuranosyl)-1*H-*benzimidazole (0.35g) was dissolved in ethanol (5mL). To the solution was added a catalytic amount of 10% palladium-carbon powder, and the mixture was stirred at 60°C under a hydrogen atmosphere for 24 hour. The insoluble material was removed by filtration, and the obtained residue and potassium carbonate (0.30g) were suspended in *N*,*N-*dimethylformamide(5mL). To the reaction mixture was added *N-*(4-bromobutyl)phthalimide (0.60g), and the mixture was stirred at 60°C for 16 hours. The insoluble material was removed by filtration, and the reaction mixture was concentrated under reduced pressure. The obtained residue was dissolved in ethanol (5mL). To the reaction mixture was added hydrazine monohydrate (0.5mL), and the mixture was stirred at 90°C for 6hours. The reaction mixture was concentrated under reduced pressure, and the obtained residue was purified by preparative reverse phase column chromatography (Shiseido CAPSELL PAC C18UG80, 5µm, 20×50mm, flow rate 30mL/minutes linear gradient, water/methanol =70/30-10/90) to give the title compound (0.02g).
¹H-NMR (CD₃OD) δ ppm:
1.55-1.90 (8H, m), 2.65-2.83 (4H, m), 2.89 (2H, t, J=7.2Hz), 3.62 (2H, t, J=7.2Hz), 3.70-3.83 (2H, m), 3.90-4.10 (5H, m), 4.19 (1H, dd, J=2.5Hz, 6.1Hz), 4.44 (1H, dd, J=6.1Hz, 7.4Hz), 5.87 (1H, d, J=7.4Hz), 6.78 (1H, dd, J=1.7Hz, 7.9Hz), 6.85 (1H, d, J=7.9Hz), 6.89 (1H, d, J=1.7Hz), 6.98 (1H, t, J=7.6Hz), 7.04 (1H, t, J=7.6Hz), 7.23 (1H, d, J=7.6Hz), 7.28 (1H, d, J=7.6Hz)

### Example 85

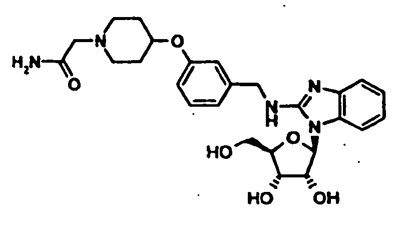

### 2-[3-(N-Carbamoylmethylpiperidin-4-yloxy)benzylamino]-1-(β-D-ribofuranosyl)-1H-benzimidazole

2-[-3-(Piperidin-4-yloxy)benzylamine]-1-(β-D-ribofuranosyl)-1*H-*benzimidazole (50mg), bromoacetamide (20mg) and potassium carbonate (23mg) were suspended in *N*,*N-*dimethylformamide (5mL), and the mixture was stirred at 60°C for 16 hours. The insoluble material was removed by filtration, and the reaction mixture was concentrated under reduced pressure. The obtained residue was purified by preparative reverse phase column chromatography (Shiseido CAPSELL PACC18UG80, 5µm, 20×50mm, flow rate 30mL/minutes linear gradient, water/methanol =90/10-10/90) to give the title compound (30mg).
¹H-NMR (DMSO-d₆) δ ppm:
1.55-1.73 (2H, m), 1.85-1.96 (2H, m), 2.20-2.35 (2H, m), 2.60-2.75 (2H, m), 2.84 (2H, s), 3.60-3.75 (2H, m), 3.95-4.02 (1H, m), 4.07-4.15 (1H, m), 4.25-4.45 (2H, m), 4.52 (2H, d, J=6.0Hz), 5.20 (1H, d, J=4.5Hz), 5.27 (1H, d, J=7.7Hz), 5.59 (1H, t, J=4.2Hz), 5.81 (1H, d, J=7.5Hz), 6.77 (1H, dd, J=1.8Hz, 8.4Hz), 6.83-7.00 (4H, m), 7.04-7.23 (4H, m), 7.27 (1H, d J=7.8Hz), 7.43 (1H, t, J=6.0Hz)

### Example 86

The compound of Table 20 was prepared in a similar manner to that described in Example 85 using the corresponding material.

**[Table 20]**

| Example No. | Structure | ¹H-NMR δ ppm |
|---|---|---|
| Example 86 | 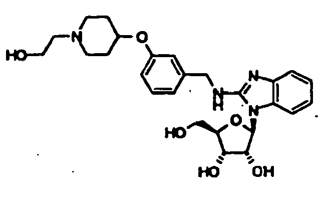 | (DMSO-d₆) 1.45-1.65 (2H, m), 1.80-1.95 (2H, m), 2.10-2.25 (2H, m). 2.37 (2H, t. J=6.2Hz), 2.60-2.75 (2H, m). 3.40-3.53 (2H, m), 3.60-3.80 (2H, m), 3.95-4.03 (1H, m), 4.05-4.14 (1H, m), 4.23-4.45 (3H, m). 4.52 (2H, d, J=6.1Hz), 5.20 (1H, d, J=4.5Hz), 5.27 (1H, d, J=7.5Hz), 5.59 (1H, t, J=4.4Hz), 5.81 (1H, d. J=7.5Hz), 6.76 (1H, dd. J=2.1 Hz, 8.1 Hz), 6.83-7.00 (4H, m), 7.10-7.23 (2H, m), 7.28 (1H, d J=7.6Hz), 7.43 (1H, t, J=6.1Hz) |

### Example 87

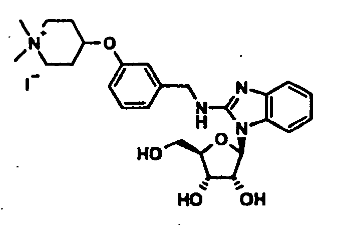

### 2-[3-(N,N-Dimethylpiperidinium-4-yloxy)benzylamino]-1-(β-D-ribofuranosyl)-1H-benzimidazole iodide

2-[3-(Piperidin-4-yloxy)benzylamino]-1-(β-D-ribofuranosyl)-1*H-*benzimidazole (0.1g) was dissolved in ethanol (5mL). To the reaction mixture was added iodomethane (78mg), and the mixture was stirred at 60°C for 16 hours. The reaction mixture was concentrated under reduced pressure, and the obtained residue was purified by preparative reverse phase column chromatography (Shiseido CAPSELL PAC C18UG80, 5µm, 20×50mm, flow rate 30mL/minutes linear gradient, water/methanol=90/10-10/90) to give the title compound (30mg).
¹H-NMR (CD₃OD) δ ppm:
2.05-2.00 (2H, m), 2.25-2.40 (2H, m), 3.23 (3H, s), 3.30 (3H, s), 3.40-3.50 (2H, m), 3.55-3.70 (2H, m), 3.78-3.93 (2H, m), 9.20-4.32 (2H, m), 4.55-4.63 (1H, m), 9.67-4.78 (3H, m), 6.10 (1H, d, J=7.7Hz), 6.90-7.15(3H, m), 7.25-7.45(4H, m), 7.50-7.60 (1H, m)

### Example 88

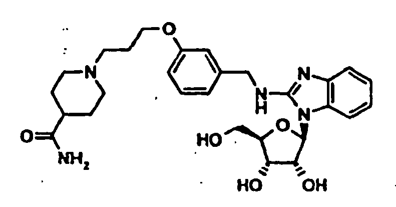

### 2-{3-[3-(4-Carbamoylpiperidin-1-yl)propoxy]benzylamino}-1-(β-D-ribofuranosyl)-1H-benzimidazole

2-[3-(3-Chloropropoxy)benzylamino]-1-(2,3,5-tri-*O-*acetyl-β-D-ribofuranosyl)-1*H-*benzimidazole (0.67g) and sodium iodide (0.52g) were suspended in acetone (15mL), and the mixture was refluxed for 16 hours. The insoluble material was removed by filtration, and the filtrate was concentrated under reduced pressure. The obtained residue, isonipecotamide (0.30g) and potassium carbonate (0.32g) were suspended in acetonitrile (5 mL), and the mixture was stirred at 70°C for 16 hours. The insoluble material was removed by filtration, and the filtrate was concentrated under reduced pressure. The obtained residue was dissolved in methanol (2mL). To the redaction mixture was added 5mol/L aqueous sodium hydroxide solution (0.5mL), and the mixture was stirred at room temperature for 1 hour. To the reaction mixture was added acetic acid (1mL), and the mixture was concentrated under reduced pressure. The obtained residue was purified by preparative reverse phase column chromatography (Shiseido CAPSELL PAC C18UG80, 5µm, 20×50mm, flow rate 30mL/minutes linear gradient, water/methanol =70/30-10/90) to give the title compound (0.38g).
¹H-NMR (CD₃OD) δppm:
1.60-2.30 (9H, m), 2.40-2.65 (2H, m), 2.85-3.05 (2H, m), 3.75-3.90 (2H, m), 3.99 (2H, t, J=6.2Hz), 4.10-4.15 (1H, m), 4.20-4.30 (1H, m), 4.50-4.70 (3H, m), 5.96 (1H, d, J=7.9Hz), 6.70-6.80 (1H, m), 6.85-7.35 (7H, m)

### Examples 89-102

The compounds of Table 21 were prepared in a similar manner to that described in Example 88 using the corresponding materials.

**[Table 21]**

| ExampleNo. | Structure | ¹H-NMR δ ppm |
|---|---|---|
| Example 89 | 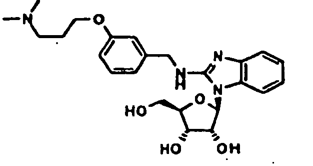 | (CD₃OD) 1.80-2.00 (2H, m), 2.22 (6H, s). 2.40-2.55 (2H, m), 3.75-3.90 (2H, m), 3.98 (2H, t, J=6.2Hz), 4.10-4.15 (1H, m), 4.20-4.30 (1H, m), 4.50-4.70 (3H, m), 5.96 (1H, d, J=7.7Hz), 6.70-7.40 (8H, m) |
| Example 90 | 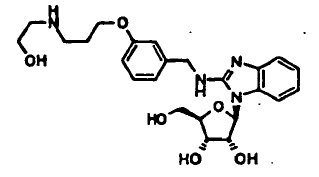 | (CO₃OD) 1.85-2.00 (2H, m), 2.71 (2H, t, J=5.5Hz), 2.78 (2H, t, J=7.1Hz), 3.64 (2H, t, J=5.5Hz), 3.75-3.85 (2H, m), 4.03 (2H, t, J=6.3Hz), 4.05-4.15 (1H, m), 4.20-4.30 (1H, m), 4.50-4.70 (3H, m), 5.95 (1H, d, J=7.1Hz), 6.70-6.85 (1H, m), 6.90-7.35 (7H, m) |
| Example 91 | 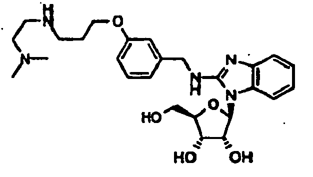 | (CD₃OD) 1.85-2.00 (2H, m), 2.2.1 (6H, s), 2.35-2.50 (2H, m), 2.60-2.80 (4H, m), 3.75-3.85 (2H, m), 4.01 (2H, t, J=6.0Hz), 4.10-4.15 (1H, m), 4.20-4.30 (1H, m), 4.50-4.70 (3H, m), 5.95 (1H, d, J=7.4Hz), 6.70-6.80 (1H, m), 6.90-7.35 (7H, m) |
| Example 92 | 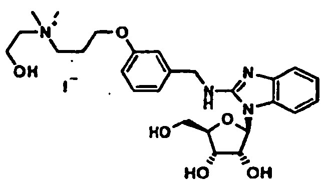 | (CD₃OD) 2.10-2,30 (2H, m), 3.10 (6H, s), 3.35-3.45 (2H, m), 3.50-3.60 (2H, m), 3.75-4.35 (8H, m), 4.55-4.70 (3H, m), 5.96 (1H, d, J=7.3Hz), 6.75-6.85 (1H, m), 6.90-7.10 (4H, m), 7.15-7.35 (3H, m) |
| Example 93 | 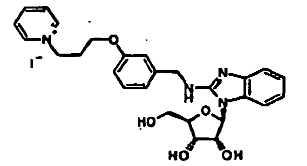 | (CD₃OD) 2.40-2.55 (2H, m), 3.75-3.90 (2H, m), 4.00-4.30 (4H, m), 4.50-4.65 (3H, m), 4.70-4.85 (2H, m), 5.97 (1H, d, J=7.3Hz), 6.55-6.75 (2H, m), 6.80-7.45 (6H, m), 7.75-7.95 (2H, m), 8.10-8.25 (1H, m), 8.85-8.95 (2H, m) |
| Example 94 | 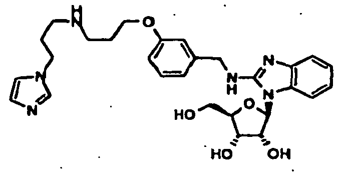 | (CD₃OD) 1.80-2.00 (4H, m), 2.51 (2H, t, J=7.3Hz), 2.70 (2H, t, J=7.1Hz), 3.75-3.85 (2H, m), 3.95-4.05 (4H, m), 4.10-4.15 (1H, m), 4.20-4.30 (1H, m), 4.50-4.70 (3H, m), 5.95 (1H, d, J=7.2Hz), 6.70-6.80 (1H, m), 6.85-7.35 (9H, m), 7.60 (1H, s) |
| Example 95 | 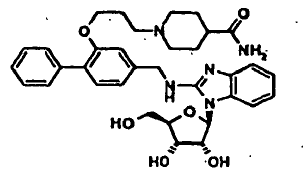 | (CD₃OD) 1.40-2.50 (11H, m), 2.75-2.95 (2H, m), 3.75-4.35 (6H, m), 4.55-4.75 (3H, m), 5.98 (1H, d, J=7.3Hz), 6.90-7.55 (12H, m) |
| Example 96 | 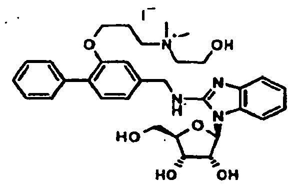 | (CD₃OD) 2.00-2.15 (2H, m), 2.95 (6H, s), 3.20-3.30 (2H, m), 3.75-3.90 (4H, m), 4.00-4.35 (4H, m), 4.55-4.75 (3H, m), 5.98 (1H, d, J=7.6Hz), 6.95-7.55 (12H, m) |
| Example 97 | 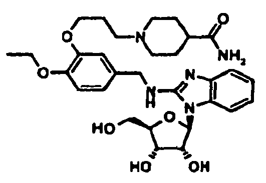 | (CD₃OD) 1.35 (3H, t, J=7.0Hz), 1.60-2.30 (9H, m), 2.45-2.55 (2H, m), 2.85-3.00 (2H, m), 3.75-3.90 (2H, m), 3.95-4.15 (5H, m), 4.20-4.30 (1H, m), 4.45-4.65 (3H, m), 5.94 (1H, d, J=7.6Hz), 6.80-7.15 (5H, m), 7.20-7.35 (2H, m) |
| Example 98 | 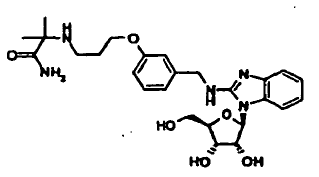 | (CD₃OD) 1.26 (6H. s). 1.85-1.95 (2H, m), 2.60-2.65 (2H, m), 3.75-3.85 (2H, m). 4.00-4.15 (3H, m), 4.20-4.30 (1H, m), 4.50-4.65 (3H, m), 5.95 (1H, d, J=7.6Hz). 6.75-6.80 (1H, m), 6.90-7.05 (4H, m), 7.15-7.30 (3H, m) |
| Example 99 | 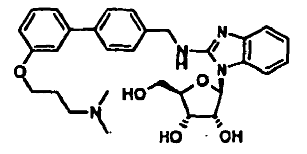 | (CD₃OD) 1.90-2.05 (2H, m), 2.27 (6H, s), 2.45-2.60 (2H, m), 3.75-3.90 (2H, m), 4.00-4.30 (4H, m), 4.55-4.80 (3H, m), 5.96 (1H, d, J=7.8Hz), 6.80-7.60 (12H, m) |
| Example 100 | 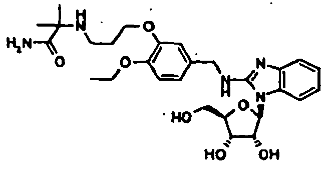 | (CD₃OD) 1.26 (6H, s), 1.35 (3H, t, J=7.1Hz), 1.85-1.95 (2H, m), 2.60-2.70 (2H, m), 3.75-3.85 (2H, m), 3.95-4.15 (5H, m), 4.20-4.30 (1H, m). 4.45-4.60 (3H, m), 5.94 (1H, d, J=7.3Hz), 6.80-7.05 (5H, m). 7.20-7.30 (2H, m) |
| Example 101 | 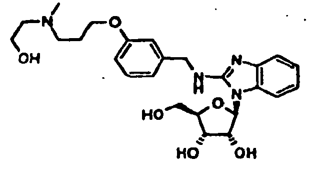 | (CD₃OD) 1.85-2.00 (2H, m), 2.29 (3H, s), 2.56 (2H, t, J=6.0Hz), 2.61 (2H, t, J=7.6Hz). 3.66 (2H, t, J=6.0Hz), 3.75-3.90 (2H, m). 4.00 (2H, t, J=6.3Hz), 4.10-4.15 (1H, m), 4.20-4.30 (1H, m), 4.50-4.70 (3H, m), 5.95 (1H, d, J=7.4Hz), 6.70-6.80 (1H, m). 6.90-7.35 (7H, m) |
| Example 102 | 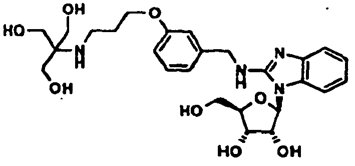 | (CD₃OD) 1.85-2.00 (2H, m), 2.83 (2H, t, J=7.0Hz), 3.55 (6H, s), 3.75-3.90 (2H, m), 4.05 (2H, t, J=6.2Hz), 4.10-4.15 (1H, m), 4.20-4.30 (1H, m). 4.50-4.70 (3H, m). 5.95 (1H, d, J=7.4Hz), 6.75-6.85 (1H, m), 6.90-7.35 (7H, m) |

### Example 103

The compounds of Table 22 can be prepared in a similar manner to that described in Example 88 using the corresponding materials.

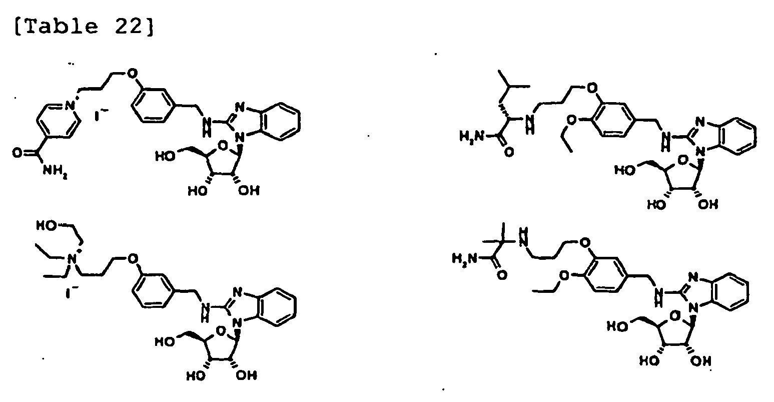

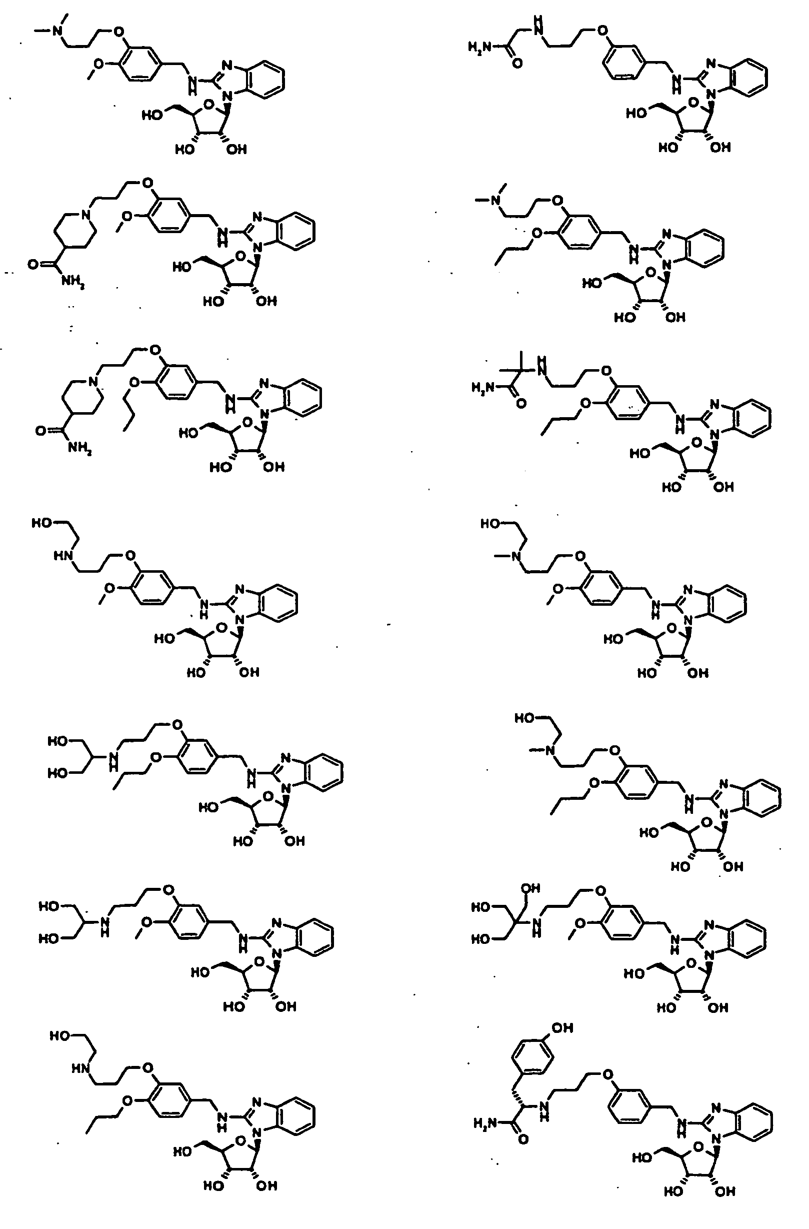

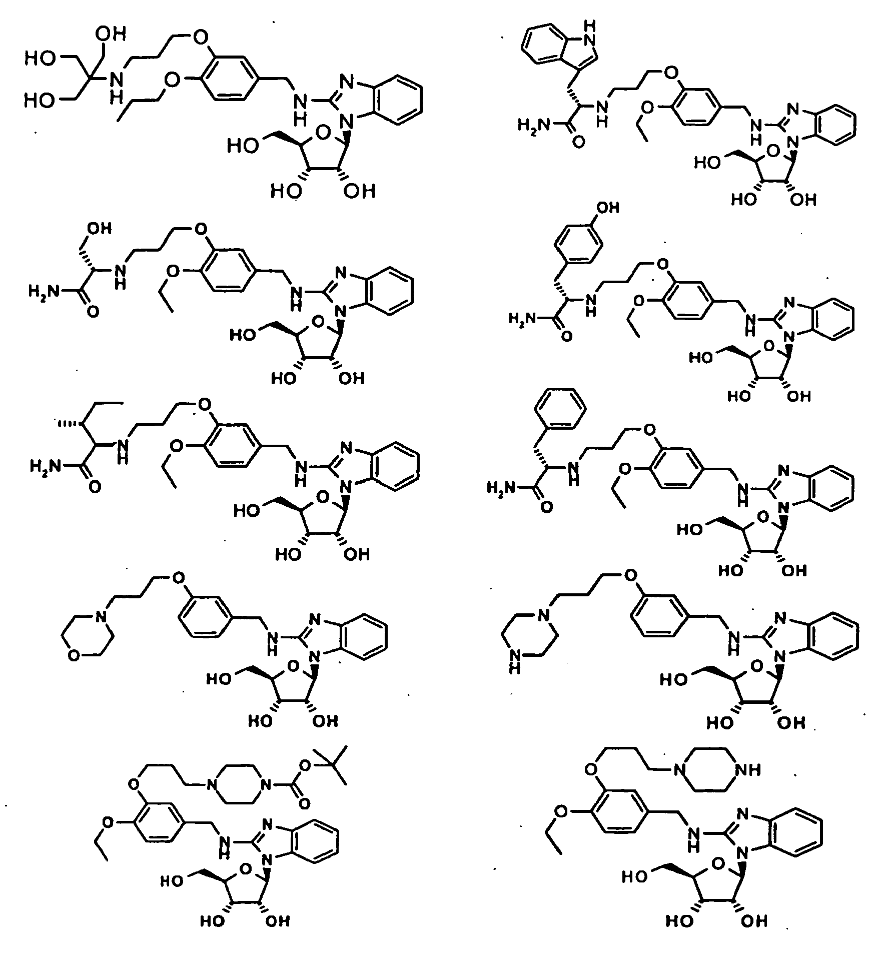

### Example 104

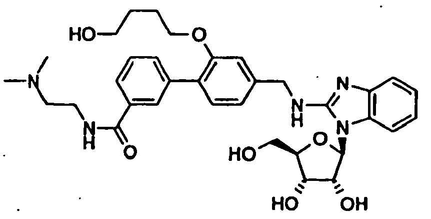

### 2-{3-(4-Hydroxybutoxy)-4-[3-(2-dimethylaminoethylcarbamoyl)phenyl]benzylamino}-1-(β-D-ribofuranosyl)-1H-benzimidazole

2-[3-(9-Hydroxybutoxy)-4-(3-carboxyphenyl)benzylamino]-1-(β-D-ribofuranosyl)-*1H*-benzimidazole (20mg), *N,N*-dimethylethylenediamine (4mg) and 1-hydroxybenzotriazole (7mg) were suspended in *N,N*-dimethylformamide (1 mL) at room temperature. To the mixture was added 1-ethyl-3-(3-dimethylaminopropyl)carbodiimide hydrochloride (10mg), and the mixture was stirred at room temperature for 13 hours. The reaction mixture was concentrated under reduced pressure, and the obtained residue was purified by preparative reverse phase column chromatography (Shiseido CAPSELL PAC C18UG80, 5µm, 20×50mm, flow rate 30mL/minutes linear gradient, water/methanol =70/30-10/90) to give the title compound (16mg).
¹H-NMR (CD₃OD) δ ppm:
1.50-1.62 (2H, m), 1.65-1.78 (2H, m), 2 . 31 (6H, s), 2.58 (2H, t, J=6.8Hz), 3.47 (2H, t, J=6.6Hz), 3.52 (2H, t, J=6.8Hz), 3.78-3.90 (2H, m), 3.99 (2H, t, J=6.3Hz), 4.10-4.19 (1H, m), 4.28 (1H, dd, J=2.3Hz, 5.8Hz), 4.58-4.75 (3H, m), 5.97 (1H, d, J=7.4Hz), 6.95-7.10 (3H, m), 7.14 (1H, s), 7.21-7.32 (3H, m), 7.44 (1H; t, J=7.8Hz), 7.66 (1H, d, J=7.8Hz), 7.73 (1H, d, J=7.8Hz), 7.97 (1H, s)

### Example 105

The compounds of Table 23 can be prepared in a similar manner to that described in Example 104 using the corresponding materials.

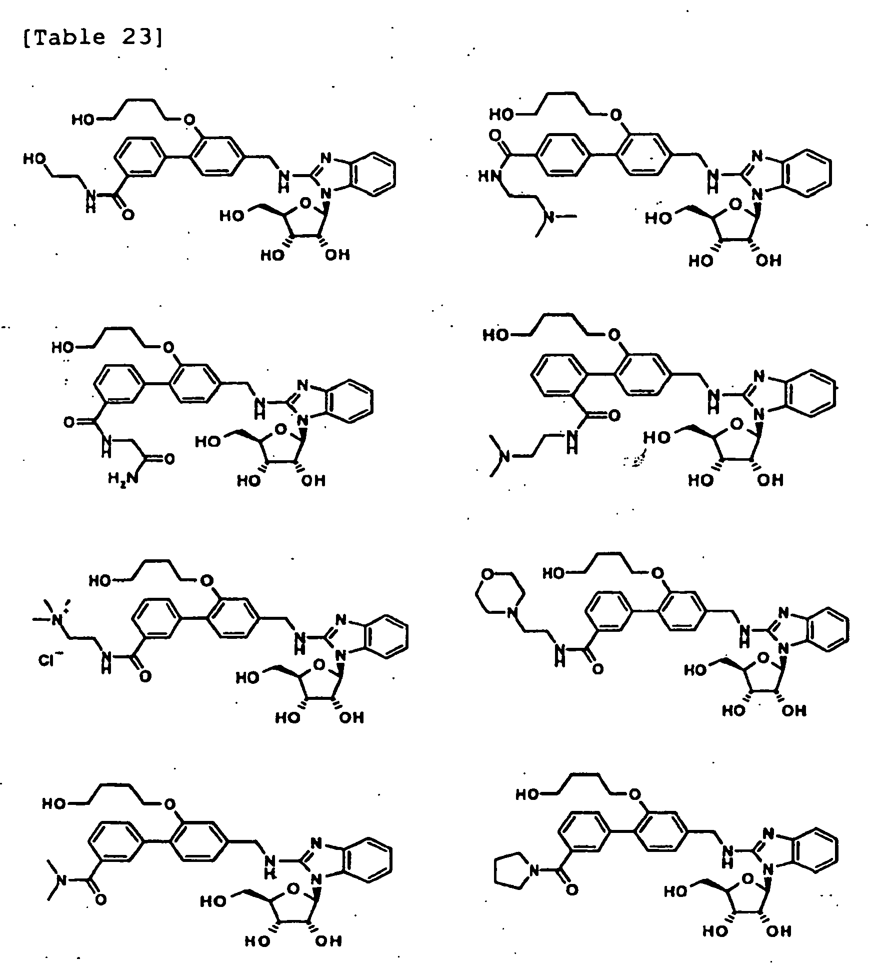

### Example 106

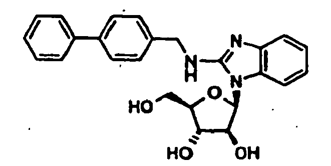

### 1-(β-D-Arabinofuranosyl)-2-(4-phenylbenzylamino)-1H-benzimidazole

2-Chloro-1-(β-D-ribofuranosyl)-*1H*-benzimidazole (0.5g) was suspended in pyridine (8.8 mL). To the stirred mixture was added dropwise 1,3-dichloro-1,1,3,3-tetraisopropyldisiloxane (0.59 mL) under ice-cooling, and the mixture was stirred at room temperature for 26 hours. To the reaction mixture was added methanol (2 mL), and the mixture was concentrated under reduced pressure. To the obtained residue was added water, and the resulting mixture was extracted with ethyl acetate. The organic layer was washed with 1mol/L hydrochloric acid, a saturated aqueous sodium hydrogen carbonate solution and brine successively and dried over anhydrous magnesium sulfate. The filtrate was concentrated under reduced pressure, and the obtained residue was purified by column chromatography on silica gel (ethyl acetate/hexane=2/9) to give 2-chloro-1-[3,5-O,O-1,1,3,3-tetra-isopropyldisiloxanyl)-β-D-ribofuranosyl]-*1H*-benzimidazole (0.35g). The obtained compound (0.34g), triethylamine (0.12 mL) and 4-dimethylaminopyridine (0.08g) were dissolved in dichloromethane (13 mL), and to the stirred mixture was added dropwise trifluoromethanesulfonylchloride (0.09 mL) under ice-cooling. The mixture was stirred at room temperature for 1hour. To the reaction mixture was added a saturated aqueous sodium hydrogen carbonate solution (10 mL), and the resulting mixture was extracted with ethyl acetate. The organic layer was washed with brine and dried over anhydrous magnesium sulfate. The filtrate was concentrated under reduced pressure, and the obtained residue was dissolved in *N,N*-dimethylformamide (3mL) . To the mixture was cesium acetate (0.17g), and the mixture was stirred at 30°C for 15 hours. To the reaction mixture was added water (10 mL), and the resulting mixture was extracted with diethyl ether. The organic layer was washed with water and brine successively and dried over anhydrous magnesium sulfate. The filtrate was concentrated under reduced pressure, and the obtained residue was dissolved in tetrahydrofuran (3.9 mL). To the stirred mixture was added dropwise 1mol/L tetrabutylammonium fluoride-tetrahydrofuran solution (1.47 mL) under ice-cooling. The mixture was stirred under ice-cooling for 1 hour. To the reaction mixture was added acetic acid (0.08 mL), and the mixture was concentrated under reduced pressure. The obtained residue was purified by column chromatography on silica gel (ethyl acetate/hexane=4/1) to give 1-(2-*O*-acetyl-β-D-arabinofuranosyl)-2-chloro-*1H*-benzimidazole (0.12g). The obtained compound (0.12g), 4-phenylbenzylamine (0.26g) and *N,N*-diisopropylethylamine (0. 37 mL) were suspended in *n*-propanol (3.6 mL), and the mixture was refluxed for 43 hours. The reaction mixture was concentrated under reduced pressure, and the obtained residue was purified by column chromatography on aminopropylated silica gel (dichloromethane/methanol=12/1) to give the title compound (0.15g).
¹H-NMR (DMSO-d₆) δ ppm:
3.63-3.85 (3H, m), 4.05-4.29 (2H, m), 4.48-4.72 (2H, m), 5.18-5.72 (3H, m), 6.16 (1H, d, J=5.4Hz), 6.78-6.97 (2H, m), 7.06-7.72 (12H, m)

### Example 107

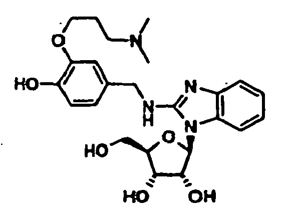

### 2-[4-Hydroxy-3-(3-dimethylaminopropoxy)benzylamino]-1-(β-D-ribofuranosyl)-1H-benzimidazole

2-[4-Benzyloxy-3-(3-chloropropoxy)benzylamino]-1-(2,3,5-tri-*O*-acetyl-β-D-ribofuranosyl)-*1H*-benzimidazole (0.05g) and sodium iodide (0.01g) were suspended in acetone (15mL), and the mixture was refluxed for 16 hours. The insoluble material was removed by filtration, and the filtrate was concentrated under reduced pressure. The obtained residue and dimethylamine (0.03g) were suspended in a mixed solvent of ethanol (1mL) and acetonitrile (1mL), and the mixture was stirred at 75°C for 24 hours. The insoluble material was removed by filtration, and the filtrate was concentrated under reduced pressure. The obtained residue was purified by preparative reverse phase column chromatography (Shiseido CAPSELL PAC C18UG80, 5µm, 20×50mm, flow rate 30mL/minutes linear gradient, water/methanol =70/30-10/90) to give 2-[4-benzyloxy-3-(3-dimethylaminopropoxy)benzylamino]-1-(β-D-ribofuranosyl)-1H-benzimidazole. The obtained compound was dissolved in methanol (2mL). To the solution was added a catalytic amount of 10% palladium-carbon powder, and the mixture was stirred at 40°C under a hydrogen atmosphere for 24 hour. The insoluble material was removed by filtration, and the filtrate was concentrated under reduced pressure to give the title compound (0.02g).
¹H-NMR (DMSO-d₆) δ ppm:
1.75-1.90 (2H, m), 2.15 (6H, s), 2.40 (2H, t, J=6.7Hz), 3.60-3.75 (2H, m), 3.85-4.15 (4H, m), 4.25-4.53 (3H, m), 5.78 (1H, d, J=7.7Hz), 6.65-7.05 (5H, m), 7.10-7.35 (3H, m)

### Example 108

The compounds of Table 24 can be prepared in a similar manner to that described in Example 107 using the corresponding materials.

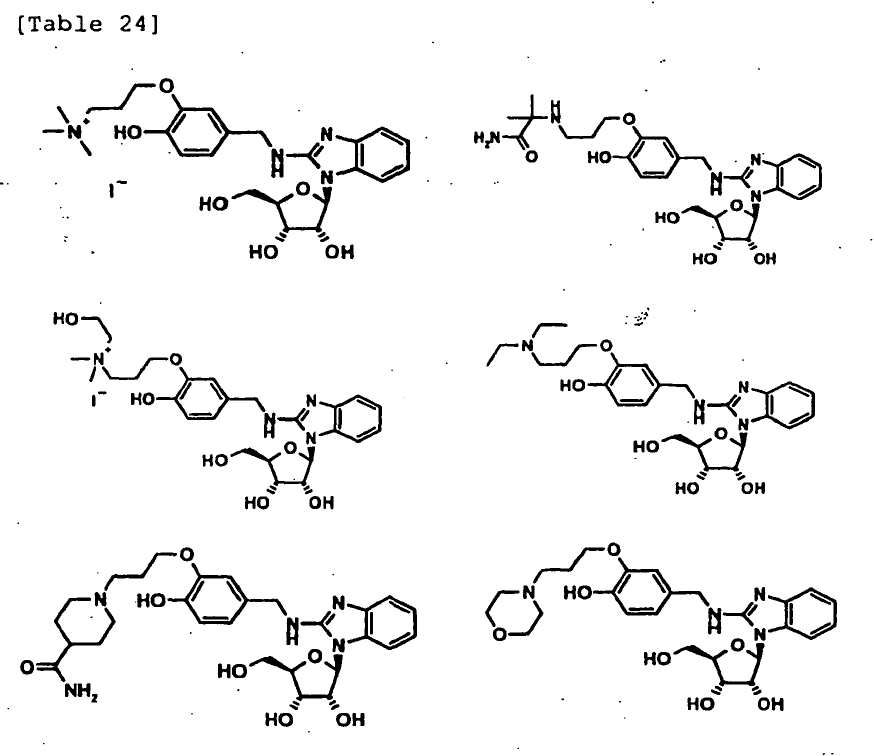

### Example 109

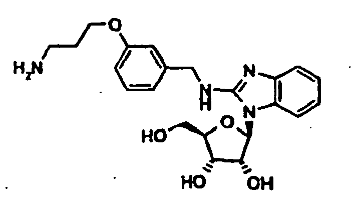

### 2-[3-(3-Aminopropoxy)benzylamino]-1-(β-D-ribofuranosyl)-1H-benzimidazole

2-(3-Hydroxybenzylamino)-1-(β-D-ribofuranosyl)-*1H-*benzimidazole (0.77g) and potassium carbonate (0.43g) were suspended in *N,N*-dimethylformamide (15mL). To the mixture was added *N*-(3-bromopropy)phthalimide (0.84g), and the mixture was stirred at 60°C for 16 hours. The insoluble material was removed by filtration, and the reaction mixture was concentrated under reduced pressure. The obtained residue was purified by column chromatography on silica gel (ethyl acetate/ethanol =10/1) to give 2-[3-(3-phthalimidepropoxy)benzylamino]-1-(β-D-ribofuranosyl)-*1H*-benzimidazole (0.75g). The obtained compound was dissolved in methanol (5 mL). To the reaction mixture was added hydrazine monohydrate (0.5mL) and the mixture was stirred at 90°C for 6 hours. The solvent was removed under reduced pressure, and the obtained residue was purified by preparative reverse phase column chromatography (Shiseido CAPSELL PAC C18UG80, 5µm, 20x50mm, flow rate 30mL/minutes linear gradient, water/methanol =70/30-10/90) to give the title compound (0.50g).
¹H-NMR (DMSO-d₆) δ ppm:
1.65-1.90 (2H, m), 2.69 (2H, t, J=6.7Hz), 3.60-3.76 (2H, m), 3.85-4.20 (4H, m), 4.33-4.46 (1H, m), 4.53 (2H, d, J=5.8Hz), 5.83 (1H, d, J=7.7Hz), 6.70-7.55 (9H, m)

### Example 110

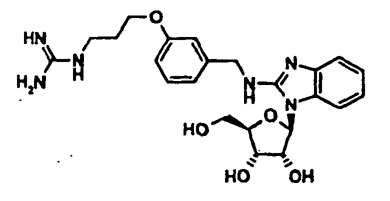

### 2-[3-(3-Guanidinopropoxy)benzylamino]-1-(β-D-ribofuranosyl)-1H-benzimidazole

2-[3-(3-Aminopropoxy)benzylamino]-1-(β-D-ribofuranosyl)-*1H*-benzimidazole (0.2g) and *N*-(benzyloxycarbonyl)-*1H*-pyrazol-1-carboxamidine (0.55g) were suspended in tetrahydrofuran (2.5mL), and the mixture was stirred at 60°C for 24 hours. The reaction mixture was concentrated under reduced pressure, and the obtained residue was dissolved in methanol (4 mL). To the solution was added a catalytic amount of 10% palladium-carbon powder, and the mixture was stirred at 40°C under a hydrogen atmosphere for 2 hour. The insoluble material was removed by filtration, and the filtrate was concentrated under reduced pressure. The obtained residue was purified by preparative reverse phase column chromatography (Shiseido CAPSELL PAC C18UG80, 5µm, 20×50mm, flow rate 30mL/minutes linear gradient, water/methanol =70/30-10/90) to give the title compound (0.01g).
¹H-NMR (CD₃OD) δ ppm:
1.95-2.05 (2H, m), 3.80-3.85 (2H, m), 4.00-4.30 (4H, m), 4.55-4.65 (3H, m), 5.45-5.55 (2H, m), 5.95 (1H, d, J=7.4Hz), 6.75-6.85 (1H, m), 6.90-7.10 (4H, m), 7.15-7.30 (3H, m)

### Example 111

The compounds of Table 25 can be prepared in a similar manner to that described in Example 110 using the corresponding materials.

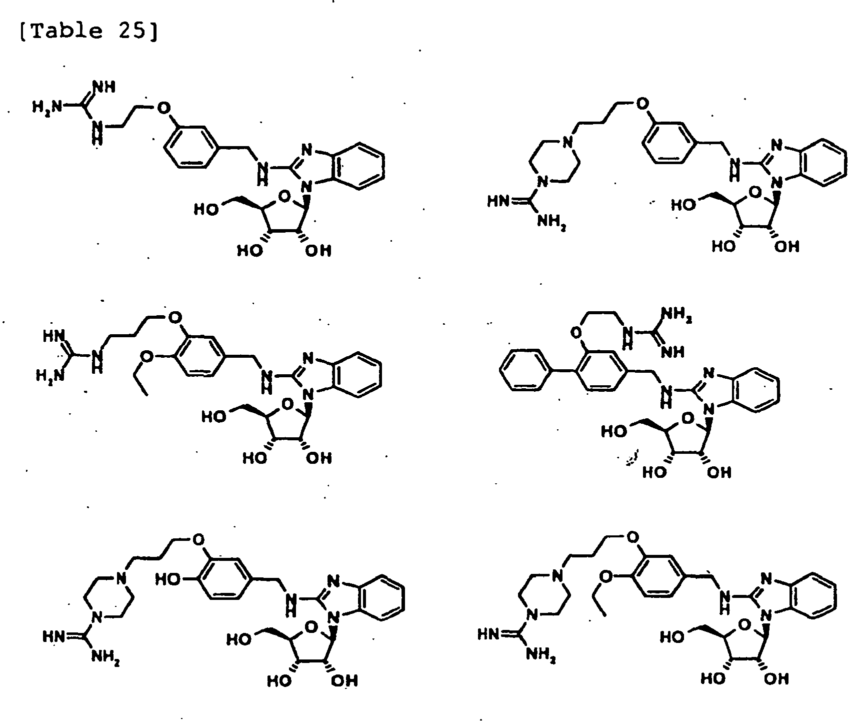

### Example 112

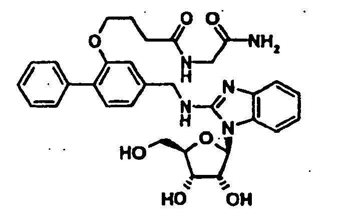

### 2-{3-[3-(Carbamoylmethylcarbamoyl)propoxy]-4-phenylbenzylamino}-1-(β-D-ribofuranosyl)-1H-benzimidazole

2-[3-(3-Carboxypropoxy)-4-phenylbenzylamino]-1-(β-D-ribofuranosyl)-*1H*-benzimidazole (50mg), glycinamide hydrochloride (17mg), 1-hydroxybenzotriazole (29mg) and triethylamine (47mg) were suspended in *N,N*-dimethylformamide (2mL) at room temperature. To the mixture was added 1-ethyl-3-(3-dimethylaminopropyl)carbodiimide hydrochloride (36mg), and the mixture was stirred at room temperature for 17 hours. The reaction mixture was concentrated under reduced pressure, and the obtained residue was purified by preparative reverse phase column chromatography (Shiseido CAPSELL PAC C18UG80, 5µm, 20×50mm, flow rate 30mL/minutes linear gradient, water/methanol =70/30-10/90) togive the title compound (23mg).
¹H-NMR (CD₃OD) δ ppm:
1.90-2.05 (2H, m), 2.25-2.40 (2H, m), 3.75 (2H, s), 3.80-3.90 (2H, m), 3.95-4..05 (2H, m), 4.10-4.35 (2H, m), 5.97 (1H, d, J=7.2Hz), 6.90-7.55 (12H, m)

### Examples 113-114

The compounds of Table 26 were prepared in a similar manner to that described in Example 112 using the corresponding materials.

**[Table 26]**

| Example No. | Structure | ¹H-NMR δ ppm |
|---|---|---|
| Example 113 | 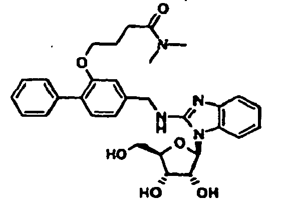 | (CD₃OD) 1.85-2.05 (2H.m), 2.25-2.40 (2H, m), 2.80 (3H, s), 2.82 (3H, s), 3.75-3.90 (2H, m), 3.95-4.05 (2H, m), 4.10-4.20 (1H, m), 4.25-4.35 (1H, m), 4.50-4.80 (3H, m), 5.98 (1H, d, J=7.4Hz), 6.95-7.55 (12H,m) |
| Example 114 | 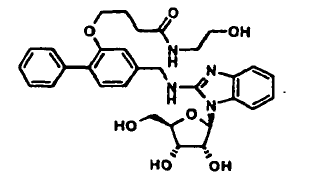 | (CD₃OD) 1.85-2.05 (2H, m), 2.20-2.30 (2H, m), 3.15-3.25 (2H, m), 3.45-3.60 (2H, m). 3.75-3.90 (2H, m). 3.98 (2H, t, J=6.2Hz), 4.10-4.35 (2H, m), 4.55-4.75 (3H, m). 5.98 (1H, d, J=7.6Hz), 6.85-7.55 (12H, m) |

### Example 115

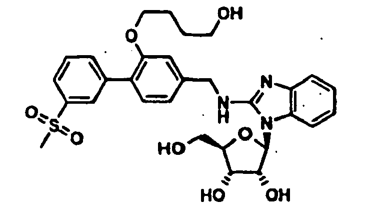

### 2-[3-(4-Hydroxybutoxy)-4-(3-methanesulfonylphenyl)benzylamino]-1-(β-D-ribofuranosyl)-1H-benzimidazole

2-Amino-1-(2,3,5-tri-*O*-acetyl-β-D-ribofuranosyl)-1H-benzimidazole (0.11g) and 3-(4-benzyloxybutoxy)-4-(3-methanesulfonylphenyl)benzaldehyde (0.24g) were suspended in tetrahydrofuran (5mL). To the reaction mixture was added sodium triacetoxyborohydride (0.12g), and the mixture was stirred at room temperature for 24 hours. After adding water to the reaction mixture, the mixture was concentrated under reduced pressure. The obtained residue was dissolved in methanol (2mL) . To the mixture was added 5mol/L aqueous sodium hydroxide solution (0. 5mL), and the mixture was stirred at room temperature for 1 hour. To the reaction mixture was added acetic acid (1mL), and the mixture was concentrated under reduced pressure. The obtained residue was purified by preparative revere phase column chromatography (Shiseido CAPSELL PAC C18UG80, 5µm, 20×50mm, flow rate 30mL/minutes linear gradient, water/methanol =70/30-10/90) to give 2- [3-(4-benzyloxybutoxy) -4-(3-methanesulfonylphenyl)benzylamino]-1-(β-D-ribofuranosyl)-*1H*-benzimidazole (0.16g). The obtained compound was dissolved in methanol (2mL). To the solution was added a catalytic amount of 10% palladium-carbon powder, and the mixture was stirred at 40°C under a hydrogen atmosphere for 24 hour. The insoluble material was removed by filtration, and the filtrate was concentrated under reduced pressure to give the title compound (0.04g).
¹H-NMR (CD₃OD) δ ppm:
1.50-1.85 (4H,m), 3.12 (3H, s), 3.50 (2H, t, J=6.5Hz), 3.77-3.90 (2H, m), 4.04 (2H, t, J=6.1Hz), 4.10-4.35 (2H, m), 4.55-4.80 - (3H, m), 5.98 (1H, d, J=7.7Hz.), 6.94-7.40 (7H, m), 7.55-7.70 (1H, m), 7.75-7.95 (1H, m), 8.13 (1H, s)

### Test Example 1

### Human CNT1 cDNA cloning

Human CNT1 cDNA was obtained by PCR amplification of human kidney cDNA (Origene). PCR reaction solution contained 1 µL cDNA, 2 units Platinum taq DNA polymerase high fidelity (Invitrogen), 1 µM primers (Forward: 5'-TGC ACT GCA TGG TTG CTG CT-3', Reverse: 5'-GTC TAA GTC CTG TGG CTT CC-3'). Amplifications for 1 cycle at 94°C for 2 minutes and 32 cycles at 94°C for 30 seconds, 58°C for 30 seconds and 68°C for 3 minutes were performed and PCR products were ligated into PCR-II-TOPO vector (Invitrogen). The amino acid sequence of cloned human CNT1 was substituted at G34E (codon, GGA to GAA), Q462R (codon, CAG to CGG) and R511C (codon, CGC to TGC) compared to a reported amino acid sequence for human CNT1 (NCBI Accession No.AAB53837.1).

### Test Example 2

### Human CNT2 cDNA cloning and construction of expression plasmid

Human CNT2 cDNA was obtained by PCR amplification of human kidney cDNA (CLONTECH). PCR reaction solution contained 1 µL cDNA, 2 units Platinum taq DNA polymerase high fidelity (Invitrogen), 1 µM primers (Forward: 5'-AGG AGC CAG AGG GAA TCA AT-3', Reverse: 5'-ACA TCT TGG TGA GTG AGT TG-3'). Amplifications for 1 cycle at 94°C for 2 minutes, 32 cycles at 94°C for 30 seconds, 58°C for 30 seconds and 68°C for 3 minutes were performed and PCR products were ligated into PCR-II-TOPO vector (Invitrogen). PCR reaction was performed with primers containing restriction enzyme sites and the constructed plasmid as a template. PCR reaction solution contained 100 ng plasmid, 2 units Pyrobest DNA polymerase (Takara), 330 nM primers (Forward: 5'-CCG CTC GAG AGG AGC CAG AGG GAA TCA AT-3' , Reverse: 5' -CGT CTA GAA CAT CTT GGT GAG TGA GTT G-3'). Amplifications for 1 cycle at 95°C for 3 minutes, 15 cycles at 98°C for 10 seconds, 60°C for 30 seconds and 72°C for 1 minute and 1 cycle at 72°C for 7 minutes were performed, and the PCR products were ligated into PCI-neo mammalian expression vector (Promega). The amino acid sequence of cloned human CNT2 was substituted at P22L (codon, CCG to CTG), S45C (codon, AGC to TGC) and I160M (codon, ATA to ATG) compared to a reported amino acid sequence for human CNT2 (NCBI Accession No.AAC51930).

### Test Example 3

### Human CNT3 cDNA cloning

Human CNT3 cDNA was obtained by PCR amplification of human small intestine cDNA (CLONTECH). PCR reaction solution contained 0.2 µL cDNA, Expand long template PCR system (Roche), 0.5 µM primers (Forward: 5'-GCC AGC CAG CAG CAA AAA-3', Reverse: 5'-TGG AGA AGT GGC TGA CCT-3'). Amplifications for 1 cycle at 94°C for 2 minutes and 33 cycles at 94°C for 10 seconds, 58 °C for 30 seconds and 68 °C for 2 minutes were performed, and PCR products were ligated into PCR-II-TOPO vector (Invitrogen). Nucleotide sequence of cloned human CNT3 was identical to a reported nucleotide sequence for human CNT3 (NCBI Accession No.NM022127) from position 1130 to 1215.

### Test Example 4

### Distribution pattern of human CNTs in human tissues

### 1) Synthesis of cDNA

Total RNAs derived from human liver, colon, testis, pancreas, lung, small intestine, stomach, placenta and muscle were purchased from Sawady Technology , and total RNAs of trachea, brain, kidney and heart were purchased from CLONTECH. Total RNA concentration was determined by RiboGreen RNA quantification reagent and kit (Molecular Probe). cDNAs were synthesized (reverse transcription). A reaction solution (16.5 µL) contained 1.5 pg total RNA and 1.5 µL random hexamer at 500 ng/µL (Invitrogen). The reaction solution was incubated at 70°C for 5 minutes, then at room temperature for 5 minutes. A reaction solution (13.5 µL) containing 6 µL 5 x BRL 1st strand buffer (Invitrogen), 3.25 µL distilled water (Nippongene), 1.5 µL of 10 mM dNTP mix (Invitrogen), 0.75 µL RNase inhibitor (Invitrogen) and 2 µL Superscript II (Invitrogen) was added to the reaction solution described above. Another reaction solution containing distilled water (Nippongene) instead of Superscript II was also added to the solution described above. After all mixtures were incubated at room temperature for 10 minutes and 42°C for 1 hour. To inactivate Superscript II, and the resulting solutions were incubated at 95°C for 10 minutes and transferred to ice immediately. Next, 1.5 µL of RNase H (Invitrogen) was added and the solutions were incubated at 37 °C for 30 minutes. At the end of the reaction, 170 µL of distilled water was added. The synthesized cDNAs were extracted with 200 µL of a mixture (phenol: chloroform: isoamylalcohol = 25:24:1) (Invitrogen) and furthermore extracted with 200 µL of a mixture (chloroform: isoamylalcohol = 24:1). After ethanol precipitation, cDNAs were dissolved in distilled water (Nippongene).

### 2) Determination of human CNTs gene expression by quantitative real-time PCR

For human CNT1 in quantitative real-time PCR, forward: 5'-ATT TAC CAG TGC TGC CGT GAG-3' and reverse: 5'-AAA CCG ACA GCA GTT GTC CAG-3' as primers and 5'-AGA GCG TCA ATC CAG AGT TCA GCC CA-3' as a probe were used. For human CNT2, forward: 5'-GGC AGC TTG CAT CTT GAA TTT C -3' and reverse: 5'-CAA AAA CGA GTG AAC CAG GAC A -3' as primers and 5'-CCT TGT TTG TCA TCA CCT GCT TGG TGA TCT-3' as a probe were used. Probes were labeled with fluorescence dye, FAM at 5' terminal and TAMRA at 3' terminal. A reaction solution (25 µL) contained 2.5 ng cDNA synthesized above, 1 x Taqman Universal master mix (Applied Biosystems), 500 nM forward and reverse primers and 200 nM probe. PCR protocol was as follows. One cycle at 50°C for 2 minutes, 1 cycle at 95°C for 10 minutes and 40 cycles at 95°C for 15 seconds and at 60°C for 1 minute. Assays were performed using GeneAmp 5500 Sequence detection system (Applied Biosystems), MicroAmp optical 96-well reaction plates (Applied Biosystems) and MicroAmp optical caps (Applied Biosystems). Signals were detected according to manufacturer's instruction (See Genome Research, 1996, vol. 6, pp. 986-994). Samples were analyzed with serially (1:10) diluted plasmid DNAs as standard curve. As shown in Figure 1, human CNT1 was expressed in kidney and liver abundantly, on the other hand, human CNT2 was expressed in small intestine and stomach abundantly.

### Test Example 5

### Distribution pattern of human CNTs in stomach and intestine

### Determination of human CNTs gene expression by quantitative real-time PCR

Total RNAs derived from funds of stomach, corpus of stomach, duodenum, jejunum, ileum and ascending colon were purchased from BIOCHAIN. Total RNA concentration was determined by RiboGreenRNA quantification reagent and kit (Molecular Probe). Primers and probes for humanCNT1, 2 were the same in Test Example 4. For human CNT3, forward: 5'-GCT GGT CCG ACC ATA TTT ACC TTA C-3' and reverse: 5'-CGC TTC CAG CAA TGG TAG AGA-3' as primers and 5'-TCA CCA AGT CTG AAC TCC ACG CCA TC-3' as a probe were used. Probe was labeled with fluorescence dye, FAM at 5' terminal and TAMRA at 3' terminal. Reaction solution (25 µL) contained Taqman EZ RT-PCR kit (Applied Biosystems), 500 nM forward and reverse primer and 200 nM probe. PCR protocol was as follows. One cycle at 50°C for 2 minutes, 1 cycle at 60°C for 30 minutes, 1 cycle at 95°C for 5 minutes and 40 cycles at 94°C for 20 seconds and at 62°C for 1 minute. Assays were performed using DNA Engine Opticon (MJ Japan) and 96 well low multipleplates (MJ Japan). Signals were detected according to manufacturer' s instruction (See Genome Research, 1996, vol. 6, pp. 986-994). Samples were analyzed with serially (1:10) diluted plasmid DNAs as standard curve. As shown in Figure 2, human CNT1 was expressed in jejunum and ileum strongly. On the other hand, human CNT2 was expressed in duodenum and jejunum strongly, and CNT2 was also expressed in stomach and colon weakly. Human CNT3 was expressed weakly in all tissues.

### Test Example 6

### Preparation of cells transiently expressing human CNT2

Expression plasmid of human CNT2 was transfected into COS-7 cells (RIKEN CELL BANK RCB0539) by lipofection method. LIPOFECTAMINE 2000 (Invitrogen) was used as a lipofection reagent. COS-7 cells were diluted in D-MEM (Invitrogen) containing 10% fetal calf serum (Sanko Junyaku) at 5 x 10⁵/ 1 mL, and seeded into collagen-coated 96-well plates (IWAKI) at 100 µL/ well and cultured at 37°C for 2 hours with 5% CO₂ condition. For each well, 0.6 µL of LIPOFECTAMINE 2000 (Invitrogen) was diluted in 25 µL of OPTI-MEM (Invitrogen), and incubated for 7 minutes at room temperature (hereinafter referred to as Lipo 2000-OPTI). For each well, 0.3 µg of plasmid was diluted in 25 µL of OPTI-MEM (Invitrogen), and the solution was added to.the Lipo 2000-OPTI and mixed gently and incubated for 30 minutes at room temperature, and was transferred 50 µL for each well to culture medium. The cells were incubated at 37°C with 5% CO₂ condition for 2 days, and used for the uptake assays.

### Test Example 7

### Measurement of inhibitory activity against uptake of adenosine through human CNT2

An Uptake buffer was prepared by addition of a mixture of non-radioisotope labeled (Sigma) and ¹⁴C-labeled (Amersham Biosciences) adenosine at the final concentration of 10 µM into a buffer, pH 7.4, containing 140 mM sodium chloride, 2 mM potassium chloride, 1 mM calcium chloride, 1 mM magnesium chloride, 10 mM HEPES 2-[4-(2-hydroxyethyl)-1-piperazinyl]ethane sulfonic acid, 5 mM tris(hydroxymethyl) aminomethane and 5 mM glucose. For measurement of basal uptake, Basal uptake measurement buffer, which contained 140 mM choline chloride instead of sodium chloride was prepared. In uptake assays, NBMPR was added to Uptake buffer and Basal uptake measurement buffer at the final concentration of 10 µM. In the case of measurement of inhibitory activity of test compounds, test compounds were dissolved in dimethyl sulfoxide, and then appropriately diluted with Uptake buffer as to prepare Measurement buffers. After removing culture medium of human CNT2 transiently expressing cells, Pretreatment buffer (Basal uptake measurement buffer without adenosine and glucose) was added to wells at 200 µL/ well and incubated at 37°C for 10 minutes. After repeating the same step again, Pretreatment buffer was removed and Measurement buffers and Basal uptake measurement buffer were added at 75 µL/ well and incubated at 37°C. After incubation for 30 minutes, Measurement buffers and Basal uptake measurement buffer were removed, and the cells were washed with Washing buffer (Basal uptake measurement buffer witch non-radioisotope labeled adenosine at 10 µM) at 200 µL/well twice. The cells were solubilized with 0.2 mol/L sodium hydroxide at 75 µL / well, and the cell lysates were transferred into PicoPlates (Perkin Elmer). After mixing with 150 µL of MicroScint-40 (Perkin Elmer), radioactivity was measured by means of scintillation counter (Perkin Elmer). One hundred % was set to the difference between the uptake in the control group and the basal uptake, and the uptake of adenosine at each test compound concentration was calculated. The test compound concentration inhibiting 50% uptake of adenosine (IC₅₀ value) was calculated using logit plot. The results are shown in Table 27.

**[Table 27]**

| Test compound | IC₅₀ (nM) |
|---|---|
| Example 27 | 46 |
| Example 33 | 104 |
| Example 50 | 9 |
| Example 35 | 150 |
| Example 36 | 213 |
| Example 88 | 55 |
| Example 89 | 184 |
| Example 92 | 172 |

### Test Example 8

### Effects of CNT2 inhibitors on plasma uric acid level

Male SD-IGS rats (5 weeks old) which were fasted overnight, were subcutaneously treated with oxonic acid (Aldrich; 100 mg/kg), and after 1 hour, purine mix (Adenosine: Inosine: Guanosine = 1:1: 1 (Adenosine (Sigma), Inosine (WAKO), Guanosine (ICN); 50 mg/kg) and test compounds (10 mg/kg) were orally administrated simultaneously. A control group was treated with oxonic acid and the purine mix, and a group administrated only oxonic acid represented endogenous plasma uric acid value. After 1 hour, blood was collected from the abdominal aorta under ether anesthesia, and the plasma was collected with Venoject II vacuum blood collecting tube (Terumo, VP-FH052). According to the method described in Journal of Chromatography B, Vol. 744 (2000), pp.129-138, plasma uric acid level in a compound of Example 27 was measured by using HPLC method mentioned below. Plasma uric acid levels in compounds of Examples 88 and 89 were measured by phosphotungstic acid method. Uric acid-Test Wako (WAKO) was used as a measurement reagent. As there is no difference of uric acid values between the HPLC method and the phosphotungstic acid method, uric acid value can be measured by either of both methods (for example, see the above Management guideline, pp. 18-19). The difference between plasma uric acid value in each study group and endogenous plasma uric acid value was calculated on the basis of 100% in the control group. The results are shown in Table 28.

**[Table 28]**

| Test compound | Percentage of increment of plasma uric acid level (%) |
|---|---|
| Example 27 | 15.2% (p<0.01) |
| Example 88 | 24.9% (p<0.01) |
| Example 89 | 45.6% (p<0.05) |

### Determination of plasma uric acid level by high-performance liquid chromatography (HPLC)

Theophylline (10 µg) as an internal standard substance was added to 0.1 mL of plasma collected with the above method, and then the samples were deproteinized with 1 mL of methanol. After the samples were centrifuged, the methanol layers were evaporated to dryness under a stream of nitrogen. The residues were dissolved in 300 µL of mobile phase, and 40 µL of the portion was injected into HPLC. Plasma uric acid concentration was determined by HPLC method according to the condition described below. Calibration curves were constructed by addition of theophylline as an internal standard substance and several concentrations of uric acid to 0.1 mL of distilled water appropriately.

### HPLC analytical condition

Column: Inertsil ODS-2 (4.6 x 250 mm)
Mobile phase
   A solution: acetonitrile
   B solution: 10 mM phosphate buffer (pH 3.0)
   A linear gradient elution method: A solution 2% to A solution 22% (25 minutes)
Column temperature: 40°C
Flow rate: 0.5 mL/minute
Detection absorbance: 284 nm

### Industrial Applicability

The benzimidazole derivatives represented by the above general formula (I) of the present invention or pharmaceutically acceptable salt thereof, or a prodrug thereof exert an excellent CNT2 inhibitory activity and can markedly inhibit the elevation of plasma uric acid level. Therefore, they are useful as agents for the prevention or treatment of diseases associated with an abnormality of plasma uric acid level.

### [SEQUENCE LISTING FREE TEXT]

Sequence Number 1: Synthetic DNA primer
Sequence Number 2: Synthetic DNA primer
Sequence Number 3: Synthetic DNA primer
Sequence Number 4: Synthetic DNA primer
Sequence Number 5: Synthetic DNA primer
Sequence Number 6: Synthetic DNA primer
Sequence Number 7: Synthetic DNA primer
Sequence Number 8: Synthetic DNA primer
Sequence Number 9: Synthetic DNA primer
Sequence Number 10: Synthetic DNA primer
Sequence Number 11: Synthetic DNA probe
Sequence Number 12: Synthetic DNA primer
Sequence Number 13: Synthetic DNA primer
Sequence Number 14: Synthetic DNA probe
Sequence Number 15: Synthetic DNA primer
Sequence Number 16: Synthetic DNA primer
Sequence Number 17: Synthetic DNA probe

## Claims

1. A benzimidazole derivative represented by the general formula: 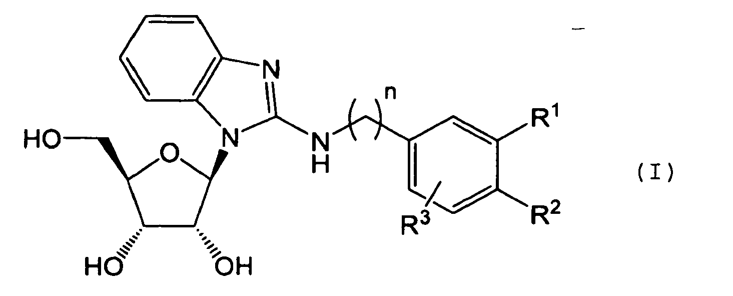 wherein
n represents 1 or 2;
R¹ and R² independently represents a hydrogen atom, a halogen atom, a cyano group, any of the following substituents (A) to (C) which may have the same or different 1 to 3 groups selected from a substituent group α, any of the following substituents (D) to (G) which may have the same or different 1 to 3 groups selected from substituent groups α or β, or any of the following substituents (H) to (M);
R³ represents a hydrogen atom, a halogen atom, any of the following substituents (a) to (C) which may have the same or different 1 to 3 groups selected from a substituent group α, or any of the following substituents (H) to (M);
(A) a C₁₋₆ alkyl group;
(B) a C₂₋₆ alkenyl group;
(C) a C₂₋₆ alkynyl group;
(D) a C₃₋₈ cycloalkyl group;
(E) a 3 to 10-membered cyclic heterocycloalkyl group;
(F) a C₆₋₁₀ aryl group;
(G) a 5 to 10-membered cyclic heteroaryl group;
(H) OR⁷;
(I) SR⁸;
(J) NR⁹R¹⁰;
(K) COOT¹¹;
(L) CONR¹²R¹³;
(M) NHCOR¹⁴
(in the groups R⁷ to R¹⁴ independently represents a hydrogen atom, or any of the following substituents (N) to (P) which may have the same or different 1 to 3 groups selected from a substituent group α, or any of the following substituents (Q) to (V) which may have the same or different 1 to 3 groups selected from substituent groups α and
(N) a C₁₋₆ alkyl group;
(O) a C₂₋₆ alkenyl group;
(P) a C₂₋₆ alkynyl group;
(Q) a C₃₋₈ cycloalkyl group;
(R) a 3 to 10-membered cyclic heterocycloalkyl group;
(S) a quaternary salt of a 3 to 10-membered cyclic nitrogen-containing heterocycloalkyl group;
(T) a C₆₋₁₀ aryl group;
(U) a 5 to 10-membered cyclic heteroaryl group;
(V) a quaternary salt of a 5 to 10-membered cyclic nitrogen-containing heteroaryl group);
and with the proviso that at least one of R¹, R² and R³ does not represent a group selected from a hydrogen atom, a halogen
atom, a hydroxy group, a C₁₋₆ alkoxy group, NH₂ and COOH
[Substituent group α]
(a) a halogen atom;
(b) a cyano group;
any of the following substituents (c) to (h) which may have the same or different 1 to 3 groups selected from a substituent group γ, or any of the following substituents (i) to (v):
(c) a C₃₋₈ cycloalkyl group;
(d) a 3 to 10-membered cyclic heterocycloalkyl group;
(e) a quaternary salt of a 3 to 10-membered cyclic nitrogen-containing heterocycloalkyl group;
(f) a C₆₋₁₀ aryl group;
(g) a 5 to 10-membered cyclic heteroaryl group;
(h) a quaternary salt of a 5 to 10-membered cyclic nitrogen-containing heteroaryl group;
(i) OR¹⁵;
(j) SR1⁶;
(k) NR¹⁷R¹⁸;
(l) N⁺R^{D}R^{E}R^{F};
(m) COOR¹⁹;
(o) NHCOR²⁰;
(p) NHC(=NH)-NH₂;
(q) C(=NH)-NH₂ (which is bound to a nitrogen atom of a nitrogen-containing heterocycloalkyl group);
(r) NR²¹CONR²²R²³;
(s) NR^{G}SO₂R^{H};
(t) SO₂R^{I} (R^{I} represents a C₁₋₆ alkyl group, a C₂₋₆ alkenylene group or a hydroxy(C₁₋₆ alkyl) group);
(u) CONR²⁴R²⁵;
(v) SO₂NR²⁶R²⁷
(in the groups R^{D-F} independently represent any of the following substituents (y1) to (y11) which may have the same or different 1 to 3 groups selected from a substituent group γ; R¹⁵, R¹⁶, R¹⁹⁻²¹ and R^{G-H} independently represent a hydrogen atom, or any of the following substituents (y1) to (y11) which may have the same or different 1 to 3 groups selected from a substituent group γ; R¹⁷, R¹⁸ and R²² to R²⁷ independently represent a hydrogen atom, or any of the following substituents (y1) to (y11) which may have the same or different 1 to 3 groups selected from a substituent group γ; or R¹⁷ and R¹⁸, R²² and R²³, R²⁴ and R²⁵, and R²⁶ and R²⁷ independently may bind together with the neighboring nitrogen atom to form a 3 to 8-membered aliphatic cyclic amino group
(y1) a C₁₋₆ alkyl group;
(y2) a C₂₋₆ alkenyl group;
(y3) a C₂₋₆ alkynyl group;
(y4) a C₃₋₈ cycloalkyl group;
(y5) a 3 to 10-membered cyclic heterocycloalkyl group;
(y6) a C₆₋₁₀aryl group;
(y7) a 5 to 10-membered cyclic heteroaryl group;
(y8) a C₃₋₈ cycloalkyl-C₁₋₆ alkyl group;
(y9) a 3 to 10-membered cyclic heterocycloalkyl-C₁₋₆ alkyl group;
(y10) a C₆₋₁₀ aryl-C₁₋₆ alkyl group;
(y11) a 5 to 10-membered cyclic heteroaryl-C₁₋₆ alkyl group)
[Substituent group β]
any of the following substituents (z1 to (z3) which may have the same or different 1 to 3 groups selected from a substituent group γ; substituent group γ:
(z1) a C₁₋₆ alkyl group;
(z2) a C₂₋₆ alkenyl group;
(z3) a C₂₋₆ alkynyl group
[Substituent group γ]
(1) a halogen atom;
(2) a nitro group;
(3) a cyano group;
(4) OR²⁸;
(5) SR²⁹;
(6) NR³⁰R^{J} (R³⁰ and R^{J} independently represent a hydrogen atom, a C₁₋₆ alkyl group, a C₂₋₆ alkenyl group, a hydroxy(C₁₋₆ alkyl) group, a C₆₋₁₀ aryl-C₁₋₆ alkyl group or a C₆₋₁₀ aryl group);
(7) N⁺R^{K}R^{L}R^{M} (R^{K-M} independently represent a C₁₋₆ alkyl group, a C₂₋₆ alkenyl group, a hydroxy (C₁₋₆ alkyl) group, a C₆₋₁₀ aryl-C₁₋₆ alkyl group or a C₆₋₁₀ aryl group);
(8) COR³¹;
(9) COOR³²;
(10) OCOR³³;
(11) NHCOR³⁴;
(12) NHC(=NH)-NH₂;
(13) C(=NH)-NH₂ (which is bound to a nitrogen atom of a heterocycloalkyl group)
(14) NR³⁵CONR³⁶R³⁷;
(15) NR^{N}COOR^{o};
(16) CONR³⁸R³⁹;
(17) SO²NR⁴⁰R⁴¹;
(18) a hydroxy(C₂₋₆ alkyl) group;
(19) a 5 to 10-membered cyclic nitrogen-containing heteroaryl group
(in the groups R²⁸, R²⁹, R³¹⁻³⁵, R^{N} and R^{O} independently represent a hydrogen atom, a C₁₋₆ alkyl group or a C₆₋₁₀ aryl-C₁₋₆ alkyl group; R³⁶ to R⁴¹ independently represent a hydrogen atom, or a C₁₋₆ alkyl group, or R³⁶ and R³⁷, R³⁸ and R³⁹, and R⁴⁰ and R⁴¹ independently may bind together with the neighboring nitrogen atom to form a 3 to 8-membered aliphatic cyclic amino group); or a pharmaceutically acceptable salt thereof; or a prodrug thereof wherein a group selected from a hydroxy group and an amino group of the benzimidazole derivative is substituted by a group selected from the group consisting of C₁₋₆ alkyl-CO-, C₁-₆ alkyl-O-C₁₋₆ alkyl-CO-, C₁₋₆ alkyl-OCO-C₁₋₆ alkyl-CO-, C₁₋₆ alkyl-OCO- and C₁₋₆ alkyl-O-C₁₋₆ alkyl-OCO-.

2. A benzimidazole derivative as claimed in claim 1 wherein n represents 1, or a pharmaceutically acceptable salt thereof, or a prodrug thereof.

3. A benzimidazole derivative as claimed in claims 1 to 2 wherein R¹ and R³ independently represent a hydrogen atom, a halogen atom, any of the substituents (A) to (C) which may have the same or different 1 to 3 groups selected from the substituent group α, or any of the substituents (H) to (M), R² independently represents a hydrogen atom, a halogen atom, a cyano group, any of the substituents (A) to (C) which may have the same or different 1 to 3 groups selected from the substituent group α, any of the substituents (D) to (G) which may have the same or different 1 to 3 groups selected from the substituent groups α and β, or any of the substituents (H) to (M), or a pharmaceutically acceptable salt thereof, or a prodrug thereof.

4. A benzimidazole derivative as claimed in claim 3 wherein R¹ represents OR⁷ (with the proviso that R⁷ represents a C₁₋₆ alkyl group which has a hydroxy group, NR¹⁷R¹⁸ or N⁺R^{D}R^{E}R^{F} (R¹⁷, R¹⁸ and R^{D-F} have the same meanings as defined in claim 1)) or a hydroxy group; R² represents OR⁷ (with the proviso that R⁷ represents a C₁₋₆ alkyl group which has a hydroxy group, NR¹⁷R¹⁸ or N⁺R^{D}R^{E}R^{F} (R¹⁷, R¹⁸ and R^{D-F} have the same meanings as defined in claim 1)), a hydroxy group, or a C₆₋₁₀ aryl group which may have a hydroxy group or OR¹⁵ (R¹⁵ has the same meaning as defined in claim 1); R³ represents a hydrogen atom; or a pharmaceutically acceptable salt thereof, or a prodrug thereof.

5. A pharmaceutical composition comprising as an active ingredient a benzimidazole derivative as claimed in claims 1 to 4, or a pharmaceutically acceptable salt thereof, or a prodrug thereof.

6. A pharmaceutical composition as claimed in claim 5 for the prevention or treatment of a disease associated with an abnormality of plasma uric acid level.

7. A pharmaceutical composition as claimed in claim 6 wherein the disease associated with an abnormality of plasma uric acid level is a disease selected from gout, hyperuricemia, urinary lithiasis, hyperuricemic nephropathy and acute uric acid nephropathy.

8. A pharmaceutical composition as claimed in claim 6 wherein the disease associated with an abnormality of plasma uric acid level is gout.

9. A pharmaceutical composition as claimed in claim 6 wherein the disease associated with an abnormality of plasma uric acid level is hyperuricemia.

10. A pharmaceutical composition as claimed in any one of claims 5 to 9 comprising in combination as an active ingredient at least one agent selected from a group consisting of colchicine, a nonsteroidal anti-inflammatory agent, an adrenocortical steroid, a uric acid synthesis inhibitor, a uricosuric drug, a urinary alkalinizer and a uric acid oxidase.

11. A pharmaceutical composition as claimed in claim 10 wherein the nonsteroidal anti-inflammatory agent is indometacin, naproxen, fenbufen, pranoprofen, oxaprozin, ketoprofen, etoricoxib or tenoxicam; the uric acid synthesis inhibitor is allopurinol, oxypurinol, febuxostat or Y-700; the uricosuric drug is probenecid, bucolome or benzbromarone; the urinary alkalinizer is sodium hydrogen carbonate, potassium citrate or sodium citrate; the uric acid oxidase is rasburicase, uricase PEG-20, a recombinant acid oxidase (uricase).

## Patentansprüche

1. Benzimadazolderivat, welches durch die nachfolgende allgemeine Formel dargestellt wird: 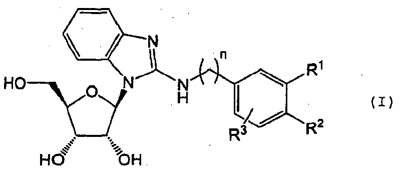 wobei:
n 1 oder 2 darstellt;
R¹ und R² unabhängig voneinander ein Wasserstoffatom, ein Halogenatom, eine Cyanogruppe, einen beliebigen der nachfolgenden Substituenten (A) bis (C), welche dieselben oder unterschiedliche aus einer Substituentengruppe α ausgewählte 1 bis 3 Gruppen aufweisen können, jeden beliebigen der nachfolgenden Substituenten (D) bis (G),
welche dieselben oder unterschiedliche aus den Substituentengruppen α und β ausgewählte 1 bis 3 Gruppen aufweisen können, oder jeden beliebigen Substituenten (H) bis (M) darstellen;
R³ ein Wasserstoffatom, ein Halogenatom, einen beliebigen der folgenden Substituenten (A) bis (C), welche dieselbe oder unterschiedliche aus einer Substituentengruppe α ausgewählte 1 bis 3 Gruppen aufweisen können, oder jeden beliebigen der nachfolgenden Substituenten (H) bis (M); wobei:
(A) eine C₁₋₆ Alkylgruppe ; ist
(B) eine C₂₋₆ Alkenylgruppe ist;
(C) eine C₂₋₆ Alkinylgruppe ist;
(C) eine C₃₋₈ Cycloalkylgruppe ist ;
(E) eine 3- bis 10-gliedrige cyclische Heterocycloalkylgruppe ist;
(F) eine C₆₋₁₀ Arylgruppe ist ;
(G) eine 5- bis 10-gliedrige cyclische Heteroarylgruppe ist ;
(H) OR⁷ ist;
(I) SR⁸ ist;
(J) NR⁹R¹⁰ ist;
(K) COOR¹¹ ist;
(L) CONR¹²R¹³ ist ;
(M) NHCOR¹⁴ ist;
(wobei in den Gruppen R⁷ bis R¹⁴ unabhängig voneinander ein Wasserstoffatom, oder einen beliebigen der nachfolgenden Substituenten (N) bis (P), welche dieselben oder unterschiedliche aus einer Substituentengruppe α ausgewählte 1 bis 3 Gruppen aufweisen können, oder jeden beliebigen der nachfolgenden Substituenten (Q) bis (V), welche dieselben oder unterschiedliche aus den Substituentengruppen α und β ausgewählte 1 bis 3 Gruppen aufweisen können, darstellen, wobei:
(N) eine C₁₋₆ Alkylgruppe ist;
(O) eine C₂₋₆ Alkenylgruppe ist;
(P) eine C₂₋₆ Alkinylgruppe ist;
(Q) eine C₃₋₈ Cycloalkylgruppe ist;
(R) eine 3- bis 10-gliedrige cyclische Heterocycloalkylgruppe ist;
(S) ein quaternäres Salz einer 3- bis 10-gliedrigen stickstoffhaltigen Heterocycloalkylgruppe ist;
(T) eine C₆₋₁₀ Arylgruppe ist;
(U) eine 5- bis 10-gliedrige cyclische Heteroarylgruppe;
(V) ein quaternäres Salz einer 5- bis 10 gliedrigen stickstoffhaltigen Heteroarylgruppe ist);
und mit der Maßgabe, dass zumindest entweder R¹, R² oder R³ keine Gruppe darstellen, welche aus einem Wasserstoffatom, einem Halogenatom, einer Hydroxygruppe,
einer C₁₋₆ Alkoxygruppe, NH₂ und COOH ausgewählt wird;
[Substituentengruppe α]
(a) ein Halogenatom;
(b) eine Cyanogruppe;
jeden beliebigen der folgenden Substituenten (c) bis (h), welche dieselben oder unterschiedliche aus einer Substituentengruppe γ ausgewählte 1 bis 3 Gruppen aufweisen können, oder jeden beliebigen der folgenden Substituenten (i) bis (v), wobei:
(c) eine C₃₋₈ Cycloalkylgruppe ist;
(d) eine 3 bis 10-gliedrige cyclische Heterocycloalkylgruppe ist;
(e) ein quaternäres Salz einer 3- bis 10-gliedrigen cyclischen stickstoffhaltigen Heterocycloalkylgruppe ist ;
(f) eine C₆₋₁₀ Arylgruppe ist;
(g) eine 5- bis 10-gliedrige cyclische Heteroarylgruppe ist;
(h) ein quaternäres Salz einer 5- bis 10-gliedrigen cyclischen stickstoffhaltigen Heteroarylgruppe ist;
(i) OR¹⁵ ist;
(j) SR¹⁶ ist ;
(k) NR¹⁷R¹⁸ ist;
(l) N⁺R^{D}R^{E}R^{F} ist;
(m) COOR¹⁹ ist ;
(o) NHCOR²⁰ ist;
(p) NHC(=NH)-NH₂ ist;
(q) C(=NH)-NH₂ ist (welcher an ein Stickstoffatom einer stickstoffhaltigen Heterocycloalkylgruppe gebunden ist);
(r) NR²¹CONR²²R²³ ist;
(s) NR^{G}SO₂R^{H} ist;
(t) SO₂R^{I} ist (wobei R^{I} *eine C₁₋₆ Alkylgruppe, ein C₂₋₆ Alkenylgruppe oder eine Hydroxy(C₁₋₆ Alkyl)Gruppe darstellt;*
*(u) CONR²⁴R²⁵* ist;
*(v) SO₂NR²⁶R²⁷* ist;
*(wobei in den Gruppen R^{D-F} unabhängig voneinander jeden beliebigen der folgenden Substituenten (y1) bis (y11) darstellen, welche* dieselben oder unterschiedliche aus einer Substituentengruppe γ ausgewählte 1 bis 3 Gruppen aufweisen können; wobei R¹⁵, R¹⁶, R¹⁹⁻²¹ und R^{G-H} unabhängig voneinander ein Wasserstoffatom oder jeden beliebigen der folgenden Substituenten (y1) bis (y11) darstellen können, welche dieselben oder unterschiedliche aus einer Substituentengruppe γ ausgewählte 1 bis 3 Gruppen aufweisen können; wobei R¹⁷, R¹⁸ und R²² bis R²⁷ unabhängig voneinander ein Wasserstoffatom oder jeden beliebigen der folgenden Substituenten (y1) bis (y11) darstellen können, welche dieselben oder unterschiedliche aus einer Substituentengruppe γ ausgewählte 1 bis 3 Gruppen aufweisen können; oder R¹⁷ und R¹⁸, R²² und R²³, R²⁴ und R²⁵, sowie R²⁶ und R²⁷ sich unabhängig voneinander an das benachbarte Stickstoffatom binden können, um eine 3- bis 8-gliedrige aliphatische cyclische Aminogruppe zu bilden, wobei:
(y1) eine C₁₋₆ Alkylgruppe ist;
(y2) eine C₂₋₆ Alkenylgruppe ist;
(y3) eine C₂₋₆ Alkinylgruppe ist;
(y4) eine C₃₋₈ Cycloalkylgruppe ist;
(y5) eine 3- bis 10-gliedrige cyclische Heterocycloalkylgruppe ist;
(y6) eine C₆₋₁₀ Arylgruppe ist:
(y7) eine 5- bis 10-gliedrige cyclische Heteroarylgruppe ist;
(y8) eine C₃₋₈ Cycloalkyl-C₁₋₆ Alkylgruppe ist;
(y9) eine 3- bis 10-gliedrige cyclische Heterocycloalkyl-C₁₋₆ Alkylgruppe ist;
(y10) eine C₆₋₁₀ Aryl-C₁₋₆ Alkylgruppe ist;
(y11) eine 5-bis 10-gliedrige cyclische Heteroaryl-C₁₋₆ Alkylgruppe ist)
[Substituentengruppe β]
jeden beliebigen der nachfolgenden Substituenten (z1) bis (z3), welche dieselben oder unterschiedliche aus einer Substituentengruppe γ ausgewählte 1 bis 3 Gruppen aufweisen können; Substituentengruppe γ, wobei:
(z1) eine C₁₋₆ Alkylgruppe ist;
(z2) eine C₂₋₆ Alkenylgruppe ist;
(z3) eine C₂₋₆ Alkinylgruppe ist;
[Substituentengruppe γ]
(1) ein Halogenatom;
(2) eine Nitrogruppe;
(3) eine Cyanogruppe;
(4) OR²¹;
(5) SR²⁹;
(6) NR³⁰R^{J} (R³⁰ und R^{J} stellen unabhängig voneinander ein Wasserstoffatom, eine C₁₋₆ Alkylgruppe, eine C₂₋₆Alkenylgruppe, eine Hydroxy(C₁₋₆ Alkyl)Gruppe, eine C₆₋₁₀ Aryl-C₁₋₆ Alkylgruppe oder eine C₆₋₁₀ Arylgruppe dar);
(7) N⁺R^{K}R^{L}R^{M} (R^{K-M} stellen unabhängig voneinander eine C₁₋₆ Alkylgruppe, eine C²⁻⁶ Alkenylgruppe, eine Hydroxy(C₁-₆ Alkyl)Gruppe, eine C₆₋₁₀ Aryl-C₁₋₆ Alkylgruppe oder eine C₆₋₁₀ Arylgruppe dar);
(8) COR³¹;
(9) COOR³²;
(10) OCOR³³;
(11) NHCOR³⁴;
(12) NHC(=NH)-NH₂;
(13) C(=NH)-NH₂ (welcher an ein Stickstoffatom einer Heterocycloalkylgruppe gebunden ist);
(14) NR³⁵CONR³⁶R³⁷;
(15) NR^{N}COOR^{O};
(16) CONR³⁸R³⁹;
(17) SO₂NR⁴⁰R⁴¹;
(18) eine Hydroxy(C₂₋₆Alkyl)Gruppe;
(19) eine 5- bis 10-gliedrige cyclische stickstoffhaltige Heteroarylgruppe;
(in den Gruppen stellen R²⁸, R²⁹, R³¹⁻³⁵, R^{N} und R^{O} unabhängig voneinander ein Wasserstoffatom, eine C₁₋₆ Alkylgruppe oder eine C₆₋₁₀ Aryl-C₁₋₆ Alkylgruppe dar; R³⁶ bis R⁴¹ stellen unabhängig voneinander ein Wasserstoffatom oder eine C₁₋₆ Alkylgruppe dar; oder R³⁶ und R³⁷, R³⁸ und R³⁹, und R⁴⁰ und R⁴¹ können sich unabhängig voneinander an das benachbarte Stickstoffatom binden, um eine 3- bis 8-gliedrige aliphatische cyclische Aminogruppe zu bilden); oder ein pharmazeutisch annehmbares Salz davon; oder ein Pro-Pharmkon davon, wobei eine Gruppe, welche aus einer Hydroxygruppe und einer Aminogruppe des Benzimidazolderivats ausgewählt wird, durch eine Gruppe substituiert wird, welche aus der Gruppe bestehend aus C₁₋₆ Alkyl-CO-, C₁₋₆ Alkyl-O-C₁₋₆ Alkyl-CO, C₁₋₆ Alkyl-OCO-C₁₋₆ Alkyl-CO-, C₁₋₆ Alkyl-OCO- und C₁₋₆ Alkyl-O-C₁₋₆ Alkyl-OCO- ausgewählt wird.

2. Benzimidazolderivat nach Anspruch 1, **dadurch gekennzeichnet, dass** n 1 darstellt, oder ein pharmazeutisch annehmbares Salz davon, oder ein Pro-Pharmakon davon.

3. Benzimidazolderivat nach Anspruch 1 und 2, **dadurch gekennzeichnet, dass** R¹ und R³ unabhängig voneinander ein Wasserstoffatom, ein Halogenatom, einen beliebigen der Substituenten (A) bis (C), welche dieselben oder unterschiedliche aus der Substituentengruppe α ausgewählte 1 bis 3 Gruppen aufweisen können, oder jeden beliebigen der Substituenten (H) bis (M) darstellen, dass R² unabhängig ein Wasserstoffatom, ein Halogenatom, eine Cyanogruppe, jeden beliebigen der Substituenten (A) bis (C), welche dieselben oder unterschiedliche aus einer Substituentengruppe α ausgewählte 1 bis 3 Gruppen aufweisen können, jeden beliebigen der Substituenten (C) bis (G), welche dieselben oder unterschiedliche aus den Substituentengruppen α und β ausgewählte 1 bis 3 Gruppen aufweisen können, oder jeden beliebigen der Substituenten (H) bis (M) darstellt, oder ein pharmazeutisch annehmbares Salz davon, oder ein Pro-Pharmakon davon.

4. Benzimidazolderivat nach Anspruch 3, **dadurch gekennzeichnet, dass** R¹ OR⁷ darstellt (unter der Maßgabe, dass R⁷ eine C₁₋₆ Alkylgruppe darstellt, welche eine Hydroxygruppe, NR¹⁷R¹⁸ oder N⁺R^{D}R^{E}R^{F} aufweist (R¹⁷, R¹⁸ und R^{D-F} haben dieselbe Bedeutung gemäß Definition in Anspruch 1)) oder eine Hydroxygruppe; dass R² OR⁷ (unter der Maßgabe, dass R⁷ eine C₁₋₆ Alkylgruppe darstellt, welche eine Hydroxygruppe, NR¹⁷R¹⁸ oder N⁺R^{D}R^{E}R^{F} aufweist (R¹⁷, R¹⁸ und R^{D-F} haben dieselbe Bedeutung gemäß Definition in Anspruch 1)), eine Hydroxygruppe, oder eine C₆₋₁₀ Arylgruppe darstellt, welche eine Hydroxygruppe oder OR¹⁵ aufweisen kann (R¹⁵ hat dieselbe Bedeutung gemäß Definition in Anspruch 1); und dass R³ ein Wasserstoffatom darstellt; oder ein pharmazeutisch annehmbares Salz davon, oder eine Pro-Pharmakon davon.

5. Pharmazeutische Zusammensetzung, welche als aktiven Bestandteil ein Benzimidazolderivat nach Anspruch 1 oder 4 oder ein pharmazeutisch annehmbares Salz davon oder ein Pro-Pharmakon davon aufweist.

6. Pharmazeutische Zusammensetzung nach Anspruch 5 zur Verhinderung oder zur Behandlung einer Krankheit, welche mit einer Abnormalität des Plasma-Harnsäurespiegels verbunden ist.

7. Pharmazeutische Zusammensetzung nach Anspüruch 6, **dadurch gekennzeichnet, dass** die mit einer Abnormalität des Plasma-Harnsäurespiegels verbundene Krankheit eine Krankheit ist, welche aus Gicht, Hyperurikämie, Harnlithiasis, hyperurikämische Nephropathie und akuter Harnsäure-Nephropathie ausgewählt wird.

8. Pharmazeutische Zusammensetzung nach Anspruch 6, **dadurch gekennzeichnet, dass** die mit einer Abnormalität des Plasma-Harnsäurespiegels verbundene Krankheit Gicht ist.

9. Pharmazeutische Zusammensetzung nach Anspruch 6, **dadurch gekennzeichnet, dass** die mit einer Abnormalität des Plasma-Harnsäurespiegels verbundene Krankheit Hyperurikämie ist.

10. Pharmazeutische Zusammensetzung nach einem der Ansprüche 5 bis 9, welche in Kombination als aktiven Bestandteil mindestens einen Wirkstoff aufweist, welcher aus einer Gruppe bestehend aus Colchicin, einem nichtsteroidalen entzündungshemmenden Wirkstoff, einem adrenokortikalem Steroid, einem Harnsäure-Synthese-Hemmstoff, einem Urikosurikum, einem Harn-Alkalinizer und einer Harnsäure-Oxidase ausgewählt wird.

11. Pharmazeutische Zusammensetzung nach Anspruch 10, **dadurch gekennzeichnet, dass** der nichtsteroidale entzündungshemmende Wirkstoff Indometacin, Naproxen, Fenbufen, Pranoprofen, Oxaprozin, Ketoprofen, Etoricoxib oder Tenoxicam ist; dass der Harnsäure-Synthese-Hemmstoff Allopurinol, Oxypurinol, Febuxostat oder Y-700 ist; dass das Urikosurikum bzw. urikosurische Mittel Probenecid, Bucolome oder Benzbromaron ist; dass es sich bei dem Harn-Alkalinizer um Natriumhydrogencarbonat, Kaliumcitrat oder Natriumcitrat handelt; und dass es sich bei der Harnsäure-Oxidase um Rasburicase, Uricase PEG-20, eine rekombinante Säureoxidase (Uricase) handelt.

## Revendications

1. Dérivé de benzimidazole représenté par la formule générale : 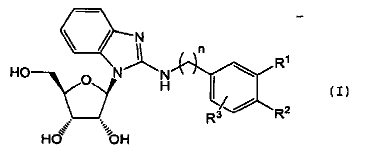 dans laquelle
n est égal à 1 ou 2 ;
R¹ et R² représentent indépendamment un atome d'hydrogène, un atome d'halogène, un groupe cyano, n'importe lequel des substituants (A) à (C) suivants qui peuvent porter 1 à 3 groupes identiques ou différents choisis parmi les groupes servant de substituant α, n'importe lequel des substituants (D) à (G) suivants qui peuvent porter 1 à 3 groupes identiques ou différents choisis parmi les groupes servant de substituants α ou β, ou n'importe lequel des substituants (H) à (M) suivants ;
R³ représente un atome d'hydrogène, un atome d'halogène, n'importe lequel des substituants (A) à (C) suivants qui peuvent porter 1 à 3 groupes identiques ou différents choisis parmi les groupes servant de substituant α ou n'importe lequel des substituants (H) à (M) suivants ;
(A) un groupe alkyle en C₁ à C₆ ;
(B) un groupe alcényle en C₂ à C₆ ;
(C) un groupe alcynyle en C₂ à C₆ ;
(D) un groupe cycloalkyle en C₃ à C₈ ;
(E) un groupe hétérocycloalkyle cyclique tri- à décagonal;
(F) un groupe aryle en C₆ à C₁₀;
(G) un groupe hétéroaryle cyclique penta- à décagonal ;
(H) un groupe OR⁷ ;
(I) un groupe SR⁸;
(J) un groupe NR⁹R¹⁰;
(K) un groupe COOR¹¹ ;
(L) un groupe CONR¹²R¹³ ;
(M) un groupe NHCOR¹⁴
(dans les groupes, R⁷ à R¹⁴ représentent indépendamment un atome d'hydrogène, ou n'importe lequel des substituants (N) à (P) suivants qui peuvent porter 1 à 3 groupes identiques ou différents choisis parmi les groupes servant de substituant α, ou n'importe lequel des substituants (Q) à (v) suivants qui peuvent porter 1 à 3 groupes identiques ou différents choisis parmi les groupes servant de substituants α et β
(N) un groupe alkyle en C₁ à C₆ ;
(O) un groupe alcényle en C₂ à C₆ ;
(P) un groupe alcynyle en C₂ à C₆ ;
(Q) un groupe cycloalkyle en C₃ à C₈ ;
(R) un groupe hétérocycloalkyle cyclique tri- à décagonal;
(S) un sel quaternaire d'un groupe hétérocycloalkyle azoté cyclique tri- à décagonal ;
(T) un groupe aryle en C₆ à C₁₀ ;
(U) un groupe hétéroaryle cyclique penta- à décagonal ;
(V) un sel quaternaire d'un groupe hétéroaryle azoté cyclique penta- à décagonal ;
et sous réserve qu'au moins un de R¹, R² et R³ ne représente pas un groupe choisi entre un atome d'hydrogène, un atome d'halogène, un groupe hydroxy, un groupe alkoxy en C₁ à C₆, un groupe NH₂ et un groupe COOH
[Groupe servant de substituant α]
(a) un atome d'halogène ;
(b) un groupe cyano ;
n'importe lequel des substituants (c) à (h) suivants qui peuvent porter 1 à 3 groupes identiques ou différents choisis parmi les groupes servant de substituant γ, ou n'importe lequel des substituants (i) à (v) suivants :
(c) un groupe cycloalkyle en C₃ à C₈ ;
(d) un groupe hétérocycloalkyle cyclique tri- à décagonal;
(e) un sel quaternaire d'un groupe hétérocycloalkyle azoté cyclique tri- à décagonal ;
(f) un groupe aryle en C₆ à C₁₀ ;
(g) un groupe hétéroaryle cyclique penta- à décagonal ;
(h) un sel quaternaire d'un groupe hétéroaryle azoté cyclique penta- à décagonal ;
(i) un groupe OR¹⁵ ;
(j) un groupe SR¹⁶ ;
(k) un groupe NR¹⁷R¹⁸ ;
(l) un groupe N⁺R^{D}R^{E}R^{F} ;
(m) un groupe COOR¹⁹ ;
(o) un groupe NHCOR²⁰ ;
(p) un groupe NHC(=NH)-NH₂ ;
(q) un groupe C(=NH)-NH₂ (qui est lié à un atome d'azote d'un groupe hétérocycloalkyle azoté ;
(r) un groupe NR²¹CONR²²R²³ ;
(s) un groupe NR^{G}SO₂R^{H} ;
(t) un groupe SO₂R^{I} (R^{I} représente un groupe alkyle en C₁ à C₆, un groupe alcénylène en C₂ à C₆ ou un groupe hydroxy(alkyle en C₁ à C₆) ;
(u) un groupe CONR²⁴R²⁵ ;
(v) un groupe SO₂NR²⁶R²⁷ ;
(dans les groupes, R^{D-F} représentent indépendamment n'importe lequel des substituants (y1) à (y11) suivants qui peuvent porter 1 à 3 groupes identiques ou différents choisis parmi des groupes servant de substituant γ, R¹⁵, R¹⁶, R¹⁹⁻²¹ et R^{G-H} représentent indépendamment un atome d'hydrogène, ou n'importe lequel des substituants (γ1) à (y11) suivants qui peut porter 1 à 3 groupes identiques ou différents choisis parmi des groupes servant de substituant ; R¹⁷, R¹⁸ et R²² à R²⁷ représentent indépendamment un atome d'hydrogène, ou n'importe lesquels des substituants (y1) à (y11) suivants qui peuvent porter 1 à 3 groupes indentiques ou différents choisis parmi des groupes servant de substituant γ ; ou bien R¹⁷ et R¹⁸, R²² et R²³, R²¹ et R²⁵, et R²⁶ et R²⁷ peuvent être liés indépendamment conjointement avec l'atome d'azote voisin pour former un groupe aminocyclique aliphatique tri- à octogonal
(y1) un groupe alkyle en C₁ à C₆ ;
(y2) un groupe alcényle en C₂ à C₆ ;
(y3) un groupe alcynyle en C₂ à C₆ ;
(y4) un groupe cycloalkyle en C₃ à C₈ ;
(y5) un groupe hétérocycloalkyle cyclique tri- à décagonal;
(y6) un groupe aryle en C₆ à C₁₀ ;
(y7) un groupe hétéroaryle cyclique penta- à décagonal ;
(y8) un groupe (cycloalkyle en C₃ à C₈) (alkyle en C₁ à C₆) ;
(y9) un groupe (hétérocycloalkyle cyclique tri- à décagonal)(alkyle en C₁ à C₆) ;
(y10) un groupe (aryle en C₆ à C₁₀ (alkyle en C₁ à C₆) ;
(y11) un groupe (hétéroaryle cyclique penta- à décagonal)-(alkyle en C₁ à C₆) ;
[Groupe servant de substituant β]
n'importe lequel des substituants (z1) à (z3) suivants qui peuvent porter 1 à 3 groupes indentiques ou différents choisis parmi des groupes servant de substituant γ ; groupe servant de substituant γ :
(z1) un groupe alkyle en C₁ à C₆ ;
(z2) un groupe alcényle en C₂ à C₆ ;
(z3) un groupe alcynyle en C₂ à C₆ ;
[Groupe servant de substituant γ]
(1) un atome d'halogène ;
(2) un groupe nitro ;
(3) un groupe cyano ;
(4) un groupe OR²⁸ ;
(5) un groupe SR²⁹ ;
(6) un groupe NR³⁰R^{J} (R³⁰ et R^{J} représentent indépendamment un atome d'hydrogène, un groupe alkyle en C₁ à C₆, un groupe alcényle en C₂ à C₆, un groupe hydroxyalkyle en C₁ à C₆, un groupe (aryle en C₆ à C₁₀) (alkyle en C₁ à C₆ ou un groupe aryle en C₆ à C₁₀) ;
(7) un groupe N⁺R^{K}R^{L}R^{M} (R^{K-M} représentent indépendamment un groupe alkyle en C₁ à C₆, un groupe alcényle en C₂ à C₆, un groupe hydroxyalkyle en C₁ à C₆, un groupe (aryle en C₆ à C₁₀) (alkyle en C₁ à C₆ ou un groupe aryle en C₆ à C₁₀) ;
(8) un groupe COR³¹ ;
(9) un groupe COOR³² ;
(10) un groupe OCOR³³ ;
(11) un groupe NHCOR³⁴ ;
(12) un groupe NHC(=NH)-NH₂ ;
(13) un groupe C(=NH)-NH₂ (qui est lié à un atome d'azote d'un groupe hétérocycloalkyle)
(14) un groupe NR³⁵CONR³⁶R³⁷ ;
(15) un groupe NR^{N}COOR^{O} ;
(16) un groupe CONR³⁸R³⁹ ;
(17) un groupe SO₂NR⁴⁰R⁴¹ ;
(18) un groupe hydroxyalkyle en C₂ à C₆ ;
(19) un groupe hétéroaryle azoté cyclique penta- à décagonal ;
(dans les groupes R²⁸, R²⁹, R³¹⁻³⁵, R^{N} et R^{O} représentent indépendamment un atome d'hydrogène, un groupe alkyle en C₁ à C₆ ou un groupe (aryle en C₆ à C₁₀) (alkyle en C₁ à C₆)_{;} R³⁶ à R⁴¹ représentent indépendamment un atome d'hydrogène ou un groupe alkyle en C₁ à C₆, ou bien R³⁶ et R³⁷, R³⁸ et R³⁹ et R⁴⁰ et R⁴¹ peuvent indépendamment être liés conjointement avec l'atome d'azote voisin pour former un groupe amino cyclique aliphatique, tri- à octogonal ; ou un de ses sels pharmaceutiquement acceptables ; ou un de ses précurseurs médicamenteux dans lesquels un groupe est choisi entre un groupe hydroxy et un groupe amino du dérivé de benzimidazole est substitué par un groupe choisi dans le groupe consistant en des groupes (alkyle en C₁ à C₆) -CO-, (alkyle en C₁ à C₆)-O(alkyle en C₁ à C₆)-CO-, (alkyle en C₁ à C₆) -OCO- (alkyle en C₁ à C₆) -CO-, (alkyle en C₁ à C₆) -OCO- et (alkyle en C₁ à C₆)-O-(alkyle en C₁ à C₆)-OCO-.

2. Dérivé de benzimidazole suivant la revendication 1, dans lequel n représente est égal à 1, ou un de ses sels pharmaceutiquement acceptables, ou un de ses précurseurs médicamenteux.

3. Dérivé de benzimidazole suivant les revendications 1 et 2, dans lequel R¹ et R³ représentent indépendamment un atome d'hydrogène, un atome d'halogène, n'importe lequel des substituants (A) à (C) qui peuvent porter 1 à 3 groupes identiques ou différents choisis parmi les groupes servant de substituants α, ou n'importe lequel des substituants (H) à (M), R² représente indépendamment un atome d'hydrogène, un atome d'halogène, un groupe cyano, n'importe lequel des substituants (A) à (C) qui peuvent porter 1 à 3 groupes identiques ou différents choisis parmi les groupes servant de substituants α, n'importe lequel des substituants (D) à (G) qui peuvent porter 1 à 3 groupes identiques ou différents choisis parmi les groupes servant de substituants α et β, ou n'importe lequel des substituants (H) à (M), ou un de ses sels pharmaceutiquement acceptables, ou un de ses précurseurs médicamenteux.

4. Dérivé de benzimidazole suivant la revendication 3, dans lequel R¹ représente un groupe OR⁷ (sous réserve que R⁷ représente un groupe alkyle en C₁ à C₆ qui porte un groupe hydroxy, NR¹⁷R¹⁸ ou N⁺R^{D}R^{E}R^{F} (R¹⁷, R¹⁸ et R^{D-F} peuvent répondre aux définitions indiquées dans la revendication 1) ) ou un groupe hydroxy ; R² représente un groupe OR⁷ (sous réserve que R⁷ représente un groupe alkyle en C₁ à C₆ qui porte un groupe hydroxy, NR¹⁷R¹⁸ ou N⁺R^{D}R^{E}R^{F} (R¹⁷, R¹⁸ et R^{D-F} répondent aux définitions indiquées dans la revendication 1)), un groupe hydroxy, ou un groupe aryle en C₆ à C₁₀ qui peut porter un groupe hydroxy ou OR¹⁵ (R¹⁵ répond à la définition indiquée dans la revendication 1) ; R³ représente un atome d'hydrogène; ou un de ses sels pharmaceutiquement acceptables, ou un de ses précurseurs médicamenteux.

5. Composition pharmaceutique comprenant comme ingrédient actif un dérivé de benzimidazole suivant les revendications 1 à 4, ou un de ses sels pharmaceutiquement acceptables, ou un de ses précurseurs médicamenteux.

6. Composition pharmaceutique suivant la revendication 5 pour la prévention ou le traitement d'une maladie associée à une anomalie du taux plasmatique d'acide urique.

7. Composition pharmaceutique suivant la revendication 6, dans laquelle la maladie associée à une anomalie du taux plasmatique d'acide urique est une maladie choisie entre la goutte, l'hyperuricémie, la lithiase urinaire, la néphropathie hyperuricémique et la néphropathie urique aigue.

8. Composition pharmaceutique suivant la revendication 6, dans laquelle la maladie associée à une anomalie du taux plasmatique d'acide urique est la goutte.

9. Composition pharmaceutique suivant la revendication 6, dans laquelle la maladie associée à une anomalie du taux plasmatique d'acide urique est l'hyperuricémie.

10. Composition pharmaceutique suivant l'une quelconque des revendications 5 à 9, comprenant en association comme ingrédient actif au moins un agent choisi dans un groupe consistant en la colchicine, un agent anti-inflammatoire non stéroïdien, un stéroïde cortico-surrénalien, un inhibiteur de synthèse d'acide urique, un médicament uricosurique, un alcalinisant urinaire et une acide urique-oxydase.

11. Composition pharmaceutique suivant la revendication 10, dans laquelle l'agent anti-inflammatoire non stéroïdien est l'indométacine, le naproxène, le fenbufène, le pranoprofène, l'oxaprozine, le kétoprofène, l'étoricoxib ou le ténoxicam ; l'inhibiteur de synthèse d'acide urique est l'allopurinol, l'oxypurinol, le fébuxostat ou le Y-700 ; le médicament uricosurique est le probénécide, le bucolome ou la benzbromarone ; l'alcalinisant urinaire est le carbonate acide de sodium ; le citrate de potassium ou le citrate de sodium ; l'acide urique-oxydase est la rasburicase, l'uricase PEG-20 ou une acide-oxydase recombinante (uricase).


### SEQUENCE LISTING

<110> KISSEI PHARMACEUTICAL CO., LTD.
   KIKUCHI, Norihiko
   NONAKA, Yoshinori
   TATANI, Kazuya
   HIRATOCHI, Masahiro
   KURAMOCHI, Yu
   ISAJI, Masayuki
   SHIMIZU, Kazuo
   MIYAGI, Takashi
<120> BENZIMIDAZOLE DERIVATIVES AND THEIR PHARMACEUTICAL USES
<130> PCT-A0450-00
<150> JP 2003/432581
   <151> 2003-12-26
<160> 17
<170> Patentin version 3.1
<210> 1
   <211> 20
   <212> DNA
   <213> Artificial Sequence
<220>
   <223> Description of Artificial Sequence: primer
<400> 1
   tgcactgcat ggttgctgct 20
<210> 2
   <211> 20
   <212> DNA
   <213> Artificial Sequence
<220>
   <223> Description of Artificial Sequence: primer
<400> 2
   gtctaagtcc tgtggcttcc 20
<210> 3
   <211> 20
   <212> DNA
   <213> Artificial Sequence
<220>
   <223> Description of Artificial Sequence: primer
<400> 3
   aggagccaga gggaatcaat 20
<210> 4
   <211> 20
   <212> DNA
   <213> Artificial Sequence
<220>
   <223> Description of Artificial Sequence: primer
<400> 4
   acatcttggt gagtgagttg 20
<210> 5
   <211> 29
   <212> DNA
   <213> Artificial Sequence
<220>
   <223> Description of Artificial Sequence: primer
<400> 5
   ccgctcgaga ggagccagag ggaatcaat 29
<210> 6
   <211> 28
   <212> DNA
   <213> Artificial Sequence
<220>
   <223> Description of Artificial Sequence: primer
<400> 6
   cgtctagaac atcttggtga gtgagttg 28
<210> 7
   <211> 18
   <212> DNA
   <213> Artificial Sequence
<220>
   <223> Description of Artificial Sequence: primer
<400> 7
   gccagccagc agcaaaaa 18
<210> 8
   <211> 18
   <212> DNA
   <213> Artificial Sequence
<220>
   <223> Description of Artificial Sequence: primer
<400> 8
   tggagaagtg gctgacct 18
<210> 9
   <211> 21
   <212> DNA
   <213> Artificial Sequence
<220>
   <223> Description of Artificial Sequence: primer
<400> 9
   atttaccagt gctgccgtga g 21
<210> 10
   <211> 21
   <212> DNA
   <213> Artificial Sequence
<220>
   <223> Description of Artificial Sequence: primer
<400> 10
   aaaccgacag cagttgtcca.g 21
<210> 11
   <211> 26
   <212> DNA
   <213> Artificial Sequence
<220>
   <223> Description of Artificial Sequence: probe
<400> 11
   agagcgtcaa tccagagttc agccca 26
<210> 12
   <211> 22
   <212> DNA
   <213> Artificial Sequence
<220>
   <223> Description of Artificial Sequence: primer
<400> 12
   ggcagcttgc atcttgaatt tc 22
<210> 13
   <211> 22
   <212> DNA
   <213> Artificial Sequence
<220>
   <223> Description of Artificial Sequence: primer
<400> 13
   caaaaacgag tgaaccagga ca 22
<210> 14
   <211> 30
   <212> DNA
   <213> Artificial Sequence
<220>
   <223> Description of Artificial Sequence: probe
<400> 14
   ccttgtttgt catcacctgc ttggtgatct 30
<210> 15
   <211> 25
   <212> DNA
   <213> Artificial Sequence
<220>
   <223> Description of Artificial Sequence: primer
<400> 15
   gctggtccga ccatatttac cttac 25
<210> 16
   <211> 21
   <212> DNA
   <213> Artificial Sequence
<220>
   <223> Description of Artificial Sequence: primer
<400> 16
   cgcttccagc aatggtagag a 21
<210> 17
   <211> 26
   <212> DNA
   <213> Artificial Sequence
<220>
   <223> Description of Artificial Sequence: probe
<400> 17
   tcaccaagtc tgaactccac gccatc.